(19) **Europäisches Patentamt / European Patent Office / Office européen des brevets**

(11) **EP 4 569 133 B1**

(12) **EUROPEAN PATENT SPECIFICATION**

(45) Date of publication and mention
of the grant of the patent:
**27.05.2026  Bulletin 2026/22**

(21) Application number: **23851091.1**

(22) Date of filing: **20.10.2023**

(51) International Patent Classification (IPC):
**C12Q 1/6806** (2018.01)

(52) Cooperative Patent Classification (CPC):
(C-Sets available)
**G16B 20/50; C12Q 1/6806; G16B 20/00;
G16B 20/20; G16B 40/00**                    (Cont.)

(86) International application number:
**PCT/CN2023/125605**

(87) International publication number:
**WO 2025/081458 (24.04.2025 Gazette 2025/17)**

(54) **METHODS FOR RELIABLE NONINVASIVE PREIMPLANTATION GENETIC TESTING**

VERFAHREN FÜR ZUVERLÄSSIGES GENETISCHES TESTEN EINER NICHT-INVASIVEN PRÄIMPLANTATION

PROCÉDÉS DE TEST GÉNÉTIQUE DE PRÉIMPLANTATION NON INVASIF FIABLE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**18.06.2025  Bulletin 2025/25**

(60) Divisional application:
**26155399.4 / 4 717 781**

(73) Proprietors:
• **Beijing Changping Laboratory
Changping District
Beijing 102206 (CN)**
• **Peking University
Beijing 100871 (CN)**

(72) Inventors:
• **HUANG, Lei
Beijing 100871 (CN)**
• **GE, Hao
Beijing 100871 (CN)**
• **ZHANG, Ruiqi
Beijing 100871 (CN)**
• **XIE, Xiaoliang
Beijing 102206 (CN)**

(74) Representative: **Vossius & Partner
Patentanwälte Rechtsanwälte mbB
Siebertstraße 3
81675 München (DE)**

(56) References cited:
WO-A1-2021/073604      WO-A2-2020/115511
CN-A- 111 440 857      CN-A- 116 665 774
US-A1- 2019 002 967

• CHONGYI CHEN ET AL: "Single-cell whole-genome analyses by Linear Amplification via Transposon Insertion (LIANTI)", SCIENCE, vol. 356, no. 6334, 14 April 2017 (2017-04-14), US, pages 189 - 194, XP055554122, ISSN: 0036-8075, DOI: 10.1126/science.aak9787
• HUANG LEI ET AL: "Noninvasive preimplantation genetic testing for aneuploidy in spent medium may be more reliable than trophectoderm biopsy", PROCEEDINGS OF THE NATIONAL ACADEMY OF SCIENCES (PNAS), vol. 116, no. 28, 9 July 2019 (2019-07-09), pages 14105 - 14112, XP093244724, ISSN: 0027-8424, Retrieved from the Internet <URL:https://pmc.ncbi.nlm.nih.gov/articles/PMC6628824/pdf/pnas.201907472.pdf> DOI: 10.1073/pnas.1907472116

**(Cont. next page)**

- SHITARA AKIHIRO ET AL: "Cell-free DNA in spent culture medium effectively reflects the chromosomal status of embryos following culturing beyond implantation compared to trophectoderm biopsy", PLOS ONE, vol. 16, no. 2, 11 February 2021 (2021-02-11), US, pages e0246438, XP093244758, ISSN: 1932-6203, Retrieved from the Internet <URL:https://pmc. ncbi.nlm.nih.gov/articles/PMC7877764/pdf/ pone.0246438.pdf> DOI: 10.1371/ journal.pone.0246438
- YIN BAOLI ET AL: "Validation of preimplantation genetic tests for aneuploidy (PGT-A) with DNA from spent culture media (SCM): concordance assessment and implication", REPRODUCTIVE BIOLOGY AND ENDOCRINOLOGY, vol. 19, no. 1, 1 December 2021 (2021-12-01), GB, XP093244767, ISSN: 1477-7827, Retrieved from the Internet <URL:https://pmc.ncbi.nlm.nih.gov/ articles/PMC7936457/pdf/ 12958_2021_Article_714.pdf> DOI: 10.1186/ s12958-021-00714-3
- DE WITTE L ET AL: "GENType: all-in-one preimplantation genetic testing by pedigree haplotyping and copy number profiling suitable for third-party reproduction", HUMAN REPRODUCTION, vol. 37, no. 7, 30 June 2022 (2022-06-30), GB, pages 1678 - 1691, XP093244715, ISSN: 0268-1161, Retrieved from the Internet <URL:https://watermark.silverchair. com/deac088.pdf? token=AQECAHi208BE49Ooan9kkhW_Er cy7Dm3ZL_9Cf3qfKAc485ysgAAA8MwggO_Bgk qhkiG9w0BBwagggOwMIIDrAIBADCCA6UGCSq GSIb3DQEHATAeBglghkgBZQMEAS4wEQQMLP RhBcBV7guGjJvhAgEQglIDdjRT8hmziuDSIwHto r41EYXuSILWzmGd1vVecAp4bHTwD_ImJkdHEc yb6rAhJs6Cht1D5d2wegCEPDRcQxh6TZnQ7P v9> DOI: 10.1093/humrep/deac088
- CHEN ET AL.: "Single- Cell Whole Genome Analyses by Linear Amplification via Transposon Insertion (LIANTI", SCIENCE, vol. 356, no. 6334, 14 April 2017 (2017-04-14), pages 189 - 194, XP055554122, ISSN: 0036-8075, DOI: 10.1126/ science.aak9787
- OU ZHANHUI, DENG YU, LIANG YUNHAO, CHEN ZHIHENG, SUN LING: "Improved Non-Invasive Preimplantation Genetic Testing for Beta-Thalassemia Using Spent Embryo Culture Medium Containing Blastocoelic Fluid", FRONTIERS IN ENDOCRINOLOGY, FRONTIERS RESEARCH FOUNDATION, CH, vol. 12, CH , XP093305495, ISSN: 1664-2392, DOI: 10.3389/ fendo.2021.793821

Remarks:

The complete document including Reference Table(s) and the Sequence Listing(s) can be downloaded from the EPO website

(52) Cooperative Patent Classification (CPC): (Cont.)

C-Sets
**C12Q 1/6806, C12Q 2535/122, C12Q 2531/101**

**Description**

**FIELD OF THE INVENTION**

The present invention relates to one or more accurate methods for noninvasive preimplantation genetic testing. Specifically, the present invention relates to systems and methods for amplifying minute amounts of DNA in a solution, and linkage analysis methods of analyzing a biological sample of culture medium from an in vitro cultured embryo to determine the susceptibility of a genetic disease in the embryo.

**BACKGROUND OF THE INVENTION**

Preimplantation genetic testing (PGT) (Handyside et al., 1989 and 1990 for first children born) as in known in the art has been widely used for clinical *in vitro* fertilization (IVF) to avoid selecting embryos with monogenic diseases (Kerem et al., 1989; Handyside et al., 1992; Liu et al., 1994; Harton et al., 1996; Ao et al., 1998; Sermon et al., 1998; Xu et al., 1999; Hussey et al., 1999; Ray et al., 2000; De Rycke et al., 2001; Moutou et al., 2001; Verlinsky et al., 2001; Sermon et al., 2001; Girardet et al., 2003; Fiorentino et al., 2006; Kahraman et al., 2014), aneuploidy (Munné et al., 1993; Wilton et al., 2001; Wells et al., 2002; Treff et al., 2010; Gutiérrez-Mateo et al., 2011; Yang et al., 2012; Scott et al., 2013; Forman et al., 2013; Wells et al., 2014; Rubio et al., 2017), structure variation(Conn et al., 1998; Scriven et al., 1998; Coonen et al 2000; Munné et al., 2000; Escudero et al., 2003; Melotte et al., 2004; Le Caignec et al., 2006; Traversa et al., 2010; Fiorentino et al., 2010; Fiorentino et al., 2010; Rius et al., 2011; Fiorentino et al., 2011; Treff et al., 2016; Zhang et al., 2017; Tan et al., 2013; Chow et al., 2018), or polygenic disease (Treff et al., 2019; Kumar et al. 2022). It has been reported that using one NGS-based testing, chromosome copy number analysis and linkage analysis and targeted haplptyping for mutation can be dome simutatiously (Yan et al., 2015). Currently for PGT, a trophectoderm (TE) biopsy is needed (Dokras et al., 1990; Handyside et al., 1990; Kokkali et al., 2005), which is invasive and subject to sampling bias.

Use of the cell-free DNA in blastocoele fluid (BF) for amplification and genetic testing was reported as a minimally invasive preimplantation genetic testing (Palini 2013, Gianaroli et al., 2014; Tobler et al., 2015; Magli et al., 2016; Zhang et al., 2016; Shangguan et al.,2017,;Capalbo et al., 2018; Tsuiko et al., 2018; Magli et al., 2018, Shitara Akihiro et al, 2021).

A completely noninvasive way for preimplantation genetic testing is described for aneuploidy and for monogenic disease using the cell-free DNA in spent embryo culture medium (Galluzzi et al.,2015; Wu et al., 2015; Xu et al., 2016, Shamonki et al., 2016; Feichtinger et al., 2017; Lane et al., 2017; Liu et al., 2017; Ho et al., 2018; Vera-Rodriguez et al., 2018; Capalbo et al., 2018; Fang et al., 2019; Huang et al., 2019; Rubio et al., 2019; Yeung et al., 2019; Jiao et al. 2019; Yin Baoli et al. 2021, Ou et al., 2022, WO 2020/115511).

Minimally invasive preimplantation genetic testing and non-invasive preimplantation genetic testing are the two major methods for avoiding biopsy damage in preimplantation genetic testing. The method of embryo culture and the approach of spent culture medium (SCM) collection, as well as the single-cell whole genome amplification (scWGA) method used for minute amounts of cell-free DNA, can directly affect the results. The success rate of amplification of samples from blastocoele fluid and spent embryo culture medium varies greatly in different clinical centers. When using the same amplification method, the success rate of amplification in BF was much lower than that in spent culture medium (SCM), such as 62.5% SCM vs 44.4% BF reported by Galluzzi et al. (2015), 89.7% SCM vs 27.4% BF reported by Capalbo et al (2018). Although the amplification success rate of SCM was higher than that of BF, the results of SCM were affected by maternal contamination and by the addition of exogenous proteins to the culture medium for embryo development. It has been shown that maternal DNA fragments in maternal blood are longer than fetal DNA fragments (Chan et al., 2004) and the bias of the amplification technique for fragment length makes it ineffective for fetal DNA amplification. In addition, it is important to ensure the accuracy of amplification while maintaining the yield for clinical testing use. Currently, available commercial single-cell amplification kits are based on DOP-PCR, MDA or MALBAC techniques, and these commercial kits differ in amplification efficiency and fidelity (Huang et al., 2015). These methods are all exponential amplification, which causes high false negatives and false positives. In 2017, a new amplification method was invented, which is a linear amplification (Chen et al., 2017, US 10,894,980 referred to as "LIANTI"). False positive rate of SNP detection was much lower than the other methods. However, these kits are not improved for the DNA fragment length characteristics and high protein contained in the culture medium, but only enlarge the reaction system, which results in the amplification efficiency of the culture medium not being high. An efficient and accurate amplification method for obtaining more embryonic DNA information in SCM or/and BF systems is urgently needed. However, the original LIANTI method's yield is too low, and the reproducibility is low, which is not acceptable for clinical application since the embryos are precious.

As for the analysis, there are many reports of non-invasive preimplantation genetic testing for aneuploidy (niPGT-A) (reviewed in Leaver et al., 2020), but only several reports on noninvasive preimplantation genetic testing for monogenic diseases (niPGT-M) (Galluzzi et al., 2015; Zhang et al., 2016; Shangguan et al., 2018; Wu et al., 2015; Liu et al., 2017; Capalbo at al., 2018; Ou et al., 2022). In the reports of niPGT-M by SCM, compared with the trophectoderm biopsy results, the genotype concordance in the successfully amplified SCM also varied greatly, from 21% to 88% (Capalbo et al., 2018;

Liu et al., 2017; Ou et al.,2022). The false-positive and false-negative rates were not mentioned in these papers, which are important metrics to assess the accuracy of a testing method. However, based on the genotype concordance rates in the reported articles, the rates of false negatives and false positives would be very high, making noninvasive PGT-M disadvantageous clinically (Capalbo et al., 2018; Cimadomo et al., 2020). Rather than the genotype concordance rate, the most important standard for PGT-M assessment is the misdiagnosis rate. The misdiagnosis rate published by ESHRE PGT consortium was very low (<0.1%) (De Rycke et al., 2017). The risk of misdiagnosis with a new testing method should be assessed and compared with the traditional PGT-M.

In addition to detecting the disease-associated mutation site, the analysis method used in the reported papers was a linkage analysis method. It is recommended that the minimum number of fully informative markers are at least two STRs or SNPs proximal and distal to the mutation site when the Allele Drop-Out (ADO) rates are below 5% (ESHRE 2020). Ou et al. used 4 informative SNPs and Liu et al. used 10 informative SNPs. Since the ADO rates of SCM or BF are much higher than 5%, simply using a few informative sites for linkage analysis can easily lead to misdiagnosis. The distances of the used informative SNP markers to the gene are crucial for the residual recombination risk.

Moreover, maternal contamination further caused diagnostic errors. Widely used linkage analysis methods are based on the Lander-Green algorithm and its variants, which do not consider the maternal contamination and require high quality sequencing data (Lander et al. 1987; Kruglyak et al. 1996; Idury et al. 1997; Kruglyak et al. 1998; Abecasis et al. 2002). Directly applying these methods result in high rates of misdiagnosis (Capalbo et al, 2018; Cimadomo et al. 2020). There are few linkage analysis methods, which has taken into account the mixing or contamination with maternal cells (Fan et al. 2002, Nabieva et al. 2020). However, They still require much higher quality sequencing data than those from culture media, and their main applications are based on blood samples or cells in the placenta or amniotic fluid of the mother during pregnancy. (Fan et al. 2002, Nabieva et al. 2020) . These existing methods will cause more diagnostic errors when applied to the sequencing data from culture media. The high false-positive and negative rate of niPGT-M is mainly related to the low initial DNA content in the spent culture medium and the existence of maternal contamination as well as high ADO rate, which make all these existing niPGT-M methods undesirable for clinical application. The key to applying niPGT-M to the clinic IVF is that the accuracy of this technique can reach the level of the current PGT-M. For niPGT-M, the accuracy should be greater than 97% by detecting the existence of the allele that carries the disease's causing mutation in the embryo. To our best knowledge, there hasn't been a linkage-analysis method for a noninvasive procedure. The most critical solution is to develop a more accurate linkage analysis method for samples with high ADO rates in the presence of maternal contamination.

## SUMMARY OF THE INVENTION

The invention is set out in the appended set of claims. In a first aspect of the invention, a method for amplifying the DNA in a solution is provided, wherein the DNA content in the solution can be at the minimum of picogram level, the method comprising the following steps:

combining lysis buffer with the solution, heating the solution, then combining protease with the solution, incubating the solution, followed by inactivation of the protease under high temperatures to obtain lysate;

combining transposome with the lysate, wherein the transposome includes a transposase and a transposon, wherein the transposon comprises a transposase binding site and an RNA polymerase promoter sequence, wherein the transposome binds to DNA in the solution, the transposase cuts the DNA in the solution to produce DNA fragments and the transposon becomes incorporated or inserted into the DNA fragments;

a 9-bp gap resulting from transposon insertion is filled and extended down to both ends of each fragment;

performing in vitro transcription using the original sequence as a template to form RNA;

combining the RNA with a DNA primer and a reverse transcription system to form a first strand cDNA via reverse transcription, wherein the DNA primer is complementary to the respective RNA strand of the sequence of transposase binding site; and

forming a second strand DNA via cycling amplification.

In a preferred embodiment of the first aspect of the invention:

(i) the solution is a culture medium or a blastocoele fluid, and wherein preferably the 3'-end of the DNA primer contains the sequence of 5'-GACAG-3' or 5'-CTGTC-3', wherein more preferably the DNA primer contains the sequence set force as 5'-AGATGTGTATAAGAGACAG-3' (SEQ ID NO.: 1), or 5'-TCTACACATATTCTCTGTC-3' (SEQ ID NO.: 2) or is at least 70%, preferably 80%, preferably 90%, preferably 95%, preferably 100% identical with SEQ ID NO.: 1 or SEQ ID NO.: 2;

(ii) the DNA primer has a length of 15bp to 20bp;

(iii) the lysis buffer is 2x to 10x lysis buffer;

(iv) the working concentration of the protease in the lysis mixture is higher than 0.6 mg/mL, preferably in the range of 0.8-2 mg/mL;
(v) the incubation is performed at 45-60°C for 1 to 3 hours;
(vi) the inactivation is performed at 80-90°C;
(vii) the working concentration of the transposome is higher than 6 nM, preferably in the range of 10-40 nM; or
(viii) the cycling amplification is performed more than 4 cycles, such as 4 to 20 cycles, preferably 6 to 15 cycles.

In another preferred embodiment of the first aspect of the invention, the method comprises the following steps: combining 2x to 10x lysis buffer with the culture medium, heating the culture medium, combining protease with the culture medium to the working concentration of 0.8-2 mg/mL and incubating the culture medium at 45-60°C for 1 to 3 hours, followed by inactivation of the protease at 80-90°C to obtain lysate;

combining transposome with the lysate to the working concentration of 10-40 nM, wherein the transposome includes a transposase and a transposon, wherein the transposon comprises a transposase binding site and an RNA polymerase promoter sequence, wherein the transposome binds to DNA in the solution, the transposase cuts the DNA in the solution to produce DNA fragments and the transposon becomes incorporated or inserted into the DNA fragments; a 9-bp gap resulting from transposon insertion is filled and extended down to both ends of each fragment; performing in vitro transcription using the original sequence as a template to form RNA;
combining the RNA with DNA primer pairing with the nucleic acid sequence conjugated with the transposome and a reverse transcription system, forming a first strand cDNA via reverse transcription, wherein the DNA primer contains the sequence set forth as 5'-AGATGTGTATAAGAGACAG-3' (SEQ ID NO.: 1) or 5'-TCTACACATATTCTCTGTC-3' (SEQ ID NO.: 2), or is at least 70%, preferably 80%, preferably 90%, preferably 95, preferably 100% identity with SEQ ID NO.: 1 or SEQ ID NO.: 2; and
forming a second strand DNA via 6 to 15 cycles of amplification.

In a second aspect of the invention, a method of analyzing a biological sample of culture medium of an in vitro cultured embryo to determine the susceptibility of a genetic disease in the embryo ist provided, comprising:

1) Amplifying the DNA molecules from the biological sample of culture medium of the in vitro cultured embryo using the method according to any one of claims 1-3;
2) Preparing DNA libraries and sequencing the DNA molecules from the biological sample to obtain a plurality of sequence reads;
3) Performing mapping and SNP calling, by a computer system, to generate the SNP data and controlling its quality;
4) Conducting haplotype pre-phasing, by a computer system, for the blood samples of both parents or solely the disease-carrying parent;
5) Calculating the likelihood of the four possible inheritance scenarios at each single SNP representing whether the genetic chains are inherited from the paternal or maternal alleles of the parents, taking into account the maternal cell contamination (MCC) rate and haplotype status;
6) Estimating the MCC rate, and determining the haplotype status of each SNP to represent whether only DNA from one parent is present in the culture medium;
7) Calculating the likelihood of observing the SNP data within regions adjacent to the disease-causing mutation site, combining the likelihood at each single SNP and the recombination probabilities;
8) Determining whether the embryo carries the disease-causing chromosome, and providing a level of confidence.

In a third aspect of the invention, a computer program is provided comprising a plurality of instructions capable of being executed by a computer system and arranged when executed to control the computer system to analyze a biological sample of culture medium of an in vitro cultured embryo to determine the susceptibility of a genetic disease in the embryo, configured, when executed, to cause the computer system to perform:

1) Perform mapping and SNP calling, by a computer system, on a plurality of sequence reads to generate the SNP data and controlling its quality, wherein the plurality of sequence reads was obtained by preparing DNA libraries and sequencing DNA molecules amplified by the method according to any of claims 1 to 3 from the biological sample of culture medium of the in vitro cultured embryo;
2) Conduct haplotype pre-phasing, by a computer system, for the blood samples of both parents or solely the disease-carrying parent;
3) Calculate the likelihood of the four possible inheritance scenarios at each single SNP representing whether the genetic chains are inherited from the paternal or maternal alleles of the parents, taking into account the maternal cell contamination (MCC) rate and haplotype status;

4) Estimate the MCC rate, and determining the haplotype status of each SNP to represent whether only DNA from one parent is present in the culture medium;

5) Calculate the likelihood of observing the SNP data within regions adjacent to the disease-causing mutation site, combining the likelihood at each single SNP and the recombination probabilities;

6) Determine whether the embryo carries the disease-causing chromosome, and providing a level of confidence.

In a preferred embodiment of the second or third aspect of the invention:

(i) the embryo has contamination caused by maternal cells and/or haplotype loss;

(ii) in step 3) of the method or step 1) caused by the computer program, mapping and SNP calling outputs the physical location, allele type, allele depth and sequencing quality for each SNP of the biological sample of culture medium of the in vitro cultured embryo;

(iii) in step 3) of the method or step 1) caused by the computer program, controlling the quality of the SNP data is performed via filtering out the SNPs with low gene quality (GQ) values of the biological sample of culture medium of the in vitro cultured embryo , preferably filtering out the SNPs with the lowest 20% GQ values of the biological sample of culture medium of the in vitro cultured embryo ;

(iv) wherein in step 3) of the method or step 1) caused by the computer program, estimating the sequencing error rate and the relative density of SNPs in the region adjacent to the disease-causing mutation site, and excluding the sample of the culture medium with an extremely low SNP density, or high sequencing error rate, wherein preferably the excluded SNP density is smaller than 0.001, or the excluded sequencing error rate is larger than 0.2;

(v) in step 4) of the method or step 2) caused by the computer program, employing any one or more of the following samples in the family: the blood sample of proband, or the blood sample of at least one of the grandparents;

(vi) in step 5) of the method or step 3) caused by the computer program, integrating the estimated sequencing error rate when calculating the likelihood for each individual SNP;

(vii) step 6) of the method or step 4) caused by the computer program further include the following steps:

a) Initially estimating the MCC rate and haplotype status of each SNP in the region adjacent to the disease-causing mutation site; and

b) Recursively updating the MCC rate and haplotype status of each SNP, wherein preferably step b) using a label-searching method or after step a), excluding the sample with abnormal MCC rate, wherein preferably the abnormal MCC rate is larger than 0.65 or less than -0.5;

(viii) in step 7) of the method or step 5) caused by the computer program, using a Bayesian model and a recursive algorithm;

(ix) in step 8) of the method or step 6) caused by the computer program, first plotting the log likelihood curve for both upstream and downstream regions of the disease-causing mutation site; or

(x) the genetic disease includes both monogenic and polygenic diseases.

In another preferred embodiment of the second or third aspect of the invention, after step 8) of the method or step 6) caused by the computer program, multiple identified SNPs of the embryo are combined to determine its susceptibility to a polygenic disease.

In a fourth aspect of the invention, a computer system for analyzing a biological sample of culture medium of an in vitro cultured embryo to determine the susceptibility of a genetic disease in the embryo is provided, comprising:

1) Means for performing mapping and SNP calling, by a computer system, on a plurality of sequence reads to generate the SNP data and controlling its quality, wherein the plurality of sequence reads was obtained by preparing DNA libraries and sequencing DNA molecules amplified by the method according to any of claims 1 to 3 from the biological sample of culture medium of the in vitro cultured embryo;

2) Means for conducting haplotype pre-phasing, by a computer system, for the blood samples of both parents or solely the disease-carrying parent;

3) Means for calculating the likelihood of the four possible inheritance scenarios at each single SNP representing whether the genetic chains are inherited from the paternal or maternal alleles of the parents, taking into account the maternal cell contamination (MCC) rate and haplotype status;

4) Means for estimating the MCC rate, and determining the haplotype status of each SNP to represent whether only DNA from one parent is present in the culture medium;

5) Means for calculating the likelihood of observing the SNP data within regions adjacent to the disease-causing mutation site, combining the likelihood at each single SNP and the recombination probabilities;

6) Means for determining whether the embryo carries the disease-causing chromosome, and providing a level of confidence.

In a preferred embodiment of the fourth aspect of the invention:

(i) the embryo has contamination caused by maternal cells and/or haplotype loss;
(ii) in means 1) mapping and SNP calling outputs the physical location, allele type, allele depth and sequencing quality for each SNP of the biological sample of culture medium of the in vitro cultured embryo ;
(iii) in means 1) controlling the quality of the SNP data is performed via filtering out the SNPs with low gene quality (GQ) values of the biological sample of culture medium of the in vitro cultured embryo ;
(iv) filtering out the SNPs with the lowest 20% GQ values of the biological sample of culture medium of the in vitro cultured embryo ;
(v) in means 1), estimating the sequencing error rate and the relative density of SNPs in the region adjacent to the disease-causing mutation site, and excluding the sample of the culture medium with an extremely low SNP density, or high sequencing error rate, wherein preferably the excluded SNP density is smaller than 0.001, or the excluded sequencing error rate is larger than 0.2;
(vi) in means 2) employing any one or more of the following samples in the family: the blood sample of proband, or the blood sample of at least one of the grandparents; or
(vii) in means 3), integrating the estimated sequencing error rate when calculating the likelihood for each individual SNP.

The computer system of claim 8, wherein means 4) further includes the following functions:

a) Initially estimating the MCC rate and haplotype status of each SNP in the region adjacent to the disease-causing mutation site; and
b) Recursively updating the MCC rate and haplotype status of each SNP, wherein preferably:

(i) function b) using a label-searching method; or
(ii) after function a), excluding the sample with abnormal MCC rate, wherein preferably the abnormal MCC rate is larger than 0.65 or less than -0.5.

In another preferred embodiment of the fourth aspect of the invention:

(i) in means 5) using a Bayesian model and a recursive algorithm;
(ii) in means 6), first plotting the log likelihood curve for both upstream and downstream regions of the disease-causing mutation site; or
(iii) the genetic disease includes both monogenic and polygenic diseases.

In yet another preferred embodiment of the fourth aspect of the invention, the computer system further comprises means for combining multiple identified SNPs of the embryo to determine its susceptibility to a polygenic disease.

**BRIEF DESCRIPTION OF THE DRAWINGS**

Fig. 1 shows the workflow of an embodiment of an exemplary method for an accurate noninvasive PGT.
Fig. 2 shows the mechanism of an exemplary amplification method with added DNA primer.
Fig. 3A-Fig. 3D shows the results of single variable titration of the amplification method, including

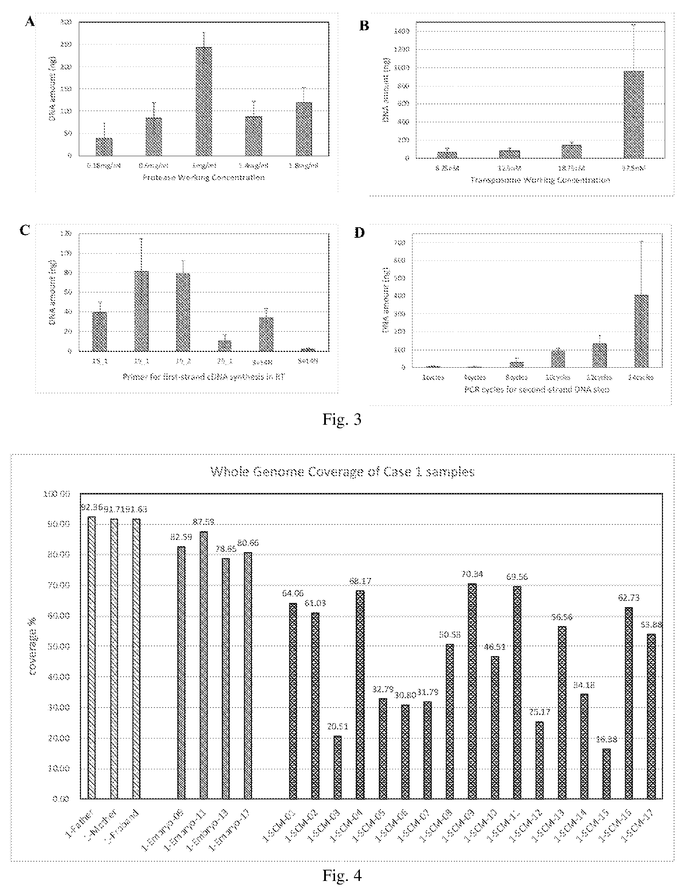

Fig. 3A) protease working concentration, Fig. 3B) transposome working concentration, Fig. 3C) primer for first-strand cDNA synthesis in reverse transcription, and Fig. 3D) PCR cycles for the second-strand DNA step.
Fig. 4 is a graph showing the genome coverage of each sample in Case 1, including gDNA of patients/proband, amplified discarded embryo DNA, and amplified culture medium DNA.

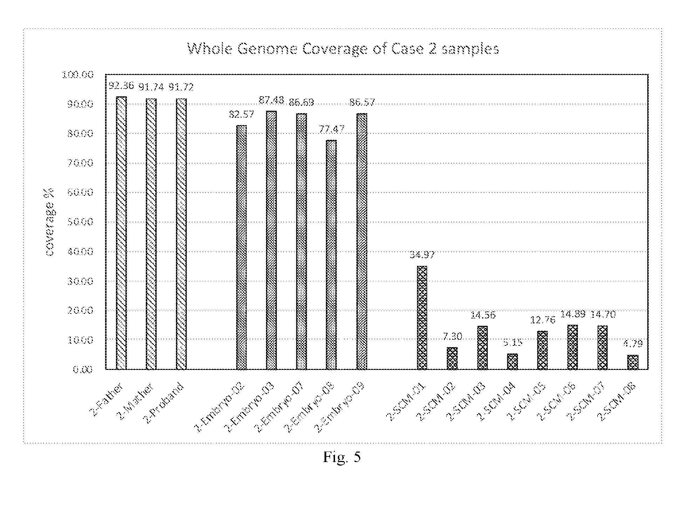

Fig. 5 is a graph showing the genome coverage of each sample in Case 2, including gDNA of patients/proband, amplified discarded embryo DNA, and amplified culture medium DNA.

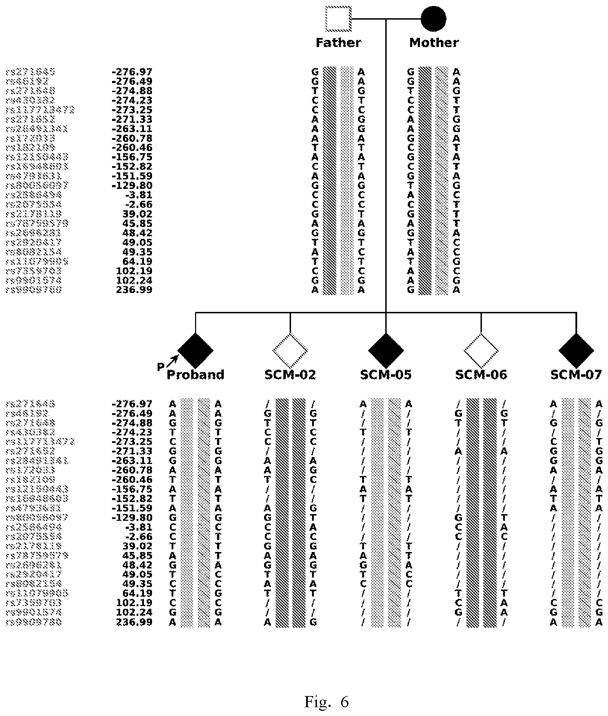

Fig. 6 is a schematic of the Case 1 family's genetic linkage analysis. The chromosome with the slash carries the disease-causing mutation.

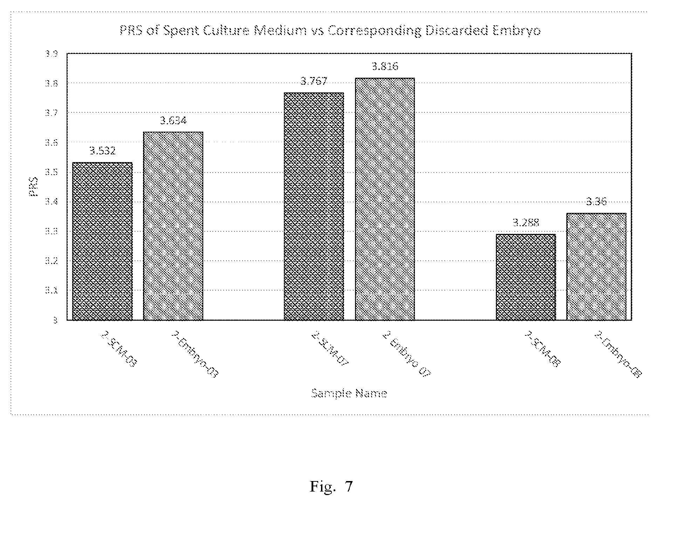

Fig. 7 shows the PRS scores of 3 discarded embryos and their corresponding spent culture media.

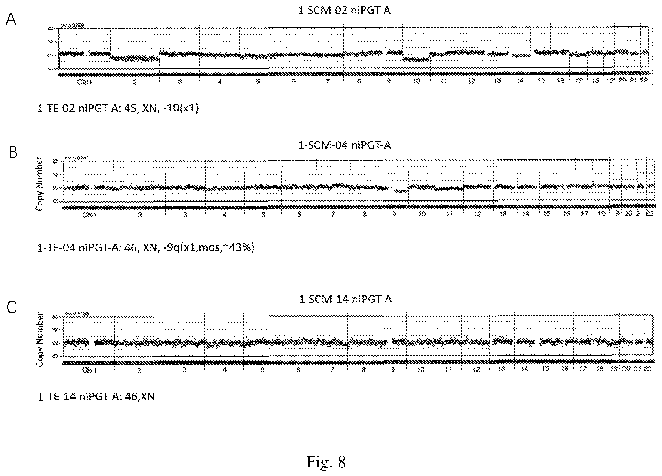

Fig. 8A-Fig. 8C shows the copy number (CN) plots of spent culture medium. Fig. 8A: An aneuploid niPGT-A profile. Fig, 8B: A mosaic niPGT-A profile. Fig. 8C: An euploid niPGT-A profile.

## DETAILED DESCRIPTION

Unless defined otherwise, all technical and scientific terms used herein have the same meaning as is commonly understood by one of skill in the art to which this invention belongs.

The term "genetic Disease" as used herein refers to a disorder caused by an abnormality in an individual's DNA or genes. These disorders can be inherited from parents or occur due to spontaneous genetic mutations.

The term "monogenic disease" as used herein refers to a genetic disorder caused by mutations in a single gene. These diseases are typically passed down from one generation to another in a predictable manner, following Mendelian inheritance patterns.

The term "polygenic disease" as used herein refers to a genetic disorder caused by the combined effect of variations in multiple genes. These diseases are often influenced by both genetic and environmental factors and do not follow simple Mendelian inheritance patterns.

The term "solution" as used herein refers to a liquid mixture in which the minor component (e.g., the DNA) is distributed within the major component (e.g., the solvent). Particularly, the solution is a complex system with high protein content, whereas the DNA in the solution can be at the minimum of picogram level. For example, the solution is a spent culture medium or blastocoele fluid.

The term "disease-causing chromosome" as used herein refers to the chromosome or chromosomal region that contains a gene or genes responsible for a particular genetic disease.

The term "disease-causing mutation site" as used herein refers to the specific location within a gene or chromosome where a mutation occurs, leading to the development of a genetic disease.

The term "transposon" as used herein refers to a nucleic acid sequence in DNA that can change its position within a genome. The transposon includes a transposase binding site and an RNA polymerase promoter sequence as described in US 10,894,980 entitled "Methods of Amplifying Nucleic Acid Sequences Mediated by Transposase/Transposon DNA Complexes" directed to a linear amplification via transposon insertion amplification process which may be referred to as "LIANTI".

The term "transposome" as used herein refers to the set of transposases bound to transposon DNA, which is a transposase/transposon DNA complex dimer.

The term "first strand cDNA synthesis" as used herein refers to forming the single-stranded DNA by reverse transcribing the RNA transcript of the original DNA fragments.

The term "second strand DNA synthesis" as used herein refers to forming a complementary strand to the first strand cDNA after RNA removal by RNase digestion.

The term "allele type" as used herein refers to the specific variant of a gene at a particular location (locus) on a chromosome. Different alleles may have different effects on traits or diseases.

The term "allele depth" as used herein refers to the number of reads (sequences) obtained for a specific allele at a given position. It indicates the abundance of that allele in the sample.

The term "single nucleotide polymorphism (SNP)" as used herein refers to a variation in a single nucleotide base pair within a DNA sequence that occurs among members of a species. SNPs can influence traits, susceptibility to diseases, and other genetic characteristics.

The term "SNP Density" as used herein refers to the number of obtained SNPs in a specific region of the genome from experimental data, divided by the number of all human common SNPs in the database with minor allele frequency larger than 0.01.

The term "haplotype" as used herein refers to a combination of alleles (genetic variants) at multiple loci on the same chromosome that are inherited together due to their physical proximity.

The term "haplotype status" as used herein refers to whether, at a specific SNP location, the allele inherited from the paternal or maternal parent is absent in the experimental data.

The term "haplotype loss" as used herein refers to the situation where certain alleles in a haplotype are lost in the obtained experimental sample.

The term "mapping" as used herein refers to aligning sequence reads to a reference genome to determine their locations.

The term "SNP calling" as used herein refers to the process of identifying SNPs from sequencing data.

The term "phasing" as used herein refers to the process of distinguishing between the paternal and maternal chromosomes within the sequencing sample from any individual or embryo. This differentiation allows the determination of which specific alleles come from each parent. It is important for understanding the inheritance pattern of genetic variants accurately, and understand how alleles are inherited together in haplotypes.

The term "pre-phasing" as used herein refers to phasing the haplotypes of parents before the disease carrying analysis is performed on the biological sample.

The term "recombination probability" as used herein refers to the probability that genetic recombination (exchange of genetic material) will occur between two specific loci on a chromosome during meiosis.

The term "recursive algorithm" as used herein refers to a method that solves a problem by repeatedly applying the same procedure until convergence.

A recitation of "a", "an" or "the" is intended to mean "one or more" unless specifically indicated to the contrary.

## WET LAB

In one aspect, the present disclosure refers to a method for amplifying the DNA in a solution, wherein the DNA content in the solution can be at the minimum of picogram amounts, the method comprising the following steps:

1) sample lysis: adding lysis buffer into the solution and heating, then adding protease and incubating to fully exposed the DNA, followed by inactivation under high temperatures to obtain the lysate;
2) DNA amplification:

a. transposome having a transposase and a transposon including a transposase binding site and an RNA polymerase promoter sequence is added into the lysate and inserted into the DNA fragment;
b. a 9-bp gap resulting from Tn5 transposon insertion is filled and extended down to both ends of each fragment;
c. performing in vitro transcription using the original sequence as a template;
d. adding DNA primer into a reverse transcription system to form a first strand cDNA via reverse transcription, wherein the DNA primer is complementary to the respective RNA strand of the sequence of the Tn5 transposition binding site; and
e. forming a second strand DNA via cycling amplification.

The transposon-based amplification methods are based on those described in US 10,894,980.

According to one general aspect, a transposition system having an RNA polymerase promoter sequence is combined with an RNA polymerase and reverse transcriptase along with a DNA primer to form a first strand cDNA via reverse transcription, wherein the DNA primer is complementary to the respective RNA strand of the sequence of the Tn5 transposition binding site. According to one aspect, transposons are inserted into DNA within the sample. An RNA polymerase is used to make RNA amplicons which are then reverse transcribed along with the DNA primer into DNA. Complements to the DNA are made and double stranded genomic DNA sequences are formed.

According to one aspect, a method for amplifying double stranded DNA is provided which includes contacting the DNA with transposases (such as TN5 transposases) each bound to a transposon DNA, wherein the transposon DNA includes a double-stranded transposase (Tnp) binding site and an RNA polymerase promoter sequence to form a transposase/transposon DNA complex dimer called a transposome. The transposon DNA may be in the form of a single stranded extension or in the form of a loop with each end connected to a corresponding strand of the double stranded transposase binding site. The transposome bind to DNA target locations along the double stranded DNA and cleave the double stranded DNA into a plurality of double stranded fragments, with each double stranded fragment having a first complex attached to an upper strand by the Tnp binding site and a second complex attached to a lower strand by the Tnp binding site. The transposon binding site is attached to each 5' end of the double stranded fragment. According to one aspect, the transposases are removed from the complex. The double stranded fragments are extended along the transposon DNA to make a double stranded extension product having T7 promoters at each end. According to one aspect, a gap which may result from attachment of the transposase binding site to the double stranded genomic DNA fragment may be filled and extended. The double stranded extension product is contacted with RNA polymerase (such as T7 RNA polymerase) to make an RNA transcript of the double stranded extension product. According to one aspect, a plurality of RNA transcripts of the double stranded extension product are made using RNA polymerase. The RNA transcripts are reverse transcribed into a plurality of corresponding single stranded DNA using a reverse transcriptase and a DNA primer complementary to the respective RNA strand of the sequence of the Tn5 transposition binding site. Complementary strands to the single stranded DNA are created to form a plurality of double stranded DNA. The double stranded DNA may then be amplified and sequenced using, for example, high-throughput sequencing methods known to those of skill in the art.

According to certain aspects, an exemplary transposon system is a Tn5 transposon system. Other useful transposon systems are known to those of skill in the art and include Tn3 transposon system (see Maekawa, T., Yanagihara, K., and Ohtsubo, E. (1996), A cell-free system of Tn3 transposition and transposition immunity, Genes Cells 1, 1007-1016), Tn7 transposon system (see Craig, N.L. (1991), Tn7: a target site-specific transposon, Mol. Microbiol. 5, 2569-2573), Tn10 tranposon system (see Chalmers, R., Sewitz, S., Lipkow, K., and Crellin, P. (2000), Complete nucleotide sequence of Tn10, J. Bacteriol 182, 2970-2972), Piggybac transposon system (see Li, X., Burnight, E.R., Cooney, A.L., Malani, N., Brady, T., Sander, J.D., Staber, J., Wheelan, S.J., Joung, J.K., McCray, P.B., Jr., et al. (2013), PiggyBac transposase tools for genome engineering, Proc. Natl. Acad. Sci. USA 110, E2279-2287), Sleeping beauty transposon system (see Ivics, Z., Hackett, P.B., Plasterk, R.H., and Izsvak, Z. (1997), Molecular reconstruction of Sleeping Beauty, a Tc1-like transposon from fish, and its transposition in human cells, Cell 91, 501-510), Tol2 transposon system (seeKawakami, K. (2007), Tol2: a versatile gene transfer vector in vertebrates, Genome Biol. 8 Suppl. 1, S7.)

According to certain aspects, an exemplary RNA polymerase is T7 RNA polymerase. Other useful RNA polymerases are known to those of skill in the art and include T3 RNA polymerase (see Jorgensen, E.D., Durbin, R.K., Risman, S.S., and McAllister, W.T. (1991) Specific contacts between the bacteriophage T3, T7, and SP6 RNA polymerases and their promoters, J. Biol. Chem. 266, 645-651), and SP6 RNA polymerase (see Melton, D.A., Krieg, P.A., Rebagliati, M.R., Maniatis, T., Zinn, K., and Green, M.R. (1984) Efficient in vitro synthesis of biologically active RNA and RNA hybridization probes from plasmids containing a bacteriophage SP6 promoter, Nucleic Acids Res. 12, 7035-7056.)

According to one aspect, transposon DNA is designed to contain a double-stranded 19 bp Tn5 transposase (Tnp) binding site at one end, linked or connected, to a strong T7 promoter sequence. Upon transposition, the Tnp and the transposon DNA bind to each other and dimerize to form transposomes. The transposomes then randomly capture or otherwise bind to DNA in the sample. Then, the transposases in the transposome cut the DNA with one transposase cutting an upper strand and one transposase cutting a lower strand to create a DNA fragment. The transposon DNA is thus inserted randomly into the DNA, leaving a 9-bp gap on both ends of the transposition/insertion site. The result is a DNA fragment with a transposon DNA Tnp binding site attached to the 5' position of an upper strand and a transposon DNA Tnp binding site attached to the 5' position of a lower strand.

After transposition, gap extension is performed to fill the 9 bp gap and complement the T7 promoter sequence originally designed in the transposon DNA. As a result, active double-stranded T7 promoter sequences are attached to both ends of each DNA fragment. Next, T7 RNA polymerase is added, and in vitro transcription is used to linearly amplify the DNA fragments, generating many RNAs that contain the same sequence as the original double stranded DNA template. Finally by reverse transcription using a reverse transcriptase and a DNA primer as described above to form DNA follwed by second strand synthesis, the amplified RNAs are converted back into double-stranded DNA molecules. The DNA fragments may then be further processed for standard library preparation, amplification and sequencing.

Particular Tn5 transposition systems are described and are available to those of skill in the art. See Goryshin, I.Y. and W.S. Reznikoff, Tn5 in vitro transposition. The Journal of biological chemistry, 1998. 273(13): p. 7367-74; Davies, D.R., et al., Three-dimensional structure of the Tn5 synaptic complex transposition intermediate. Science, 2000. 289(5476): p. 77-85; Goryshin, I.Y., et al., Insertional transposon mutagenesis by electroporation of released Tn5 transposition complexes. Nature biotechnology, 2000. 18(1): p. 97-100 and Steiniger-White, M., I. Rayment, and W.S. Reznikoff, Structure/function insights into Tn5 transposition. Current opinion in structural biology, 2004. 14(1): p. 50-7. Kits utilizing a Tn5 transposition system for DNA library preparation and other uses are known. See Adey, A., et al., Rapid, low-input, low-bias construction of shotgun fragment libraries by high-density in vitro transposition. Genome biology, 2010. 11(12): p. R119; Marine, R., et al., Evaluation of a transposase protocol for rapid generation of shotgun high-throughput sequencing libraries from nanogram quantities of DNA. Applied and environmental microbiology, 2011. 77(22): p. 8071-9; Parkinson, N.J., et al., Preparation of high-quality next-generation sequencing libraries from picogram quantities of target DNA. Genome research, 2012. 22(1): p. 125-33; Adey, A. and J. Shendure, Ultra-low-input, tagmentation-based whole-genome bisulfite sequencing. Genome research, 2012. 22(6): p. 1139-43; Picelli, S., et al., Full-length RNA-seqfrom single cells using Smart-seq2. Nature protocols, 2014. 9(1): p. 171-81 and Buenrostro, J.D., et al., Transposition of native chromatin for fast and sensitive epigenomic profiling of open chromatin, DNA-binding proteins and nucleosome position. Nature methods, 2013. See also WO 98/10077, EP 2527438 and EP 2376517. A commercially available transposition kit is marketed under the name NEXTERA and is available from Illumina.

In vitro transcription (IVT) by T7 RNA polymerase is useful to create RNA amplicons. See Van Gelder, R.N., et al., Amplified RNA synthesized from limited quantities of heterogeneous cDNA. Proceedings of the National Academy of Sciences of the United States of America, 1990. 87(5): p. 1663-7; Kawasaki, E.S., Microarrays and the gene expression profile of a single cell.

Annals of the New York Academy of Sciences, 2004. 1020: p. 92-100; Livesey, F.J., Strategies for microarray analysis of limiting amounts of RNA. Briefings in functional genomics & proteomics, 2003. 2(1): p. 31-6; Tang, F., K. Lao, and M.A. Surani, Development and applications of single-cell transcriptome analysis. Nature methods, 2011. 8(4 Suppl): p. S6-11; Hashimshony, T., et al., CEL-Seq: single-cell RNA-Seq by multiplexed linear amplification. Cell reports, 2012. 2(3): p. 666-73; and Shankaranarayanan, P., et al., Single-tube linear DNA amplification for genome-wide studies using a few thousand cells. Nature protocols, 2012. 7(2): p. 328-38. According to the present disclosure, IVT advantageously provides linear amplification, with all the copies generated from the original DNA template. The resulting RNA molecules can be reverse transcribed into single stranded DNA followed by complementary strand formation resulting in double stranded DNA, which are amplicons linearly amplified from the original DNA template.

In one instance, the solution is a culture medium or a blastocoele fluid. Particularly, the culture medium or the blastocoele fluid has high protein content and low input amount of DNA. According to one aspect described below, the method is applied to culture medium as merely exemplary.

In one instance, the lysis buffer is 2x to 10x lysis buffer. The original volume of the culture medium is much larger than that for a single cell. The amplification of as much proportion of the culture medium as possible was achieved by using a very small amount of highly concentrated lysis buffer. This results in the largest percentage amount of culture medium DNA to be used as a template in this amplification system.

In one instance, the working concentration of the protease in the lysis mixture is higher than 0.6 mg/mL, preferably in the range of 0.8-2 mg/mL. For example, the working concentration of the protease is 0.6 mg/mL, 0.8 mg/mL, 1 mg/mL, 1.2 mg/mL, 1.4 mg/mL, 1.6 mg/mL, 1.8 mg/mL, 2 mg/mL or higher. The amount of protease is increased to remove the extra exogenous serum proteins.

In one instance, the incubation is performed at 45-60°C for 1 to 3 hours.

In one instance, the inactivation is performed at 80-90°C. The protease reaction time is shortened and the protease inaction temperature is increased to ensure that there is no excess protease residue in the subsequent reaction.

In one instance, the working concentration of the transposome is higher than 6 nM, preferably at the range of 10-40 nM. For example, the working concentration of the transposome is 6 nM, 6.25nM, 8 nM, 10 nM, 12.5 nM, 14 nM, 16 nM, 18.75 nM, 20 nM, 22 nM, 24 nM, 26 nM, 28 nM, 30 nM, 32 nM, 34 nM, 36 nM, 37.5 nM, 40 nM or higher.

In one instance, the DNA primer has a length of 15bp to 25bp. Preferably, the 3'-end of the DNA primer contains the sequence of 5'-GACAG-3' or 5'-CTGTC-3'. For example, the random primer 5'-NNNNNNNNNNNNNNNGACAG-3' or 5'-NNNNNNNNNNNNNNNCTGTC-3' can be used. More preferably, the DNA primer contains the sequence set forth as 5'-AGATGTGTATAAGAGACAG-3' (Seq. ID No.: 1), or 5'-TCTACACATATTCTCTGTC-3' (Seq. ID No.: 2), or has at least 70%, preferably 80%, preferably 90%, preferably 95, preferably 100% identity with Seq. ID No.: 1 or Seq. ID No.: 2. For example, primer 5'-AGATGTGTATAAGAG-3' can be used.

The distribution of the DNA in the culture medium was measured and it was determined that both long and short fragments of cell-free DNA were present in the culture medium. Since current commercialized kits all tend to amplify the long fragments, transposon-based amplification methods described herein based on those described in US 10,894,980 as modified herein are designed to amplify both long (e.g., greater than 1kb, in some instances) and short fragments (e.g., 100bp~600bp, or less than 1kb, in some instances). The transposon amplification methods described herein do not use self-priming and use a DNA primer added with proper concentration during the reverse transcription step for the highest efficiency. The RNAs generated in *in vitro* transcription are specialized by a sequence of complete transposon DNA sequence at the 3' end.

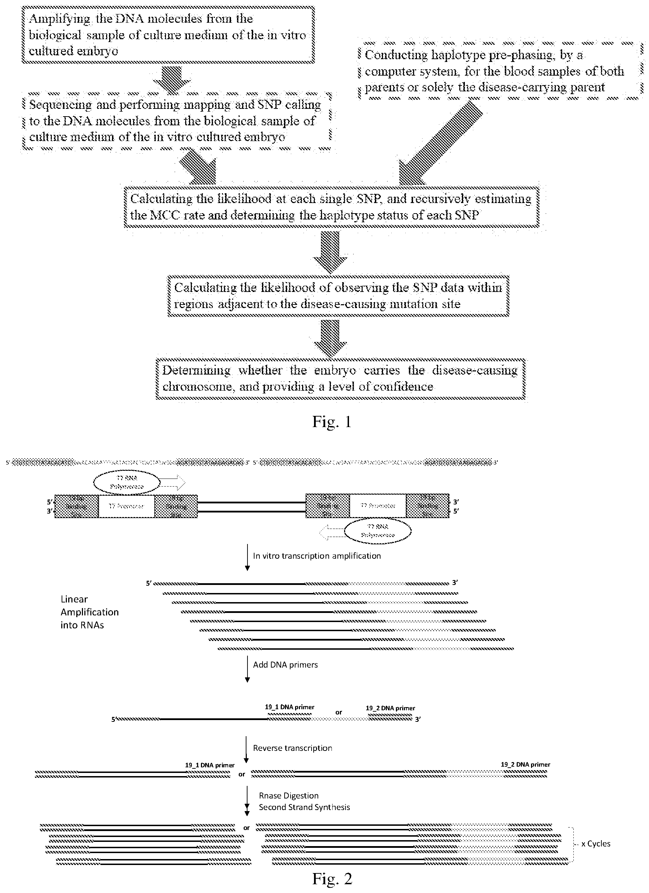

Two primers designed for the reverse transcription, named 19_1 and 19_2, and their binding positions are shown in Fig. 2.

In one instance, the cycling amplification is performed more than 4 cycles, such as from 4 to 20 cycles, preferably 6 to 15 cycles. After the formation of the first strand of cDNA in reverse transcription, additional amplification cycles were used to amplify the second strand of DNA. Linear amplification, such as described in US 10,894,980 and C Chen, D Xing, L Tan, H Li, G Zhou, L Huang, XS Xie, Science 2017; 356 (6334): 189-194, is a high-precision amplification, however, the product yield of linear amplification is lower than exponential amplification. In order to facilitate clinical testing methods, the present disclosure provides methods that increase the yield of amplification. In one instance, this is accomplished during the reaction where the second strand is formed by increasing the number of the amplification cycles in order to obtain enough DNA for sequencing library preparation.

The methods disclosed herein achieve an amplification success rate of nearly 100% while keeping the fidelity for amplification.

## **DRY LAB**

In another aspect, the present disclosure refers to a method of analyzing a biological sample of culture medium of an in vitro cultured embryo to determine the susceptibility of a genetic disease in the embryo, comprising:

1) Amplifying the DNA molecules from the biological sample of culture medium of the in vitro cultured embryo and/or for reference only, the biological sample of a discarded embryo in the family;

2) Preparing DNA libraries and sequencing the DNA molecules from the biological sample to obtain a plurality of sequence reads;

3) Performing mapping and SNP calling, such as by a computer system, to generate the SNP data and controlling its quality;

4) Conducting haplotype pre-phasing, such as by a computer system, for the blood samples of both parents or solely the disease-carrying parent;

5) Calculating, such as by a computer system, the likelihood of the four possible inheritance scenarios at each single SNP representing whether the genetic chains are inherited from the paternal or maternal alleles of the parents, taking into account the maternal cell contamination (MCC) rate and haplotype status;

6) Estimating the MCC rate, such as by a computer system, and determining the haplotype status of each SNP to represent whether only DNA from one parent is present in the culture medium;

7) Calculating, such as by using a computer system, the likelihood of observing the SNP data within regions adjacent to the disease-causing mutation site, combining the likelihood at each single SNP and the recombination probabilities;

8) Determining whether the embryo carries the disease-causing chromosome, and providing a level of confidence.

In some instances disclosed herein, aforementioned step 5 and step 6 provide specific, technological improvements over some existing techniques that use SNP data to determine whether an embryo carries a disease-causing chromosome. For example, if the wet lab provides very high quality biological samples that result in highly precise and accurate SNP data without maternal contamination from the samples, then one or more computations performed in step 5 and step 6 might offer only marginal improvement in accuracy. For example, methods for amplifying (as recited in aforementioned step 1) minute amounts of DNA in a solution are sometimes used to improve the quality of resulting SNP data. Nevertheless, despite attempts at improving the quality (e.g., staying free of contaminants, amplifying, etc.) of biological samples, this is not always possible to the desired extent or level of confidence. Moreover, high ADO rates and maternal contamination are the intrinsic property of the DNA content in certain biological samples, such as a biological sample of culture medium from an in vitro cultured embryo, and are inevitable even if the wet lab can reach near-perfect performance. In contrast to prior art systems, the novel and nonobvious computations performed in step 5 and step 6 provide an improvement in the precision with which a biological sample of culture medium from an in vitro cultured embryo is analyzed to more accurately determine the susceptibility of a genetic disease in the embryo. Rather than being solely dependent on the wet lab and its known techniques to provide just very high quality biological samples for linkage analysis and/or non-invasive preimplantation genetic testing, this disclosure includes one or more additional computational operations performed in step 5 and/or step 6 to overcome various shortcomings in the prior art. In other words, prior art systems do not have a linkage-analysis method for a non-invasive procedure similar to the features disclosed herein-for example, several instances disclosed herein enable a markedly accurate linkage analysis method for even those samples with high ADO rates in the presence of maternal contamination. Steps 1 to 8 are further elaborated as follows.

Step 1): Amplifying the DNA molecules from the biological sample of culture medium of the in vitro culture embryo and/or, for reference only, the biological sample of a discarded embryo in the family

In one instance, the embryo has contamination caused by maternal cells and/or haplotype loss. The biological sample of a discarded embryo in the family is used for the following deduction of pre-phasing when neither a proband nor grandparents are present, which will be discussed in detail below.

Step 2): Preparing DNA libraries and sequencing the DNA molecules from the biological sample to obtain a plurality of sequence reads.

The amplicons were sheared and library preparation was performed. The DNA sequencing libraries were sequenced on an Illumina platform to generate raw sequencing data.

Step 3): Performing mapping and SNP calling, by a computer system, to generate the SNP data and controlling its quality.

Raw paired-end reads were trimmed Illumina sequencing adapters, inline barcodes, binding sequences and T7 promoter sequences, and low-quality ends by cutadapt. Clean reads were mapped to human reference genome hg19 with BWA mem. Each BAM file was marked duplicates and sorted by MarkDuplicatesSpark of GATK 4.2.0. Base quality score recalibration (BQSR) was performed using BaseRecalibrator to generate a recalibration table, followed by ApplyBQSR to adjust the base quality accordingly. For variant calling, GATK HaplotypeCaller produced a genomic variant call format (GVCF) file and VCF file for each sample. GenotypeGVCFs was utilized to perform joint genotyping across all samples. SNPs were filtered using variant quality score recalibration (VQSR) with HapMap 3.3, Omni 2.5, 1000 Genome phase I, and dbSNP 151 as SNP training sets. A 99% sensitivity threshold was chosen to filter SNPs accurately. Biallelic SNPs were retained for subsequent analysis. The VCF contains the chromosome number, the physical location, identification number (ID), and allele type as well as the sequencing quality, sequencing depth (DP, Depth), and allele depth (AD) for each SNP of each blood sample, the biological sample of the culture medium of the in vitro cultured embryo and/or the biological sample of the discarded embryo in the family.

SNPs with two different alleles are considered and all SNPs with DP values less than 5, or sequencing quality values (QUAL) less than 30, or gene quality (GQ) values less than 10 in the blood samples are excluded, because they are considered to be of too poor quality and too small signal-to-noise ratio.

After removing the low-quality SNPs, 10MB (Million Base pairs) upstream and downstream of the disease-causing mutation site in the biological sample is defined as the Adjacent Region of the disease-causing mutation site. The Sequencing Error Rate (SER) in the Adjacent Region is estimated. SNPs with genotype 00 of both father and mother, or genotype 11 of both father and mother are selected. Ideally, at such SNPs, genotypes that are identical to the parents' genotype should only be detected. Therefore, sequencing data with genotypes different from those of the parents must be the wrong reads. Hence, the estimated Sequencing Error Rate (SER) is defined as the proportion of the total number of erroneous reads versus the total number of reads.

The relative density of SNPs detected in the adjacent region of the disease-causing mutation site is estimated, and defined as the ratio of the total number of SNPs detected in this area compared to the total number of human common SNPs within this region. Here, human common SNPs are defined as all SNPs with Minor Allele Frequency (MAF) greater than or equal to 0.01, and the Minor Allele Frequency of a group of alleles refers to the frequency of its second most frequent allele in a population. The human common SNP data updated in 2018 by dbSNP [Sherry et al., 2001] is used to calculate the relative SNP density for each sample.

After completing the above steps, the sample of culture medium with an abnormal MCC rate (>0.65 or <-0.5) or low SNP density (<0.001) or a high sequencing error rate (>0.2) is considered to be of extremely low quality and thus excluded. Such culture medium are categorized into "Undetermined" and the reasons as low data quality are outputted. All the samples passing the quality control will come into the next stage.

Step 4): Conducting: haplotype pre-phasing: by a computer system, for the blood samples of both parents or solely the disease-carrying parent.

To deduce parental haplotypes, genomic data of any one or more of the following samples in the family is used: the blood sample of proband, or the blood sample of at least one of the grandparents. When neither a proband nor grandparents are present, discarded embryos are used for reference only, for the deduction. Since discarded embryos, for reference only, are the samples after amplification, which will have errors in the process of amplification to be introduced, in order to ensure the accuracy of the deduction, a minimum of two discarded embryo data are used in parallel to perform the deduction and correct each other for Mendelian errors.

Plink 1.9 with the option "--mendel" is used to identify sites with Mendelian errors. Additionally, BEAGLE 4.0 is used to phase the genotype data, taking into account the parents-offspring relationships and simultaneously eliminating sites that contain Mendelian errors.

Step 5): Calculating the likelihood of the four possible inheritance scenarios at each single SNP representing whether the genetic chains are inherited from the paternal or maternal alleles of the parents, taking into account the maternal cell contamination (MCC) rate and haplotype status.

For the single-SNP likelihood, the binomial distribution is used to build a model (see the appendix section of Nabieva et al. [2020]), where the parameters of the binomial distribution used were the sequencing error rate $\widehat{SER}$, MCC rate $\hat{\alpha}$, and detected AD values of the two alleles at each single SNP. Specifically, the following

$$l_{11} = \left(\frac{1-\hat{\alpha}}{2}, 0, \frac{1}{2}, \frac{\hat{\alpha}}{2}\right)^{\top}, \quad l_{12} = \left(\frac{1-\hat{\alpha}}{2}, 0, \frac{\hat{\alpha}}{2}, \frac{1}{2}\right)^{\top},$$

$$l_{21} = \left(0, \frac{1-\hat{\alpha}}{2}, \frac{1}{2}, \frac{\hat{\alpha}}{2}\right)^{\top}, \quad l_{22} = \left(0, \frac{1-\hat{\alpha}}{2}, \frac{\hat{\alpha}}{2}, \frac{1}{2}\right)^{\top}.$$

defines as the theoretical proportion of four chromosomes (paternal inherited chromosome, paternal noninherited chromosome, maternal inherited chromosome, maternal non-inherited chromosome) in embryonic culture medium. To take LFLoH(See below for definition) into consideration, the two formulae below

$$h(\mathrm{H}) = \begin{cases} (1,1,0,0)^{\top} & \text{If } \mathrm{H} = \mathrm{F} \\ (0,0,1,1)^{\top} & \text{If } \mathrm{H} = \mathrm{M} \\ (1,1,1,1)^{\top} & \text{If } \mathrm{H} = \mathrm{FM} \text{ or } H = \mathrm{Unknown} \\ (1,0,0,0)^{\top} & \text{If } \mathrm{H} = \mathrm{F}_1 \\ (0,1,0,0)^{\top} & \text{If } \mathrm{H} = \mathrm{F}_2 \end{cases}$$

And

$$\widehat{l}_{ij} = \frac{l_{ij} \circ h\left(H_n\right)}{l_{ij}^{\top} h\left(H_n\right)}.$$

are used, where H is the status of haplotype at this SNP.

Here, ∘ denotes product by position. Paternal genotype at the n-th SNP is denoted as fGT = fGT1/fGT2 and the maternal one as mGT = mGT1/mGT2, where fGT1, fGT2,mGT1,mGT2 $\in$ {0, 1}. The following

$$GT_n = (fGT_1, fGT_2, mGT_1, mGT_2)^T,$$

is denoted as a four-dimensional vector and denotes

$$\hat{p}_{ij}^{(n)} = \widehat{l}_{ij}^{\top} \cdot GT_n,$$

then $\hat{p}_{ij}^{(n)}$ is the theoretical probability that an ALT gene is obtained by randomly sampling one read at n-th SNP in the culture medium when MCC rate and haplotype loss are taken into consideration. Therefore, the single-SNP likelihood is formulated as

$$\hat{L}_{ij}^{(n)} = \frac{AD_n!}{AD_{0,n}! \cdot AD_{1,n}!} \cdot \left[\hat{p}_{ij}^{(n)} \widehat{SER} + \left(1 - \hat{p}_{ij}^{(n)}\right)\left(1 - \widehat{SER}\right)\right]^{AD_{0,n}}$$
$$\cdot \left[\hat{p}_{ij}^{(n)}\left(1 - \widehat{SER}\right) + \left(1 - \hat{p}_{ij}^{(n)}\right)\widehat{SER}\right]^{AD_{1,n}} \quad \text{for } i, j \in \{1, 2\}$$

In some instances, the same computer system may performs all of the steps of the method for determining the susceptibility of a genetic disease in the embryo. In other instances, one or more distinct computer processors in a computer system (e.g., computer subsystems) may perform one or more of the steps of the aforementioned method without necessarily performing all of them. In other words, the computer system may comprise multiple subsystems, each with its own computer processor, so that the computational load of performing the numerous steps recited in the aforementioned method is distributed.

Step 6): Estimating the MCC rate, and determining the haplotype status of each SNP to represent whether only DNA from one parent is present in the culture medium.

Step 6) further includes the following steps:

Step (a): Initially estimating the MCC rate and haplotype status of each SNP in the region adjacent to the disease-causing mutation site.

Maternal Cell Contamination rate (MCC rate) is defined as the proportion of maternal DNA in the culture medium of an embryo compared to the total amount of DNA and this value should theoretically be between 0 and 1. All SNPs with paternal genotype 00 and maternal genotype 11, or with paternal genotype 11 and maternal genotype 00 in the adjacent region are selected. At these SNPs, it is determined theoretically whether each read detected in the culture medium is from the father or the mother, and the proportion of reads from the mother over all reads is calculated and used as an initial estimate of MCC rate. In contrast to prior art systems which omit a linkage-analysis method for a non-invasive procedure similar to the features disclosed herein, this disclosure provides for various computational operations on SNP data that enable a more accurate linkage analysis method even for samples with high ADO rates in the presence of maternal contamination, as explained herein. In particular, in wet labs using non-invasive procedures, poor data quality can sometimes occur because missing DNA fragments from one parent are often longer in length and contain dozens or more SNPs, thus the ability to sequence is negatively effected and diminished. The computational operations disclosed herein solve a real world, technological problem by enabling an otherwise unusable biological sample to become useful to determine the susceptibility of a genetic disease in an embryo.

In some examples, a method of preparation is disclosed herein to prepare an otherwise unsequenceable biological sample of culture medium of an in vitro cultured embryo where the underlying biological sample is low quality for, among other things, having high ADO rates in the presence of maternal contamination. The preparation method transforms the SNP data resulting from the otherwise unsequenceable biological sample into a state that is capable of being analyzed to determine a susceptibility of a genetic disease in the embryo. For purposes of determining the susceptibility of a genetic disease in an embryo, low quality DNA samples result in mostly unsequencable data. Moreover, DNA in embryos enter the culture medium in fragments, so if certain longer DNA fragments do not enter the culture medium, it results in the inability of sequencing (e.g., a phenomenon defined as Large Fragment Loss of Haplotypes (LFLoH) herein). The present disclosure describes a method of preparation of the seemingly unsequencable data into a transformed state where the resulting SNP data overcomes the shortcomings of LFLoH, and the previously unusable biological sample is now rendered usable. Because the resulting SNP data from the wet lab is based on a biological sample with less than ideal quality due to aforementioned circumstances, novel and nonobvious computational steps are performed on the SNP data to transform the seemingly unusable data into an usable one; in some instances, particularly for the purposes of determing whether an embryo awaiting implantation carries the disease-carrying chromosome or not. Because of the innovative computational steps disclosed herein, another round of sequencing in the wet lab may be avoided, thus saving on time, supplies, labor, and other resources. As elaborated below in step 6(b), analyzing LFLoH provides insights into whether parental DNA fragments are detected at each SNP, which may result in the final haplotype status labels at all SNPs to be iternatively updated to be more accurate until the MCC rate converges or other final estimation criterion is reached.

Notably, the aforementioned method of preparation is different from a method of treatment. The method of preparation recited herein is more than simply identifying a natural correlation between two naturally occurring properties of biological samples. For example, simply using the natural correlation between heightened cfDNA levels in a collected bodily sample and organ transplant health, to identify a potential organ rejection might not be sufficient to recite patent eligible subject matter. In contrast, in some examples disclosed herein, the computational method disclosed in step 5 and/or step 6 enriches a previously unusable biological sample into a transformed state that provides to-be-parents with an accurate, non-invasive verification of the susceptibility of a genetic disease in their embryo. By avoiding invasive procedures such as sticking a big needle into the amniotic sac of a pregnant woman, a number of deaths of to-be-mothers and fetuses can be avoided. The disclosed method of preparation provides a technical solution to a real world problem that previously resulted in higher risk to fetuses, women, and their health.

Ideally, it is advantageous to be able to detect DNAs from both parents in the embryo culture medium. However, in practice, in embryo culture medium, it has been observed that a significant portion of the genome can only be detected from one parent. Such a type of DNA loss is different from traditional sense of Allele Drop-Out (ADO), which arises due to amplification heterogeneity, resulting in some short DNA fragments that are not successfully amplified in a certain round of amplification, and thus are amplified significantly less than other DNA fragments to be detected. It reflects the efficiency of amplification methods, and it involves DNA fragments that are usually short and contain only about 1 or 2 SNPs. However, in the culture medium samples obtained by non-invasive methods, the missing DNA fragments from one parent is often longer in length and contain dozens or more SNPs. Such haplotype loss is not due to low amplification efficiency, but rather due to poor data quality. DNA in embryos enters the culture medium in fragments, so if certain longer DNA fragments do not enter the culture medium, it results in the inability of sequencing. We define Large Fragment Loss of Haplotypes (LFLoH) as the phenomenon that long and contiguous DNA fragments from one or both parents do not enter the culture medium and thus cannot be detected in sequencing. The present disclosure provides a model that addresses LFLoH in a two-step process.

First, SNPs that can be directly determined from their AD values are labeled as either containing only paternal DNA (F), only maternal DNA (M), or both (FM) Others remain unlabeled.

In the second step, the surrounding SNPs in the upstream and downstream of these labeled SNPs are searched, and it is then determined whether the surrounding SNPs could be labeled with the same label as the labeled SNP in the first step. Specifically, in the first step, T is assumed to be an integer threshold determined in advance (the default value is 3).

For SNP in the adjacent region of the disease-causing mutation site, those satisfying one of the following conditions are labeled as F.

- The paternal genotype is 00 and the maternal genotype is 11. $AD_0 > T$ and $AD_1 = 0$.
- The paternal genotype is 11 and the maternal genotype is 00. $AD_1 > T$ and $AD_0 = 0$.
- The paternal genotype is 01 and the maternal genotype is 00. $AD_0 = 0$ and $AD_1 > T$.
- The paternal genotype is 01 and the maternal genotype is 11. $AD_0 > T$ and $AD_1 = 0$.

Some SNPs labeled as F can be further label as F1 or F2, indicating that the detected SNP is from the paternal source chromosome or the maternal source chromosome of the father. Specifically, SNP labeled F is labeled as F1, if the SNP satisfies one of the following.

- The paternal genotype is 1|0 and the maternal genotype is 00. $AD_1 > T$ and $AD_0 = 0$.
- The paternal genotype is 0|1 and the maternal genotype is 11. $AD_0 > T$ and $AD_1 = 0$,

where 0|1 and 1|0 denote the paternal genotype, and the number at left hand side and right hand side of | denote the paternal source and maternal source gene of the father at this SNP respectively.

Similarly, a SNP labeled F is labeled as F2, if the SNP satisfies one of the following two conditions.

- The paternal genotype is 0|1 and the maternal genotype is 00. $AD_1 > T$ and $AD_0 = 0$.
- The paternal genotype is 1|0 and the maternal genotype is 11. $AD_0 > T$ and $AD_1 = 0$,

SNPs satisfying one of the following conditions arelabeled as M.

- The paternal genotype is 00 and the maternal genotype is 11. $AD_1 > T$ and $AD_0 = 0$.
- The paternal genotype is 11 and the maternal genotype is 00. $AD_0 > T$ and $AD_1 = 0$.

Due to the existence of MCC, no SNP is labeled as M1 or M2. SNPs satisfying one of the following conditions are labeled as FM.

- The paternal genotype is 00 and the maternal genotype is 11. $AD_1 > [T/2]$ and $AD_0 > [T/2]$;
- The paternal genotype is 00 and the maternal genotype is 11. $AD_1 > [T/2]$ and $AD_0 > [T/2]$;
- The paternal genotype is 01 and the maternal genotype is 11. $AD_1 > [T/2]$ and $AD_0 > [T/2]$;
- The paternal genotype is 01 and the maternal genotype is 00. $AD_1 > [T/2]$ and $AD_0 > [T/2]$; where [] denotes the floor function.

After completing the initial labeling procedure, there are 5 possible labels: F, M, FM, F1, F2. Suppose there are several labeled SNPs among the adjacent region of the disease-causing mutation site, with an ordinal number $-N_1 \leq s_1 < s_2 < ... < s_D \leq N_2$. Then, for a particular SNP $s_d$ that has been labeled, a search is carried out between the SNPs with ordinal number $s_d$-1 and $s_d$+1. The $s_d$-th SNP is called the central SNP and the region between $s_d$-1-th and $s_d$+1-th SNP is called the searching region. The search region is divided into two parts: the upstream part and the downstream part of the $s_d$-th SNP. In the downstream searching, each SNP in the downstream searching region is assigned a label. The n-th SNP during downstream searching from its nearest labeled SNP upstream from it is denoted as $H_n^{down}$ and during upstream searching from its nearest labeled SNP downstream from it is denoted as $H_n^{up}$ .

The search process only in the downstream region is described as follows as an example. First, $H_{s_d}^{down}$ is initialized as the label assigned in the first labeling step. Next, assume $s_d$, $s_d$ +1, ..., $s_d$ +m-1 are all labeled the same as $H_{s_d}^{down}$ . The next step is to search the $(s_d + m)$-th SNP and determine whether the same label can be assigned to this SNP. The same label is assigned to $(s_d + m)$-th SNP if and only if

$$\frac{\sum_{i,j=1}^{2} \mathbb{P}\left(AD_{s_d:s_d+m} \middle| F_{s_d} = i, M_{s_d} = j, H_{s_d:s_d+m}^{down} = H_{s_d}^{down}\right)}{\sum_H \sum_{i,j=1}^{2} \mathbb{P}\left(AD_{s_d:s_d+m} \middle| F_{s_d} = i, M_{s_d} = j, H_{s_d:s:d+m-1}^{down} = H_{s_d+m}^{down} = H\right)} \geq \lambda_0.$$

Here, $H_{s_d:s_d+m}^{down} = H_{s_d}^{down}$ denotes $H_l^{down} = H_{s_d}^{down}$, $\forall l = s_d$, $s_d + 1$, ..., $s_d + m$, and $\lambda_0$ is a pre-determined threshold. H in the summation refers to summing over H =F, M, FM, F1,F2 and $AD_{s_d:s_d+m}$ denotes $\{AD_{s_d}, AD_{s_d+1}, ..., AD_{s_d+m}\}$ . $P(\cdot \mid \cdot)$ refers to the conditional probability, specifically, the probability of the observed AD values at the $s_d$-th SNP, conditioned on the knowledge of the inherited chromosome from both parents at the $s_d$-th SNP as well as the labels of nearby SNPs. The conditional probability is calculated in exactly the same way as the recursion elaborated on in the next section. For each central SNP, the hyplotype state for at most 10 SNPs upstream or downstream is inferred.

Finally, as a result of the searching procedure, there will be at most two labels at each SNP. For those SNPs with less than two labels, a label is added as "Unknown". Then, the two labels of each SNP are merged to finalize the labeling procedure of LFLoH. According to the principle of final labeling, a SNP is labeled as F or M only after sufficience confidence that only paternal or maternal DNA fragments are detected, otherwise the SNP is labeled as FM. Specifically, the final label of the n-th SNP is

$$H_n = \begin{cases} F & \text{If } \{H_n^{\text{up}}, H_n^{\text{down}}\} = \{F^1\} \text{ or } \{F^1, F\} \text{ or } \{F^1, FM\} \\ & \quad \text{or } \{F^1, \text{Unknown}\} \text{ or } \{F_2\} \text{ or } \{F^2, F\} \text{ or } \{F^2, \text{Unknown}\} \\ M & \text{If } \{H_n^{\text{up}}, H_n^{\text{down}}\} = \{M\} \text{ or } \{M, \text{Unknown}\} \\ FM & \text{Otherwise} \end{cases}.$$

Step (b): Recursively updatine the MCC rate and haplotype status of each SNP

The initial calculation of MCC rate in previous sections is performed based on SNPs with homozygous but different parental genotypes, and it is assumed that both paternal and maternal DNA fragments are detected at these SNPs. However, analyzing Large Fraction Loss of Haplotype (LFLoH) can provide new insights into whether parental DNA fragments are detected at each SNP, which can facilitate a re-estimation of MCC rate. With the new estimate of MCC rate, the likelihood will change and the final labels at all SNPs should be updated. Since the labels at SNPs are closely entangled with the MCC rate estimate, and both cannot be estimated at the same time, an iterative estimation method is adopted, whereby the estimate of each one is updated with the present estimate of the other one and this process is repeated until convergence. Under each stage, the MCC rate is first estimated using all SNPs labeled as FM, and then the LFLoH for each SNP is estimated using the new MCC rate estimate. This process is repeated until the difference between two adjacent estimates of MCC rate does not exceed a threshold ( set to be 0.1 or 0.05) and the estimate in the final iterate is used as the final estimate of MCC rate and the corresponding LFLoH states are adopted as the final labels of haplotype status. If the MCC rate estimates do not converge after 10 rounds of iteration, the average of the 10 MCC rate estimates is then taken as the final estimate, and the haplotype status is updated using this estimate.

At least one advantage of the recursive procedure described herein is that it reduces the calculation of the likelihood function to the calculation of the recombination probability and the single-SNP likelihood. The computational load on the processors in the computer system are improved because of the technological advancement resulting from the recursive procedure. In addition to a reduced processor load, the recursive procedure may result in fewer memory usage because of the consolidation equations being calculated. The recursive procedure described herein is novel and nonobvious, and is not well understoond, routine, and conventional.

Step 7: Calculating the likelihood of observing the SNP data within regions adjacent to the disease-causing mutation site, combining the likelihood at each single SNP and the recombination probabilities.

The present disclosure provides a method of recursively calculating likelihood. Firstly, if the disease-causing mutation site is on the paternal chromosome, all SNPs whose final haplotype status is labeled as F or FM and where the paternal genotype is heterozygous are selected for recursion. If the disease-causing mutation site is on the maternal chromosome, all SNPs whose final haplotype status is labeled M or FM and where the maternal genotype is heterozygous are selected. The disease-causing mutation site is still denoted as the 0-th SNP. Among all retained SNPs downstream of it have ordinal numbers 1, 2, ..., $N_2$, while the upstream SNPs have ordinal numbers -1,-2, ... - $N_1$. For the i-th SNP, it is denoted

$$F_i = \begin{cases} 1 & \text{If the embryonic chromosome inherited from the father was actually from the grandfather} \\ 2 & \text{If the embryonic chromosome inherited from the father was actually from the grandmother} \end{cases},$$

$$M_i = \begin{cases} 1 & \text{If the embryonic chromosome inherited from the mother was actually from the grandfather} \\ 2 & \text{If the embryonic chromosome inherited from the mother was actually from the grandmother} \end{cases}$$

$AD_i$ is denoted as the Allele of Depth (AD) at the i-th SNP and $AD_{i,j} = \{AD_i, AD_{i+1}, ..., AD_{i+j}\}$ for i < j. Since given the genotype and haplotype labels at the disease-causing mutation site, the recombination and sequencing data in the upstream and downstream are conditionally independent, and the upstream and downstream analysis are dealt with separately. Here, formulating the recursion for the downstream region is further described. The probability that the parental embryonic chromosome is inherited from the grandfather or grandmother and the maternal embryonic chromosome is inherited from grandfather or grandmother is calculated, conditioned on the observation of all detected AD values and the inferenced haplotype status for SNPs within the adjacent region. Specifically, the goal is to calculate

$$\mathbb{P}\left(F_0 = i, M_0 = j \middle| AD_{0:N_2}, H_{0:N_2}\right) \text{ for } i, j \in \{1, 2\}.$$

The following Bayesian formula is utilized

$$\mathrm{P}\left(F_0 = i, M_0 = j | AD_{0:N_2}, H_{0:N_2}\right) \propto \mathrm{P}\left(F_0 = i, M_0 = j\right)\mathrm{P}\left(AD_{0:N_2} | F_0 = i, M_0 = j, H_{0:N_2}\right)$$

with non-informative prior:

$$\mathbb{P}\left(F_0 = i, M_0 = j\right) = \frac{1}{4} \quad \text{for } i, j \in \{1, 2\}.$$

The next step is to recursively calculate $P(AD_{0:N_2}|F_0 = i, M_0 = j, H_{0:N_2})$.

$$\mathrm{P}\left(AD_{n:N_2} | F_n = i, M_n = j, H_{n:N_2}\right)$$
$$= L_{ij}^{(n)} \sum_{k,l=1}^{2} \mathrm{P}\left(AD_{n+1:N_2} | F_{n+1} = k, M_{n+1} = l, H_{n+1:N_2}\right)\mathrm{P}(F_{n+1} = k, M_{n+1} = l | F_n = i, M_n = j)$$

The single-SNP likelihood is $L_{ij}^{(n)} = \mathrm{P}(AD_n | F_n = i, M_n = j, H_n)L_{ij}^{(N_2)}$ and the initial value is $L_{ij}^{(N_2)}$. $P(F_{n+1} = k, M_{n+1} = l | F_n = i, M_n = j)$ is the recombination probability.

The recursive procedure described above reduces the calculation of the likelihood function to the calculation of the recombination probability and the single-SNP likelihood. The estimated recombination probability between any two positions on the human genome is obtained from a high-precision database of recombination rates as described in Kong et al. [2002]. The single-SNP likelihood has been described in step (5).

Step 8): Determining whether the embryo carries the disease-causing chromosome, and providing a level of confidence.

After calculating the likelihood, it is determined which one of the father's two chromosomes is inherited to the embryo and the same for the mother's chromosome by calculating Log Likelihood Ratio (LLR). If the disease-causing mutation site is on the paternal chromosome, the following is defined

$$\mathrm{LLR} = \log\left(\frac{\sum_{j=1}^{2} \mathbb{P}\left(\mathrm{AD}_{0:N} \middle| \mathrm{F}_0 = 1, \mathrm{M}_0 = j\right)}{\sum_{j=1}^{2} \mathbb{P}\left(\mathrm{AD}_{0:N} \middle| \mathrm{F}_0 = 2, \mathrm{M}_0 = j\right)}\right) ;$$

otherwise we defined

$$\mathrm{LLR} = \log\left(\frac{\sum_{i=1}^{2} \mathbb{P}\left(\mathrm{AD}_{0:N} \middle| \mathrm{F}_0 = i, \mathrm{M}_0 = 1\right)}{\sum_{i=1}^{2} \mathbb{P}\left(\mathrm{AD}_{0:N} \middle| \mathrm{F}_0 = i, \mathrm{M}_0 = 2\right)}\right) .$$

A positive LLR indicates that the data considered favors that the allele is inherited from the father of the disease-carrying parent. Otherwise, the data supports that it is inherited from the mother of the disease-carrying parent.

However, it is recognized that how many SNPs to use for the calculation of likelihood cannot be determined in advance. If a certain fixed number of SNPs are used for the calculation, or if all SNPs within a certain fixed physical distance to the disease-causing mutation site are used for the calculation, it cannot be determined with certainty whether the information implied by these SNPs is enough to support conclusions and to what extent the likelihood varies with the number of used SNPs.

Therefore, the log-likelihood ratio curves are plotted as a function of the number of used SNPs and the property of this curve is exploited to determine whether the embryo carries the disease-causing mutation site. SNPs are successively added into the calculation of likelihood and for each $N_2 = 1, 2, ...$, the likelihood is calculated based on which parent's chromosome the disease-causing mutation site is on and the result is denoted as $LLR_{N_2}$. The graph of $LLR_{N_2}$ is then

plotted against the physical distance of the $N_2$-th SNP to the disease-causing mutation site. Ideally, when the number of used SNPs is small, the data itself contains insufficient information about whether the embryo carries the disease-carrying allele, so the absolute value of the log-likelihood ratio is expected to be small and the curve should be close to the horizontal axis. As the number of used SNPs increases, more and more information is taken into consideration, and if the internal consistency of the data is good enough, the curve will rise or fall to a plateau with a sufficiently large absolute value. To make the log-likelihood curve more stable, the log-likelihood change for each pair of adjacent SNPs is limited to no more than 5. The log-likelihood value of the SNP for adjacent SNP pairs is clipped with log-likelihood change larger than this specified threshold.

Finally, when the number of used SNPs is large enough to determine whether the embryo carries the disease-carrying allele, adding new SNPs can hardly change the log-likelihood ratio anymore. Therefore, when the vertical coordinates of the curve tend to be stable, the sign of the stable value can be used as a criterion to determine whether the embryo carries the disease-carrying allele, and its absolute value serves as a measure for the confidence level. In practice, the Steady Log Likelihood Ratio (SLLR) downstream of the disease-causing mutation site is defined as where

$$\mathrm{SLLR}_{\mathrm{down}} = \frac{1}{N_0} \sum_{n=n_0}^{N_0+n_0} \widehat{\mathrm{LLR}}_n,$$

$$n_0 = \max\left\{ n : \left( \max_{n \le i \le n+N_0} \widehat{\mathrm{LLR}}_i - \min_{n \le i \le n+N_0} \widehat{\mathrm{LLR}}_i \right) \le \varepsilon_0 \right\}$$

Here, $\varepsilon_0$ is a predetermined threshold. In experiments, $\varepsilon = 0.1$. Here, $N_0$ is the number of SNPs needed to identify the stability, which is by default 30. If there is no consecutive 30 SNPs whose log-likelihood range is within 0.1, then $N_0$ is reduced to 10 to detect whether the curve stabilizes. If either upstream or downstream region has less than 10 SNPs in the data, this region is ignored and the confidence interval according to the curve on the other side is determined. This happens when the disease-causing mutation site is at either end of the chromosome, or near the spindles. Similarly, we compute the upstream steady log likelihood SLLRup and denote their arithmetic average as Average Steady Log-Likelihood Ratio(ASLLR).

According to SLLR, ASLLR, and other properties of the log-likelihood curve, the confidence level of judgements is classified into four categories: Highly Confident, Moderately Confident, Possible, and Undetermined. Specifically, if SLLRup • SLLRdown < 0 for a sample, the sample is classified into "Undetermined" directly, since the opposite conclusion is obtained from the upstream and downstream regions. For the remaining samples, the Maximally Different Log-Likelihood Ratio (MDLLR) is defined as:

$$\mathrm{MDLLR} := \begin{cases} \min\left\{ \min\left\{ \widehat{\mathrm{LLR}}_n : -N_1 \le n \le N_2 \right\}, 0 \right\} & \text{If } \mathrm{SLLR}_{\mathrm{up}} > 0, \mathrm{SLLR}_{\mathrm{down}} > 0 \\ -\max\left\{ \max\left\{ \widehat{\mathrm{LLR}}_n : -N_1 \le n \le N_2 \right\}, 0 \right\} & \text{If } \mathrm{SLLR}_{\mathrm{up}} < 0, \mathrm{SLLR}_{\mathrm{down}} < 0 \end{cases}.$$

Finally the confidence level is determined by

- Highly Confident: If ASLLR > 4, MDLLR = 0, and $|\mathrm{SLLR}_{\mathrm{up}}| > 1$, $|\mathrm{SLLR}_{\mathrm{down}}| > 1$, $N_0 = 30$.
- Moderately Confident: If ASLLR $\le$ 4, MDLLR = 0, $|\mathrm{SLLR}_{\mathrm{up}}| > 1$, $|\mathrm{SLLR}_{\mathrm{down}}| > 1$, $N_0 = 30$; or ASLLR > 4, MDLLR $\ge$ -5, $N_0 = 30$.
- Possible: If none of the statements above holds and the sample is not categorized as 'undetermined'.

Following the above protocol, the genotype and which allele is inherited from parents at any single SNP can be determined. This information can then be used to execute the disease carrying analysis for the monogenic disease, and combine multiple identified SNPs of the embryo to determine its susceptibility of a polygenic disease.

The present disclosure also refers to a method of analyzing the chromosome aneuploidy via a biological sample of culture medium of an in vitro cultured embryo to do preimplantation genetic testing for aneuploidy.

## COMPUTER SYSTEM

Any of the computer systems mentioned herein may utilize any suitable number of subsystems. In some instances, a computer system includes a single computer apparatus, where the subsystems can be the components of the computer apparatus. In other instances, a computer system can include multiple computer apparatuses, each being a subsystem, with internal components.

A computer system can include a plurality of the same components or subsystems, e.g., connected together by external interface or by an internal interface. In some instances, computer systems, subsystems, or apparatuses can communicate over a network. In such instances, one computer can be considered a client and another computer a server, where each can be part of a same computer system. A client and a server can each include multiple systems, subsystems, or components.

It should be understood that any of the instances of the present invention can be implemented in the form of control logic using hardware (e.g. an application specific integrated circuit or field programmable gate array) and/or using computer software with a generally programmable processor in a modular or integrated manner. Based on the disclosure and teachings provided herein, a person of ordinary skill in the art will know and appreciate other ways and/or methods to implement instances of the present invention using hardware and a combination of hardware and software.

Any of the software components or functions described in this application may be implemented as software code to be executed by a processor using any suitable computer language such as, for example, Java, C++ or Perl using, for example, conventional or object-oriented techniques. The software code may be stored as a series of instructions or commands on a computer readable medium for storage and/or transmission, suitable media include random access memory (RAM), a read only memory (ROM), a magnetic medium such as a hard-drive or a floppy disk, or an optical medium such as a compact disk (CD) or DVD (digital versatile disk), flash memory, and the like. The computer readable medium may be any combination of such storage or transmission devices.

Such programs may also be encoded and transmitted using carrier signals adapted for transmission via wired, optical, and/or wireless networks conforming to a variety of protocols, including the Internet. As such, a computer readable medium according to an instance of the present invention may be created using a data signal encoded with such programs. Computer readable media encoded with the program code may be packaged with a compatible device or provided separately from other devices (e.g., via Internet download). Any such computer readable medium may reside on or within a single computer program product (e.g. a hard drive, a CD, or an entire computer system), and may be present on or within different computer program products within a system or network. A computer system may include a monitor, printer, or other suitable display for providing any of the results mentioned herein to a user.

Any of the methods described herein may be totally or partially performed with a computer system including one or more processors, which can be configured to perform the steps. Thus, instances can be directed to computer systems configured to perform the steps of any of the methods described herein, potentially with different components performing a respective steps or a respective group of steps. Although presented as numbered steps, steps of methods herein can be performed at a same time or in a different order. Additionally, portions of these steps may be used with portions of other steps from other methods. Also, all or portions of a step may be optional. Additionally, any of the steps of any of the methods can be performed with modules, circuits, or other means for performing these steps.

## EXAMPLES

The invention will now be illustrated, but not limited, by reference to the specific instances described in the following examples.

### Example 1: Study Subjects

The first case was an autosomal dominant genetic disease. The wife has a family history of an autosomal dominant disorder and has suffered from hereditary osteogenesis imperfecta (OI), which is characterized by easy bone fracture without obvious cause or minimal injury. The couple already had an affected son who also suffers from OI. The genetic diagnosis of the wife showed a deletion c.299-14_c.302del at the COL1A1 (collagen, type I, alpha 1) gene, already known to cause this disease. This couple underwent PGT-M treatment. Eighteen metaphase-II oocytes were collected and fertilized by intra-cytoplasmic sperm injection (ICSI). Twelve embryos developed to the blastocyst stage, and several trophectoderm cells were biopsied from each embryo for PGT-M.

The second case was an autosomal recessive disease. Both parents carried a deafness recessive heredity disorder. The genetic diagnosis of the wife showed an insertion c.99_100insT at the USH2A gene. The genetic diagnosis of the husband showed a duplication c.991dupA and a mutation c.5699G>T at the USH2A gene. The couple already had a deaf daughter whose genetic diagnosis showed the insertion c.99_100insT, the duplication c.991dupA and the mutation c.5699G>T at the USH2A gene that was inherited from her parents. These mutations cause the deafness . This couple underwent PGT-M treatment. Nine metaphase-II oocytes were collected and fertilized by ICSI. Four embryos developed to the blastocyst stage, and several trophectoderm cells were biopsied from each embryo for PGT-M.

**Blastocyst Biopsy and PGT-M Testing.** Standard protocols were used for ICSI. Embryos were cultured individually in 15-$\mu$L microdrops of the equilibrated Vitrolife G-series media with human serum albumin (HSA) (LifeGlobal) overlain with mineral oil in Miri incubators (ESCO) at 37 °C in a dry atmosphere of 5% $O_2$, 6-7% $CO_2$ balanced with $N_2$. Embryos were evaluated on day 5 or 6 for Trophectoderm (TE) biopsy. A few TE cells from each hatching blastocyst were biopsied and send to an outside PGT lab for PGT-M testing.

### Reference **Example 2: Sample Collections**

**Culture Medium Sample and Discarded Embryo Collection.** Immediately after the blastocysts were transferred to another dish for biopsy, 13-$\mu$L spent culture media were removed from each of the residual spent medium drops. The discarded embryos and their corresponding spent culture media samples were also collected for analysis. Each discarded embryo was gently moved with a pipette tip to the edge of its microdrop and then removed and transferred into a RNase-DNase-free PCR tube containing 3 $\mu$L of lysis buffer. Pipette tips were changed between collections of each sample to avoid cross-sample contamination. Media drops incubated and collected under identical conditions to those used for blastocyst culture, but never containing embryos, served as negative controls.

All samples were immediately frozen after collection and stored at -80 °C until analyzed.

### Reference **Example 3: Protocol for Lysis**

**Spent Culture Medium Lysis.** The frozen 13-$\mu$L spent culture medium samples were thawed and gently mixed, and 10.8 $\mu$L was removed to a PCR tube (Maximum Recovery, Axygen) containing 1.2 $\mu$L 10x lysis buffer (1x lysis buffer: 60 mM Tris-Ac pH 8.3, 2 mM EDTA pH 8.0, 15 mM DTT, 0.5 $\mu$M carrier ssDNA (5'-TCAGGTTTTCCTGAA-3', Thermo Fisher Scientific oligo with PAGE purification)), spun down. It was heated at 75°C for 30 min. Then incubated at 75°C for 30 min. 0.5 $\mu$L 35 mg/mL QIAGEN protease (dissolved in water and stored at 4°C) was added, spun down, and incubated at 55°C for 2 hrs followed by 80°C for 30 min. The resulting lysate in PCR tubes could be subject to modified linear transposon-based amplification as described herein immediately, or stored in a freezer for later use.

**Discarded Embryo Lysis.** The frozen discarded embryos were thawed and heated at 75°C for 30 min. Then the discarded embryo samples were lysed by adding 0.5 $\mu$L 5mg/mL Qiagen Protease and heating (2 h at 55°C and 30 min at 80 °C) in 3 $\mu$L of lysis buffer.

### Example 4: Whole Genome Amplification

**Transposome preparation.** Transposon DNA (5'/Phos/CTGTCTCTTATACACATCTGAACAGAATTTAATAC GACTCACTATAGGGAGATG TGTATAAGAGACAG-3', Thermo Fisher Scientific oligo with PAGE purification) was annealed by gradual cooling in annealing buffer (20 mM Tris-Ac pH 8.3, 50 mM NaCl, 2 mM EDTA pH 8.0) into a 1.5 $\mu$M self-looping structure. The 1.5 $\mu$M annealed transposon DNA was then mixed with an equal volume of ~1 $\mu$M Tn5 transposase (Lucigen, EZ-Tn5™ Transposase) and incubated at room temperature for 30 min, dimerizing into transposomes with a final concentration of ~0.25 $\mu$M. The transposomes can be stored at -20°C for a long time, and each single-cell transposon-based amplification needs ~0.5$\mu$L transposome.

**Whole genome amplification by modified LIANTI (Linear Amplification via Transposon Insertion) method.** Starting with the culture medium lysate, a 20 $\mu$L transposition mixture was assembled in the buffer containing 2.5 mM MgCl$_2$ and 18.75 nM transposome. The transposition reaction was carried out at 55°C for 12 min. Then 0.84 $\mu$L mixture of EDTA and ssDNA was added to each tube and was incubated at 68 °C for 30 min. After removing transposase reaction, 0.2 $\mu$L Q5 HF DNA Polymerase (New England Biolabs) was added and was heated at 73°C for 45 seconds in the presence of 2 mM MgCl$_2$ and 200 $\mu$M dNTPs for fragments end filling and extension. 0.5 $\mu$L 4mg/mL Qiagen protease was added and the PCR tube was heated at 50°C for 1 hour in the presence of 4 mM EDTA to inactivate DNA polymerase, followed by protease heat inactivation by 77°C for 20 min in the presence of 450 mM NaCl in a total volume of ~25 $\mu$L. DNA fragments were amplified to RNAs in a 90 $\mu$L T7 in vitro transcription reaction mixture overnight at 37 °C as described in in US 10,894,980 and C Chen, D Xing, L Tan, H Li, G Zhou, L Huang, XS Xie Science 2017; 356 (6334): 189-194

The next day, 10 $\mu$L 0.5M EDTA was added to each tube and RNAs were column purified (Zymo Research). 18 $\mu$L RNAs were transferred to a PCR tube containing 3.1 $\mu$L mixture of 6.5mM each dNTPs, 9.7 $\mu$M carrier ssDNA (5'-TCAGGTTTTCCTGAA-3', Thermo Fisher Scientific oligo with PAGE purification) and 3.2 U/$\mu$L SUPERase In RNase Inhibitor (Invitrogen). The denaturation incubation was at 70 °C 1min, 90 °C 15s, and followed by ice quenching. The reverse transcription reaction for the first stand was carried out in a 30 $\mu$L SuperScript IV reserve reaction system including 0.67 mM each dNTPs, 0.6 U/$\mu$L SUPERase In RNase Inhibitor (Invitrogen) and 6 U/$\mu$L SuperScript IV Reserve Transcriptase (Invitrogen) in SuperScript IV buffer, with the primer 5'-AGATGTGTATAAGAGACAG-3', Thermo Fisher Scientific oligo with PAGE purification, and the incubation program was 25°C 1 min, 37°C 1 min, 42°C 1 min, 50°C 1 min, 55°C 15 min, 60°C 10 min, 65°C 12 min, 70°C 8 min, 75°C 5 min, 80°C 10 min. RNA was then removed by incubation at 37°C for 30 min with 10 ng/$\mu$L affinity-purified RNase A (Invitrogen) and 0.08 U/$\mu$L RNase H (New England Biolabs). Second strand synthesis was carried out in a 100 $\mu$L Q5 DNA polymerase system (New England Biolabs, 1X Q5 reaction buffer, 1X Q5 High GC enhancer, 200 $\mu$M dNTPs, 0.5 $\mu$M primer, 0.02 U/$\mu$L Q5 DNA polymerase), with the primer 5'-NNNNNNNNNGGGAGATGTGTATAAGAGACAG-3', Thermo Fisher Scientific oligo with PAGE purification. Each tube was heated at 98°C for 30 s, then using 10 cycles of 10 s at 98°C, 30s at 58°C, 30s at 60°C, 30s at 65°C, 2.5 min at 70°C, then 72°C 6 min for strand extension. The resulted amplicons were column purified into 23 μL elution buffer and stored at -20°C.

The DNA concentration of the product after amplification was measured using a Qubit 2.0 fluorometer (Thermo-Fisher Scientific) with the Qubit dsDNA HS Assay kit (Life Technologies). In the first case, 17 spent culture media were amplified successfully. In the second case, 9 spent culture media were amplified using this method. 8 culture media were amplified successfully, but one failed. The amplification success rate of the spent culture medium was 96.15%.

**Discarded Embryo DNA Amplification.** DNA was amplified following the LIANTI amplification method described in US 10,894,980 and C Chen, D Xing, L Tan, H Li, G Zhou, L Huang, XS Xie. Science 2017; 356 (6334): 189-194, except the DNA primer 19_1 5'-AGATGTGTATAAGAGACAG-3', Thermo Fisher Scientific oligo with PAGE purification was added in the reaction of the first strand cDNA synthesis. Four discarded embryos in the first case and five discarded embryos in the second case were all amplified successfully.

## Example 5: Comparison of Variables in the Lysis and Amplification Methods

A diploid human cell line, BJ primary human foreskin fibroblast (ATCC CRL-2522), was used as described in the LIANTI paper (Chen 2017) and the comparison paper of single-cell whole genome amplification methods (Huang 2015). The BJ cell line was used as a standard sample for different lysis and amplification methods' comparison. In order to compare the performance of different amplification methods on spent culture medium, the BJ cell line was cultured in Eagle's minimum essential medium (ATCC) supplemented with 10% fetal bovine serum, 100 I.U./mL penicillin and 100 μg/mL streptomycin in 5% $CO_2$ 37°C incubator. After 48 hours, the culture medium of BJ cell line was collected. Then BJ cells were trypsinized and washed in 1X PBS, followed by resuspension into 1X PBS. Single BJ cells were then mouth-pipetted into PCR tubes containing 3 μL lysis buffer.

The LIANTI procedure that described in US 10,894,980 and C Chen, D Xing, L Tan, H Li, G Zhou, L Huang, XS Xie. Science 2017; 356 (6334): 189-194 was used to amplify three single cells and three culture medium samples, employing 0.5 mg/ml (working concentration) QIAGEN protease, 6.25 nM (working concentration) transposome. No DNA was successfully amplified.

Then, the amplification method described in Examples 3-4 was used to amplify culture medium DNA, except adjusting a single variable each time. Particularly, the variables include the working concentration of the protease, the working concentration of the transposome, the PCR cycles and the primer sequence. Three repetitions were performed per variable.

The comparison results were shown in Table 1 and Fig. 3. When 0.5 mg/ml (working concentration) QIAGEN protease or 6.25 nM (working concentration) transposome was used in combination with DNA primer 19_1, considerable DNA can be successfully amplified. When the working concentration of QIAGEN protease or transposome was increased, the amplified DNA amount increased accordingly.

When no DNA primer was added (wo), as with the LIANTI procedure described in US 10,894,980 and C Chen, D Xing, L Tan, H Li, G Zhou, L Huang, XS Xie. Science 2017; 356 (6334): 189-194, no DNA was successfully amplified. When primer 19_1, 19_2, 15_1 or random primer 3+14N with specific sequence was added, however, considerable DNA can be successfully amplified. The amplification result of primer 25_1 is not as good as that of primer 19_1 or 19_2, probably due to the increasing mismatch of primer 25_1 with the RNA strand of the sequence of Tn5 transposition binding site. Preferably, the length of primer is 15bp to 20bp. As compared with 3+14N, when random primer 5+14N was added, no DNA was successfully amplified, verifying the importance of the 3'-end sequence of the DNA primer.

| Protocol | Sample types | Variables types | Variables information | rep1 (DNA amount ng) | rep2 (DNA amount ng) | rep3 (DNA amount ng) |
|---|---|---|---|---|---|---|
| Original LIANTI Protocol | Single cell | \ | \ | 3.42 | 2.31 | 0 |
| | Culture medium | \ | \ | 0 | 0 | 1.5 |
| Amplification Method of the Present Invention | Culture medium | Protease Working Concentration | 0.16 mg/ml | 36.3 | 16.5 | 61.8 |
| | | | 0.6 mg/ml | 103.5 | 98.4 | 51.6 |
| | | | 1 mg/ml | 132.6 | 315 | 282 |
| | | | 1.4 mg/ml | 74.7 | 133.5 | 53.4 |
| | | | 1.8 mg/ml | 117.6 | 89.4 | 149.7 |
| | | Transposome Working Concentration | 6.25 nM | 106.8 | 11.775 | 65.1 |
| | | | 12.5 nM | 64.8 | 72.3 | 113.4 |

(continued)

| Protocol | Sample types | Variables types | Variables information | rep1 (DNA amount ng) | rep2 (DNA amount ng) | rep3 (DNA amount ng) |
|---|---|---|---|---|---|---|
| | | | 18.75 nM | 171 | 105 | 156 |
| | | | 37.5 nM | 369 | 1197 | 1308 |
| | | Primer | 15 1 | 49.2 | 26.55 | 40.8 |
| | | | 19_1 | 78.3 | 116.1 | 48.3 |
| | | | 19 2 | 63.6 | 84.6 | 88.8 |
| | | | 25 1 | 10.68 | 17.1 | 3.84 |
| | | | 3+14N | 44.7 | 30.3 | 26.55 |
| | | | 5+14N | 2.34 | 3.54 | 1.8 |
| | | PCR cycles | 1 cycle | 1.56 | 0 | 10.14 |
| | | | 4 cycles | 0 | 2.97 | 5.52 |
| | | | 8 cycles | 53.7 | 23.4 | 9.21 |
| | | | 10 cycles | 100.8 | 104.7 | 73.2 |
| | | | 12 cycles | 178.5 | 130.8 | 92.1 |
| | | | 14 cycles | 459 | 678 | 78.3 |

### Example 6: Library Preparation for Sequencing

Upon library preparation, the amplicons were subject to a conventional sonication process (Covaris S2) to the length required by sequencing platforms. The final insert size was suitable for 2X150 bp pair-end Illumina sequencing. Library preparation was performed by NEBNext Ultra II DNA Library Prep Kit for Illumina (New England Biolabs), following the instructions of the manufacturer and skipping the optional size selection step. For the gDNA of blood samples, all the samples were subject to a conventional sonication process (Covaris S2). Library preparation was performed by a PCR-free Library Prep Kit, NEBNext Ultra II DNA Library Prep Kit for Illumina (NEB #E7645S/L).

**Illumina sequencing.** The bulk samples, culture medium samples, and discarded embryo samples were sequenced on the Illumina NovaSeq 6000 System or Illumina HiSeq HiSeq 4000 platform with 2X150 bp pair-end sequencing. Sequencing lanes were shared by 24 samples with NEBNext Indexes. Each gDNA sample was sequenced to generate ~90 Gb raw data and each culture medium sample or discarded embryo was sequenced to generate ~30 Gb raw data.

### Example 7: Mapping and SNP Calling

The genome coverage and allele dropout rate of each sample was calculated. Although the LIANTI process that is described in US 10,894,980 and C Chen, D Xing, L Tan, H Li, G Zhou, L Huang, XS Xie. Science 2017; 356 (6334): 189-194 offers the highest genome coverage compared to other single-cell whole genome amplification methods (Chen et al 2017), the genome coverage of the DNAs in the spent culture medium exhibited very low genome coverage and a high allele dropout rate. In the first case, the genome coverage of the 17 spent culture media varied from 16.38% to 70.34%, as shown in Fig. 4. In the second case, the genome coverage of the 9-success amplified spent culture media varied from 4.79% to 34.97%, as shown in Fig. 5. Their ADO rate was much higher than 5%. The genome coverage was far from reaching the standard that allows linkage analysis with several loci. (ESHRE 2020).

### Example 8: Linkage Analysis by Bayesian and Disease Carrying Analysis

The Dry Lab method described above was utilized for linkage analysis.

In the first case, the heterozygous SNPs in chromosome 17 of the wife and the homozygous SNPs of the husband were the subject of analysis. In Fig. 6, the linkage analysis plots for four SCMs in Case 1 are shown. Among these four SCMs, 1-SCM-02 had the highest and tightest number of SNP loci, so in determining the absence of the inherited result of the mother's disease-causing allele in the embryo, the corresponding confidence level was 'High Confidence'. In 1-SCM-05 and 1-SCM-07, although large fractions of haplotypes were lost, the detected SNPs were calculated by Bayesian models to support that the disease-causing allele was inherited in the embryos with 'High Confidence' level of the disease-carrying analysis. In contrast, in 1-SCM-06, fewer SNP loci were detected than in the other three SCMs, a 'Moderate Confidence' level of the disease-carrying analysis in determining the absence of an inherited disease-causing allele.

For the second case, to carry out the linkage analysis of each embryo, the genomes of the blood samples of the wife, husband, and disease-carrying daughter were sequenced. All SNPs of the three individuals were called with a sequencing depth >10x within 2 Mb of the USH2A gene. The heterozygous SNPs in chromosome 1 of the wife and homozygous SNPs of the husband for the

| Case | Chrom | Pos | Disease-carring Parent | Disease Allele | Sample Name | Sample Type | Rate of GQ <= 3 | GQ Threshold | err_rate(10M) | #of SNP /common SNPs | init_mcc_rate (10M) | final_mcc_rate (10M) | Allele Classification | Truth | Classification |
|---|---|---|---|---|---|---|---|---|---|---|---|---|---|---|---|
| 1 | 17 | 48276965 | Mather | 1 | 1-SCM-01 | SCM | 0.159 | 6 | 0.014 | 0.037 | 0.296 | 0.085 | 2 | 2 | High Confidence |
| | | | | | 1-SCM-02 | SCM | 0.124 | 6 | 0.009 | 0.038 | 0.131 | -0.087 | 2 | 2 | High Confidence |
| | | | | | 1-SCM-03 | SCM | 0.131 | 6 | 0.007 | 0.012 | 0.318 | 0.3 | 2 | 2 | Undetermined |
| | | | | | 1-SCM-04 | SCM | 0.173 | 6 | 0.015 | 0.039 | 0.031 | 0.054 | 2 | 2 | Moderate Confidence |
| | | | | | 1-SCM-05 | SCM | 0.114 | 6 | 0.007 | 0.127 | 0.032 | 0.064 | 1 | 1 | Moderate Confidence |
| | | | | | 1-SCM-06 | SCM | 0.144 | 6 | 0.011 | 0.018 | 0.331 | -0.315 | 2 | 2 | Possible |
| | | | | | 1-SCM-07 | SCM | 0.149 | 6 | 0.013 | 0.019 | 0.278 | 0.33 | 1 | 1 | High Confidence |
| | | | | | 1-SCM-08 | SCM | 0.145 | 6 | 0.014 | 0.033 | -0.076 | -0.002 | 1 | 1 | Possible |
| | | | | | 1-SCM-10 | SCM | 0.148 | 6 | 0.015 | 0.028 | 0.126 | 0.019 | / | 1 | Undetermined |
| | | | | | 1-SCM-12 | SCM | 0.099 | 6 | 0.009 | 0.02 | -0.53 | -0.108 | / | 2 | Rule Out |
| | | | | | 1-SCM-14 | SCM | 0.124 | 6 | 0.01 | 0.022 | 0.025 | -0.063 | 1 | 1 | High Confidence |
| | | | | | 1-SCM-15 | SCM | 0.149 | 6 | 0.043 | 0.008 | 0.663 | 0.365 | / | 2 | Rule Out |
| | | | | | 1-SCM-16 | SCM | 0.158 | 6 | 0.01 | 0.039 | -0.057 | 0.044 | 1 | 1 | High Confidence |
| | | | | | 1-SCM-11 | SCM of Discaded Embryo | 0.154 | 6 | 0.014 | 0.041 | 0.083 | 0.048 | 1 | 1 | High Confidence |
| | | | | | 1-SCM-13 | SCM of Discaded Embryo | 0.147 | 6 | 0.013 | 0.033 | 0.051 | -0.002 | 1 | 1 | Possible |
| | | | | | 1-SCM-17 | SCM of Discaded Embryo | 0.123 | 6 | 0.012 | 0.036 | 0.081 | 0.067 | 2 | 2 | High Confidence |
| 2 | 1 | 216595579 | Mather | 1 | 2-SCM-01 | SCM | 0.105 | 6 | 0.016 | 0.021 | 0.208 | -0.219 | 2 | 2 | High Confidence |
| | | | | | 2-SCM-04 | SCM | 0.097 | 9 | 0.011 | 0.003 | 0.256 | 0.605 | / | 1 | Possible |
| | | | | | 2-SCM-05 | SCM | 0.086 | 6 | 0.009 | 0.01 | 0.003 | -0.139 | 1 | 1 | Moderate Confidence |

(continued)

| Case | Chrom | Pos | Disease-carring Parent | Disease Allele | Sample Name | Sample Type | Rate of GQ <= 3 | GQ Threshold | err_rate(10M) | #of SNP /common SNPs | init_mcc_rate (10M) | final_mcc_rate (10M) | Allele Classification | Truth | Classification |
|---|---|---|---|---|---|---|---|---|---|---|---|---|---|---|---|
| | | | | | 2-SCM-02 | SCM of Discaded Embryo | 0.122 | 6 | 0.011 | 0.005 | 0.313 | 0.313 | 2 | 2 | Possible |
| | | | | | 2-SCM-03 | SCM of Discaded Embryo | 0.144 | 6 | 0.017 | 0.009 | 0.085 | -0.502 | / | 1 | Rule Out |
| | | | | | 2-SCM-06 | SCM of Discaded Embryo | 0.129 | 6 | 0.016 | 0.01 | -0.002 | -0.362 | 2 | 2 | Undetermined |
| | | | | | 2-SCM-07 | SCM of Discaded Embryo | 0.116 | 6 | 0.019 | 0.011 | 0.195 | -0.423 | 1 | 1 | High Confidence |
| | | | | | 2-SCM-09 | SCM of Discaded Embryo | 0.18 | 6 | 0.012 | 0.002 | -0.409 | 0.01 | / | 1 | Undetermined |
| | 1 | 216498799 | Father | 1 | 2-SCM-01 | SCM | 0.105 | 6 | 0.016 | 0.021 | 0.208 | -0.219 | 2 | 2 | Moderate Confidence |
| | | | | | 2-SCM-04 | SCM | 0.097 | 9 | 0.011 | 0.032 | 0.256 | 0.605 | 1 | 1 | Possible |
| | | | | | 2-SCM-05 | SCM | 0.086 | 6 | 0.009 | 0.01 | 0.003 | -0.139 | 1 | 1 | Moderate Confidence |
| | | | | | 2-SCM-02 | SCM of Discaded Embryo | 0.122 | 6 | 0.011 | 0.005 | 0.313 | 0.313 | 1 | 1 | Possible |
| | | | | | 2-SCM-05 | SCM of Discaded Embryo | 0.144 | 6 | 0.017 | 0.009 | 0.085 | -0.502 | 1 | 1 | Rule Out |
| | | | | | 2-SCM-06 | SCM of Discaded Embryo | 0.129 | 6 | 0.016 | 0.01 | -0.002 | -0.362 | 1 | 1 | High Confidence |
| | | | | | 2-SCM-07 | SCM of Discaded Embryo | 0.116 | 6 | 0.019 | 0.011 | 0.195 | -0.423 | 2 | 2 | High Confidence |

(continued)

| Case | Chrom | Pos | Disease-carring Parent | Disease Allele | Sample Name | Sample Type | Rate of GQ <= 3 | GQ Threshold | err_rate(10M) | #of SNP /common SNPs | init_mcc_rate (10M) | final_mcc_rate (10M) | Allele Classification | Truth | Classification |
|---|---|---|---|---|---|---|---|---|---|---|---|---|---|---|---|
| | | | | | 2-SCM-09 | SCM of Dis-caded Em-bryo | 0.18 | 6 | 0.012 | 0.002 | -0.409 | 0.01 | 1 | 1 | Possible |

mutation site (c.99_100insT) that is carried by the wife were the focus of analysis, and the heterozygous SNPs in chromosome 1 of the husband and homozygous SNPs of the wife for the mutation sites (c.991dupA and c.5699G>T) that is carried by the husband were the focus of analysis.

The results for all spent culture medium in cases 1 and 2 are summarized in Table 2.

Table 2

For the first case, the culture medium for discarded embryo 1-SCM-11 was omitted due to the lack of a reliable standard for that sample, since the corresponding discarded embryo was not collected. Among the remaining 16 culture media, two were excluded because of unusual maternal cell contamination (MCC) rates. Both MCC rates were outside the normative range (1-SCM-12 had a rate of -0.53, and 1-SCM-15 had a rate of +0.663) prior to calibration, leading to their elimination from subsequent disease-carrying analysis. The only culture medium deemed 'undetermined' in instance 1 was 1-SCM-10, attributed to the divergent signs of the stationary log-likelihood ratio on either side of the disease-causing mutation site. In the upstream, the log-likelihood ratio was convergent around +10.1 and that of the downstream region converged to a value around -11.3. Despite this, disease-carrying analysis was carried out for 13 samples, classifying 8 as high confidence, 2 as moderate confidence, and 3 as possible. These results agreed with the biopsy examination.

For the second case, all 14 samples (7 culture media for both parental sides) are counted into the final result. 2-SCM-03 was disqualified in the quality control stage due to its abnormal MCC rate (-0.501) after calibration, precluding it from further analysis for both disease-causing mutation sites. Moreover, 2-SCM-04 (maternal side) and 2-SCM-08 (maternal side) were labeled 'undetermined,' indicating an uncertain result about whether the inherited gene from the mother was disease-causing. Of the remaining 10 samples (6 paternal and 4 maternal), 3 were categorized as 'highly confident,' 2 as 'moderate confident,' and 5 as 'possible.' Again, these results were consistent with those from biopsy examinations.

As an illustration, the culture medium 1-SCM-01 in the first case was the subject of analysis. Qualified SNPs were selected within a region extending 10Mbp upstream and downstream from the disease-causing mutation site, enabling the estimation of basic statistics like sequencing error rate and Maternal Cell Contamination (MCC) rate. This includes removing all SNPs with either parental allele depth lower than 5, or with QUAL value lower than 30. All SNPs with more than two alleles were removed. In this region, out of 74935 SNPs, 11913 had a Genotype Quality (GQ) value of 3 or lower. All SNPs with a GQ value of 6 or lower were eliminated, which corresponded to the lowest 20 percent of all SNP GP values. Among the remaining SNPs, 4911 SNPs were identified where both parents exhibited the same homozygous genotype (i.e., either both were 00 or both were 11). Within this subset, 220 SNPs exhibited at least one erroneous read. These 4911 SNPs generated a total of 45499 reads, with 629 erroneous reads, yielding an estimated sequencing error rate of 0.0138.

On scrutinizing all qualified SNPs, 484 SNPs were identified wherein both parents displayed different but homogeneous genotypes (i.e., either the paternal genotype was 00 and the maternal genotype was 11, or vice versa). The total number of reads in these SNPs was 4779, with 3097 derived from the mother and the remainder from the father. This data yielded an MCC rate of 0.296, calculated as (3097*2-4779)/4779. Post calibration, 332 of these 484 SNPs were classified as either 'containing paternal chromosome only' or 'containing maternal chromosome only'. The remaining 152 SNPs, regarded as 'containing both parental chromosomes', were used to calculate the estimated MCC rate after calibration. These SNPs generated a total of 1761 reads, with 955 from the mother and the rest from the father, resulting in an estimated MCC rate of 0.0846. Using the calibrated MCC rate, the log-likelihood value was interatively computed from the disease-causing mutation site. The log-likelihood ratio of any segment of SNPs, either upstream or downstream, was negative. From the 21st SNP upstream (the 48003174th base pair), the log-likelihood ratio stabilized around -15.1. Meanwhile, in the downstream region, from the 7th SNP (the 48321621st base pair), the log-likelihood ratio stabilized around -11.2. Based on this analysis, it was deduced that this sample did not inherit the disease-carrying gene from the mother and labeled this result as 'highly confident'. Comparison of the allele classification results of SCM with the results of TE-biopsy or discarded-embryo produced a consistent rate of 100%. The accuracy of this method decribed herein is high enough for PGT-M.

### Example 9: Disease-Carrying Analysis for PGT-P

To demonstrate the efficacy of the method described herein in determining the susceptibility of polygenic disease, the Polygenetic Risk Score (PRS) of type 2 diabetes was calculated for the second case. The PRS is derived from a combination of multiple genetic variants, specifically single nucleotide polymorphisms (SNPs), which are directly associated to the disease. From the 139 prevalent SNPs related to type 2 diabetes mentioned in Xue, et al. 2018, 57 SNPs were identified to be present in the parental samples of the family. For each SNP, a Bayesian model was employed separately for determining the genotype of each disease-related SNP. Possible scores for the SNPs are 0, 1, or 2, which represent homozygous non-effect allele, heterozygous allele, and homozygous effect allele, respectively. PRS is defined as the weighted sum of these scores, with the weight equal to the absolute value of the log odds ratio to the minor allele at each SNP. We examined three discarded embryos from the second case, along with their corresponding culture mediums. The results indicate an alignment and correlation between the PRS scores of the discarded embryos and those derived from the culture mediums, as elaborated in Table 3 and Fig. 7.

Table 3.

| Embryo Name | 2-Embryo-03 | 2-Embryo-07 | 2-Embryo-08 |
|---|---|---|---|
| PRS in the culture medium | 3.532 | 3.767 | 3.288 |
| PRS in the discarded embryo | 3.634 | 3.814 | 3.36 |

**Example 10: Non-Invasive Preimplantation Genetic Testing for Aneuploidy**

In the addition to the niPGT-M and niPGT-P, the sequencing data of the present invention is used for non-invasive preimplantation genetic testing for aneuploidy (niPGT-A). After high-quality reads were aligned to the human reference genome hg19, copy number variation (CNV) detection for each sample was performed. Mapped reads were subjected to correction of GC bias resulting from DNA's GC contents. The read counts for each bin of 1000kb were define as the copy number (CN). CNV analysis was conducted using HMMcopy (v1.28.0) and DNAcopy (v1.58.0) at a resolution of 1Mb. Segmentation was performed via HMMcopy with an e value of 0.9999, and using DNAcopy with an alpha value of 0.0001. CNVs were identified by using the results from both HMMcopy and DNA to identify aneuploid segments larger than 10 Mb, while retaining double-positive counts as true events. The cytoband location of those aneuploid segments was calculated using the cytoband file for hg19 from the University of California, Santa Cruz, database (available at world wide website hgdownload.cse.ucsc.edu/downloads.html ).

In the first case, the CN plots of the spent culture medium was compared with TE-biopsy. The CNV results of the spent culture medium were abnormal when the corresponding TE-biopsy results were abnormal. For example, in Fig. 8 (a), the niPGT-A result of 1-SCM-02 showed a monosomy 10 aneuploidy, which was consistant with the corresponding TE result. In Fig. 8 (b), the niPGT-A result of 1-SCM-04 showed chromosome loss on chromosome 9's q arm, which was also consistant with its TE-biopsy result. In Fig. 8 (c), the niPGT-A result of 1-SCM-14 was normal, which the corresponding TE-biopsy result was also normal. Theses results demonstrate that the method described herein could analysize the copy number of spent culture medium.

The features disclosed in the foregoing description, or the following claims, expressed in their specific forms or in terms of a means for performing the disclosed function, or a method or process for attaining the disclosed result, as appropriate, may, separately, or in any combination of such features, be utilized for realizing the invention in diverse forms thereof.

The foregoing invention has been described in some detail by way of illustration and example, for purposes of clarity and understanding. It will be obvious to one of skill in the art that changes and modifications may be practiced within the scope of the appended claims. Therefore, it is to be understood that the above description is intended to be illustrative and not restrictive. The scope of the invention should, therefore, be determined not with reference to the above description, but should instead be determined with reference to the following appended claims, along with the full scope of equivalents to which such claims are entitled.

The patents, published applications, and scientific literature referred to herein establish the knowledge of those skilled in the art.

instanceOther instances of the disclosure may be directed to specific instances relating to each individual aspect, or specific combinations of these individual aspects.

The sequences used in the present invention are summarized in Table 4.

| Seq. ID No. | Description | Sequence Information |
|---|---|---|
| 1 | DNA Primer 19_1 | 5'-AGATGTGTATAAGAGACAG-3' |
| 2 | DNA Primer 19_2 | 5'-TCTACACATATTCTCTGTC-3' |
| 3 | carrier ssDNA | 5'-TCAGGTTTTCCTGAA-3' |
| 4 | LIANTI transposon DNA | 5'/Phos/CTGTCTCTTATACACATCTGAACAGAATTTAATA CGACTCACTATAGGGAGATGTGTATAAGAGACAG-3' |

(continued)

| Seq. ID No. | Description | Sequence Information |
|---|---|---|
| 5 | DNA Primer 2nd strand | 5'-NNNNNNNNGGGAGATGTGTATAAGAGACAG-3' |
| 6 | DNA Primer 15_1 | 5'-AGATGTGTATAAGAG-3' |
| 7 | DNA Primer 25_1 | 5'-AGATGTGTATAAGAGACAGTCTACA-3' |
| 8 | DNA Primer 3+14N | 5'-NNNNNNNNNNNNNNGACAG-3' |
| 9 | DNA Primer 5+14N | 5'-AGATGNNNNNNNNNNNNNNN-3' |

MODES

Aspects of the present disclosure are directed to a method for amplifying the DNA in a solution, wherein the DNA content in the solution can be at the minimum of picogram level, the method including the following steps: combining lysis buffer with the solution, heating the solution, then combining protease with the solution, incubating the solution, followed by inactivation of the protease under high temperatures to obtain lysate; combining transposome with the lysate, wherein the transposome includes a transposase and a transposon, wherein the transposon comprises a transposase binding site and an RNA polymerase promoter sequence, wherein the transposome binds to DNA in the solution, the transposase cuts the DNA in the solution to produce DNA fragments and the transposon becomes incorporated or inserted into the DNA fragments; a 9-bp gap resulting from transposon insertion is filled and extended down to both ends of each fragment; performing in vitro transcription using the original sequence as a template to form RNA; combining the RNA with a DNA primer and a reverse transcription system to form a first strand cDNA via reverse transcription, wherein the DNA primer is complementary to the respective RNA strand of the sequence of transposition binding site; and forming a second strand DNA via cycling amplification. According to one aspect, the solution is a culture medium or a blastocoele fluid. According to one aspect, the DNA primer has a length of 15bp to 20bp. According to one aspect, the 3'-end of the DNA primer contains the sequence of 5'-GACAG-3 or 5'-CTGTC-3', preferably the DNA primer contains the sequence set force as 5'-GAT GTGTATAAGAGACAG-3' (Seq. ID No.: 1), or 5'-TCTACACATATTCTCTGTC-3' (Seq. ID No.: 2) or is at least 70%, preferably 80%, preferably 90%, preferably 95, preferably 100% identity with Seq. ID No.: 1 or Seq. ID No.: 2. According to one aspect, the lysis buffer is 2x to 10x lysis buffer. According to one aspect, the working concentration of the protease in the lysis mixture is higher than 0.6 mg/mL, preferably in the range of 0.8-2 mg/mL. According to one aspect, the incubation is performed at 45-60°C for 1 to 3 hours. According to one aspect, the inactivation is performed at 80-90°C. According to one aspect, the working concentration of the transposome is higher than 6 nM, preferably in the range of 10-40 nM. According to one aspect, the cycling amplification is performed more than 4 cycles, such as 4 to 20 cycles, preferably 6 to 15 cycles.

The present disclosure provides a method for amplifying the DNA in a culture medium, wherein the DNA content in the culture medium can be at the minimum of picogram level, the method includes the following steps: combining 2x to 10x lysis buffer with the culture medium, heating the culture medium, combining protease with the culture medium to the working concentration of 0.8-2 mg/mL and incubating the culture medium at 45-60°C for 1 to 3 hours, followed by inactivation of the protease at 80-90°C to obtain lysate; combining transposome with the lysate to the working concentration of 10-40 nM, wherein the transposome includes a transposase and a transposon, wherein the transposon comprises a transposase binding site and an RNA polymerase promoter sequence, wherein the transposome binds to DNA in the solution, the transposase cuts the DNA in the solution to produce DNA fragments and the transposon becomes incorporated or inserted into the DNA fragments; a 9-bp gap resulting from transposon insertion is filled and extended down to both ends of each fragment; performing in vitro transcription using the original sequence as a template to form RNA; combining the RNA with DNA primer pairing with the nucleic acid sequence conjugated with the transposome and a reverse transcription system, forming a first strand cDNA via reverse transcription, wherein the DNA primer contains the sequence set forth as 5'-AGATGTGTATAAGAGACAG-3' (Seq. ID No.: 1) or 5'-TCTACACATATTCTCTGTC-3' (Seq. ID No.: 2), or is at least 70%, preferably 80%, preferably 90%, preferably 95, preferably 100% identity with Seq. ID No.: 1 or Seq. ID No.: 2; and c. forming a second strand DNA via 6 to 15 cycles of amplification.

The present disclosure provides a method of analyzing a biological sample of culture medium of an in vitro cultured embryo to determine the susceptibility of a genetic disease in the embryo, including 1) amplifying the DNA molecules from the biological sample of culture medium of the in vitro cultured embryo and/or the biological sample of a discarded embryo in the family; 2) preparing DNA libraries and sequencing the DNA molecules from the biological sample to obtain a plurality of sequence reads; 3) performing mapping and SNP calling, by a computer system, to generate the SNP data and controlling its quality; 4) conducting haplotype pre-phasing, by a computer system, for the blood samples of both parents or solely the disease-carrying parent; 5) calculating the likelihood of the four possible inheritance scenarios at each single SNP representing whether the genetic chains are inherited from the paternal or maternal alleles of the parents, taking into account the maternal cell contamination (MCC) rate and haplotype status; 6) estimating the MCC rate, and determining the haplotype status of each SNP to represent whether only DNA from one parent is present in the culture medium; 7) calculating the likelihood of observing the SNP data within regions adjacent to the disease-causing mutation site, combining the likelihood at each single SNP and the recombination probabilities; 8) determining whether the embryo carries the disease-causing chromosome, and providing a level of confidence. According to one aspect, the embryo has contamination caused by maternal cells and/or haplotype loss. According to one aspect, in step 3) mapping and SNP calling outputs the physical location, allele type, allele depth and sequencing quality for each SNP of the biological sample of culture medium of the in vitro cultured embryo and/or the biological sample of the discarded embryo in the family. According to one aspect, in step 3) controlling the quality of the SNP data is performed via filtering out the SNPs with low gene quality(GQ) values of the biological sample of culture medium of the in vitro cultured embryo and/or the biological sample of the discarded embryo in the family. According to one aspect, including filtering out the SNPs with the lowest 20% GQ values of the biological sample of culture medium of the in vitro cultured embryo and/or the biological sample of the discarded embryo in the family. According to one aspect, in step 3), estimating the sequencing error rate and the relative density of SNPs in the region adjacent to the disease-causing mutation site, and excluding the sample of the culture medium with an extremely low SNP density, or high sequencing error rate. According to one aspect, the excluded SNP density is smaller than 0.001, or the excluded sequencing error rate is larger than 0.2. According to one aspect, in step 4) employing any one or more of the following samples in the family: the blood sample of proband, the biological sample of the discarded embryo, or the blood sample of at least one of the grandparents. According to one aspect, in step 5), integrating the estimated sequencing error rate when calculating the likelihood for each individual SNP. According to one aspect, step 6) further includes the following steps: a) initially estimating the MCC rate and haplotype status of each SNP in the region adjacent to the disease-causing mutation site; and b) recursively updating the MCC rate and haplotype status of each SNP. According to one aspect, step b) includes using a label-searching method. According to one aspect, after step a), excluding the sample with abnormal MCC rate. According to one aspect, the abnormal MCC rate is larger than 0.65 or less than -0.5. According to one aspect, in step 7) using a Bayesian model and a recursive algorithm. According to one aspect, in step 8), first plotting the log likelihood curve for both upstream and downstream regions of the disease-causing mutation site. According to one aspect, the genetic disease includes both monogenic and polygenic diseases. According to one aspect, after step 8), combining multiple identified SNPs of the embryo to determine its susceptibility to a polygenic disease.

The present disclosure provides a method of analyzing a biological sample of culture medium of an in vitro cultured embryo to determine the susceptibility of a genetic disease in the embryo, including 1) amplifying the DNA molecules from the biological sample of culture medium of the in vitro cultured embryo using the method according to any one of claims 1-12, and/or amplifying the DNA molecules from the biological sample of a discarded embryo in the family; 2) preparing DNA libraries and sequencing the DNA molecules from the biological sample to obtain a plurality of sequence reads; 3) performing mapping and SNP calling, by a computer system, to generate the SNP data and controlling its quality; 4) conducting haplotype pre-phasing, by a computer system, for the blood samples of both parents or solely the disease-carrying parent; 5) calculating the likelihood of the four possible inheritance scenarios at each single SNP representing whether the genetic chains are inherited from the paternal or maternal alleles of the parents, taking into account the maternal cell contamination (MCC) rate and haplotype status; 6) estimating the MCC rate, and determining the haplotype status of each SNP to represent whether only DNA from one parent is present in the culture medium; 7) calculating the likelihood of observing the SNP data within regions adjacent to the disease-causing mutation site, combining the likelihood at each single SNP and the recombination probabilities; 8) determining whether the embryo carries the disease-causing chromosome, and providing a level of confidence. According to one aspect, the embryo has contamination caused by maternal cells and/or haplotype loss. According to one aspect, in step 3) mapping and SNP calling outputs the physical location, allele type, allele depth and sequencing quality for each SNP of the biological sample of culture medium of the in vitro cultured embryo and/or the biological sample of the discarded embryo in the family. According to one aspect, in step 3) controlling the quality of the SNP data is performed via filtering out the SNPs with low gene quality(GQ) values of the biological sample of culture medium of the in vitro cultured embryo and/or the biological sample of the discarded embryo in the family. According to one aspect, including filtering out the SNPs with the lowest 20% GQ values of the biological sample of culture medium of the in vitro cultured embryo and/or the biological sample of the discarded embryo in the family. According to one aspect, in step 3), estimating the sequencing error rate and the relative density of SNPs in the region adjacent to the disease-causing mutation site, and excluding the sample of the culture medium with an extremely low SNP density, or high sequencing error rate. According to one aspect, the excluded SNP density is smaller than 0.001, or the excluded sequencing error rate is larger than 0.2. According to one aspect, in step 4) employing any one or more of the following samples in the family: the blood sample of proband, the biological sample of the discarded embryo, or the blood sample of at least one of the grandparents. According to one aspect, in step 5), integrating the estimated sequencing error rate when calculating the likelihood for each individual SNP. According to one aspect, step 6) further includes the following steps a) initially estimating the MCC rate and haplotype status of each SNP in the region adjacent to the disease-causing mutation site; and b) recursively updating the MCC rate and haplotype status of each SNP. According to one aspect, step b) uses a label-searching method. According to one aspect, after step a), excluding the sample with abnormal MCC rate. According to one aspect, the abnormal MCC rate is larger than 0.65 or less than -0.5. According to one aspect, in step 7) using a Bayesian model and a recursive algorithm. According to one aspect, in step 8), first plotting the log likelihood curve for both upstream and downstream regions of the disease-causing mutation site. According to one aspect, the genetic disease includes both monogenic and polygenic diseases. According to one aspect, step 8) includes combining multiple identified SNPs of the embryo to determine its susceptibility to a polygenic disease.

The present disclosure provides a computer program including a plurality of instructions capable of execution by a computer system and arranged when executed to control the computer system to analyze a biological sample of culture medium of an in vitro cultured embryo to determine the susceptibility of a genetic disease in the embryo, including one or more of the steps of 1) amplifying the DNA molecules from the biological sample of culture medium of the in vitro cultured embryo, and/or amplifying the DNA molecules from the biological sample of a discarded embryo in the family; 2) preparing DNA libraries and sequencing the DNA molecules from the biological sample to obtain a plurality of sequence reads; 3) performing mapping and SNP calling, by a computer system, to generate the SNP data and controlling its quality; 4) conducting haplotype pre-phasing, by a computer system, for the blood samples of both parents or solely the disease-carrying parent; 5) calculating the likelihood of the four possible inheritance scenarios at each single SNP representing whether the genetic chains are inherited from the paternal or maternal alleles of the parents, taking into account the maternal cell contamination (MCC) rate and haplotype status; 6) estimating the MCC rate, and determining the haplotype status of each SNP to represent whether only DNA from one parent is present in the culture medium; 7) calculating the likelihood of observing the SNP data within regions adjacent to the disease-causing mutation site, combining the likelihood at each single SNP and the recombination probabilities; 8) determining whether the embryo carries the disease-causing chromosome, and providing a level of confidence. According to one aspect, the embryo has contamination caused by maternal cells and/or haplotype loss. According to one aspect, in step 3) mapping and SNP calling outputs the physical location, allele type, allele depth and sequencing quality for each SNP of the biological sample of culture medium of the in vitro cultured embryo and/or the biological sample of the discarded embryo in the family. According to one aspect, in step 3) controlling the quality of the SNP data is performed via filtering out the SNPs with low gene quality(GQ) values of the biological sample of culture medium of the in vitro cultured embryo and/or the biological sample of the discarded embryo in the family. According to one aspect, including filtering out the SNPs with the lowest 20% GQ values of the biological sample of culture medium of the in vitro cultured embryo and/or the biological sample of the discarded embryo in the family. According to one aspect, in step 3), estimating the sequencing error rate and the relative density of SNPs in the region adjacent to the disease-causing mutation site, and excluding the sample of the culture medium with an extremely low SNP density, or high sequencing error rate. According to one aspect, the excluded SNP density is smaller than 0.001, or the excluded sequencing error rate is larger than 0.2. According to one aspect, in step 4) employing any one or more of the following samples in the family: the blood sample of proband, the biological sample of the discarded embryo, or the blood sample of at least one of the grandparents. According to one aspect, in step 5), integrating the estimated sequencing error rate when calculating the likelihood for each individual SNP. According to one aspect, step 6) further includes the following steps a) initially estimating the MCC rate and haplotype status of each SNP in the region adjacent to the disease-causing mutation site; and b) recursively updating the MCC rate and haplotype status of each SNP. According to one aspect, step b) uses a label-searching method. According to one aspect, after step a), excluding the sample with abnormal MCC rate. According to one aspect, the abnormal MCC rate is larger than 0.65 or less than -0.5. According to one aspect, in step 7) using a Bayesian model and a recursive algorithm. According to one aspect, in step 8), first plotting the log likelihood curve for both upstream and downstream regions of the disease-causing mutation site. According to one aspect, the genetic disease includes both monogenic and polygenic diseases. According to one aspect, after step 8), combining multiple identified SNPs of the embryo to determine its susceptibility to a polygenic disease.

The present disclosure provides a computer system for analyzing a biological sample of culture medium of an in vitro cultured embryo to determine the susceptibility of a genetic disease in the embryo, including 1) means for amplifying the DNA molecules from the biological sample of culture medium of the in vitro cultured embryo, and/or amplifying the biological sample of a discarded embryo in the family; 2) means for preparing DNA libraries and sequencing the DNA molecules from the biological sample to obtain a plurality of sequence reads; 3) means for performing mapping and SNP calling, by a computer system, to generate the SNP data and controlling its quality; 4) means for conducting haplotype pre-phasing, by a computer system, for the blood samples of both parents or solely the disease-carrying parent; 5) means for calculating the likelihood of the four possible inheritance scenarios at each single SNP representing whether the genetic chains are inherited from the paternal or maternal alleles of the parents, taking into account the maternal cell contamination (MCC) rate and haplotype status; 6) means for estimating the MCC rate, and determining the haplotype status of each SNP to represent whether only DNA from one parent is present in the culture medium; 7) means for calculating the likelihood of observing the SNP data within regions adjacent to the disease-causing mutation site, combining the likelihood at each single SNP and the recombination probabilities; 8) means for determining whether the embryo carries the disease-causing chromosome, and providing a level of confidence. According to one aspect, the embryo has contamination caused by maternal cells and/or haplotype loss. According to one aspect, in means 3) mapping and SNP calling outputs the physical location, allele type, allele depth and sequencing quality for each SNP of the biological sample of culture medium of the in vitro cultured embryo and/or the biological sample of the discarded embryo in the family. According to one aspect, in means 3) controlling the quality of the SNP data is performed via filtering out the SNPs with low gene quality(GQ) values of the biological sample of culture medium of the in vitro cultured embryo and/or the biological sample of the discarded embryo in the family. According to one aspect, including filtering out the SNPs with the lowest 20% GQ values of the biological sample of culture medium of the in vitro cultured embryo and/or the biological sample of the discarded embryo in the family. According to one aspect, in means 3), estimating the sequencing error rate and the relative density of SNPs in the region adjacent to the disease-causing mutation site, and excluding the sample of the culture medium with an extremely low SNP density, or high sequencing error rate. According to one aspect, the excluded SNP density is smaller than 0.001, or the excluded sequencing error rate is larger than 0.2. According to one aspect, in means 4) employing any one or more of the following samples in the family: the blood sample of proband, the biological sample of the discarded embryo, or the blood sample of at least one of the grandparents. According to one aspect, in means 5), integrating the estimated sequencing error rate when calculating the likelihood for each individual SNP. According to one aspect, means 6) further includes the following functions a) initially estimating the MCC rate and haplotype status of each SNP in the region adjacent to the disease-causing mutation site; and b) recursively updating the MCC rate and haplotype status of each SNP. According to one aspect, function b) uses a label-searching method. According to one aspect, after function a), excluding the sample with abnormal MCC rate. According to one aspect, the abnormal MCC rate is larger than 0.65 or less than -0.5. According to one aspect, in means 7) using a Bayesian model and a recursive algorithm. According to one aspect, in means 8), first plotting the log likelihood curve for both upstream and downstream regions of the disease-causing mutation site. According to one aspect, the genetic disease includes both monogenic and polygenic diseases.

According to one aspect, further including means for combining multiple identified SNPs of the embryo to determine its susceptibility to a polygenic disease.

The present disclosure provides a method of preparing an otherwise unsequenceable biological sample of culture medium of an in vitro cultured embryo into a transformed state that is capable of being analyzed to determine a susceptibility of a genetic disease in the embryo, including 1) amplifying a DNA molecules from the biological sample of culture medium of the in vitro cultured embryo and/or the biological sample of a discarded embryo in a family; 2) preparing DNA libraries and sequencing the DNA molecules from the biological sample to obtain a plurality of sequence reads; 3) performing mapping and SNP calling, by a computer system, to generate the SNP data and controlling its quality; 4) conducting haplotype pre-phasing, by the computer system, for the blood samples of both parents or solely the disease-carrying parent; 5) calculating, by the computer system, the likelihood of the four possible inheritance scenarios at each single SNP representing whether the genetic chains are inherited from the paternal or maternal alleles of the parents, taking into account the maternal cell contamination (MCC) rate and haplotype status; 6) transforming the SNP data by estimating, by the computer system, the MCC rate, and determining the haplotype status of each SNP to represent whether only DNA from one parent is present in the culture medium; 7) calculating, by the computer system, the likelihood of observing the SNP data within regions adjacent to the disease-causing mutation site, combining the likelihood at each single SNP and the recombination probabilities; and 8) determining, by the computer system, whether the embryo carries the disease-causing chromosome, and providing a level of confidence, wherein the biological sample has high ADO rates in a presence of maternal contamination.

**References**

**The following full citation references correspond respectively to the short form citations used above and are incorporated therein as if set forth fully therein.**

1. Handyside AH, Penketh RJ, Winston RM, Pattinson JK, Delhanty JD, Tuddenham EG. Biopsy of human preimplantation embryos and sexing by DNA amplification. The Lancet. 1989 Feb 18;333(8634):347-9.

2. Handyside AH, Kontogianni EH, Hardy KR, Winston RM. Pregnancies from biopsied human preimplantation embryos sexed by Y-specific DNA amplification. Nature. 1990 Apr 19;344(6268):768-70.

3. Kerem BS, Rommens JM, Buchanan JA, Markiewicz D, Cox TK, Chakravarti A, Buchwald M, Tsui LC. Identification of the cystic fibrosis gene: genetic analysis. Science. 1989 Sep 8;245(4922):1073-80.

4. Handyside AH, Lesko JG, Tarin JJ, Winston RM, Hughes MR. Birth of a normal girl after in vitro fertilization and preimplantation diagnostic testing for cystic fibrosis. New England Journal of Medicine. 1992 Sep 24;327(13):905-9.

5. Liu J, Lissens W, Silber SJ, Devroey P, Liebaers I, Van Steirteghem A. Birth after preimplantation diagnosis of the cystic fibrosis$\Delta$ F508 mutation by polymerase chain reaction in human embryos resulting from intracytoplasmic sperm injection with epididymal sperm. Jama. 1994 Dec 21;272(23):1858-60.

6. Harton GL, Tsipouras P, Sisson ME, Starr KM, Mahoney BS, Fugger EF, Schulman JD, Kilpatrick MW, Levinson G, Black SH. Preimplantation genetic testing for Marfan syndrome. MHR: Basic science of reproductive medicine. 1996 Sep 1;2(9):713-5.

7. Ao A, Wells D, Handyside AH, Winston RM, Delhanty JD. Preimplantation genetic diagnosis of inherited cancer: familial adenomatous polyposis coli. Journal of assisted reproduction and genetics. 1998 Mar;15:140-4.

8. Sermon K, Goossens V, Seneca S, Lissens W, De Vos A, Vandervorst M, Van Steirteghem A, Liebaers I. Preimplantation diagnosis for Huntington's disease (HD): clinical application and analysis of the HD expansion in affected embryos. Prenatal Diagnosis: Published in Affiliation With the International Society for Prenatal Diagnosis. 1998 Dec;18(13):1427-36.

9. Xu K, Shi ZM, Veeck LL, Hughes MR, Rosenwaks Z. First unaffected pregnancy using preimplantation genetic diagnosis for sickle cell anemia. Jama. 1999 May 12;281(18):1701-6.

10. Hussey ND, Donggui H, Froiland DA, Hussey DJ, Haan EA, Matthews CD, Craig JE. Analysis of five Duchenne muscular dystrophy exons and gender determination using conventional duplex polymerase chain reaction on single cells. Molecular human reproduction. 1999 Nov 1;5(11):1089-94.

11. Ray PF, Gigarel N, Paul Bonnefont J, Attié T, Hamamah S, Frydman N, Vekemans M, Frydman R, Munnich A. First specific preimplantation genetic diagnosis for ornithine transcarbamylase deficiency. Prenatal diagnosis. 2000 Dec;20(13):1048-54.

12. De Rycke M, Van de Velde H, Sermon K, Lissens W, De Vos A, Vandervorst M, Vanderfaeillie A, Van Steirteghem A, Liebaers I. Preimplantation genetic diagnosis for sickle - cell anemia and for β - thalassemia. Prenatal Diagnosis: Published in Affiliation With the International Society for Prenatal Diagnosis. 2001 Mar;21(3):214-22.

13. Moutou C, Gardes N, Rongieres C, Ohl J, Bettahar - Lebugle K, Wittemer C, Gerlinger P, Viville S. Allele - specific amplification for preimplantation genetic diagnosis (PGD) of spinal muscular atrophy. Prenatal diagnosis. 2001 Jun;21(6):498-503.

14. Verlinsky Y, Rechitsky S, Schoolcraft W, Strom C, Kuliev A. Preimplantation diagnosis for Fanconi anemia combined with HLA matching. Jama. 2001 Jun 27;285(24):3130-3.

15. Sermon K, Seneca S, De Rycke M, Goossens V, Van de Velde H, De Vos A, Platteau P, Lissens W, Van Steirteghem A, Liebaers I. PGD in the lab for triplet repeat diseases-myotonic dystrophy, Huntington's disease and Fragile-X syndrome. Molecular and Cellular Endocrinology. 2001 Oct 22;183: S77-85.

16. Girardet A, Hamamah S, Anahory T, Dechaud H, Sarda P, Hedon B, Demaille J, Claustres M. First preimplantation genetic diagnosis of hereditary retinoblastoma using informative microsatellite markers. MHR: Basic science of reproductive medicine. 2003 Feb 1;9(2):111-6.

17. Fiorentino F, Biricik A, Nuccitelli A, De Palma R, Kahraman S, Iacobelli M, Trengia V, Caserta D, Bonu MA, Borini A, Baldi M. Strategies and clinical outcome of 250 cycles of preimplantation genetic diagnosis for single gene disorders. Human Reproduction. 2006 Mar 1;21(3):670-84.

18. Kahraman S, Beyazyurek C, Yesilipek MA, Ozturk G, Ertem M, Anak S, Kansoy S, Aksoylar S, Kuşkonmaz B, Oniz H, Slavin S. Successful haematopoietic stem cell transplantation in 44 children from healthy siblings conceived after preimplantation HLA matching. Reproductive biomedicine online. 2014 Sep 1;29(3):340-51.

19. Munné S, Lee A, Rosenwaks Z, Grifo J, Cohen J. Fertilization and early embryology: Diagnosis of major chromosome aneuploidies in human preimplantation embryos. Human reproduction. 1993 Dec 1;8(12):2185-91.

20. Wilton L, Williamson R, McBain J, Edgar D, Voullaire L. Birth of a healthy infant after preimplantation confirmation of euploidy by comparative genomic hybridization. New England Journal of Medicine. 2001 Nov 22;345(21):1537-41.

21. Wells D, Escudero T, Levy B, Hirschhorn K, Delhanty JD, Munne S. First clinical application of comparative genomic hybridization and polar body testing for preimplantation genetic diagnosis of aneuploidy. Fertility and sterility. 2002 Sep 1;78(3):543-9.

22. Treff NR, Su J, Tao X, Levy B, Scott Jr RT. Accurate single cell 24 chromosome aneuploidy screening using whole genome amplification and single nucleotide polymorphism microarrays. Fertility and sterility. 2010 Nov 1;94(6):2017-21.

23. Gutiérrez-Mateo C, Colls P, Sánchez-García J, Escudero T, Prates R, Ketterson K, Wells D, Munné S. Validation of microarray comparative genomic hybridization for comprehensive chromosome analysis of embryos. Fertility and sterility. 2011 Mar 1;95(3):953-8.

24. Yang Z, Liu J, Collins GS, Salem SA, Liu X, Lyle SS, Peck AC, Sills ES, Salem RD. Selection of single blastocysts for fresh transfer via standard morphology assessment alone and with array CGH for good prognosis IVF patients: results from a randomized pilot study. Molecular cytogenetics. 2012 Dec;5:1-8.

25. Scott Jr RT, Upham KM, Forman EJ, Hong KH, Scott KL, Taylor D, Tao X, Treff NR. Blastocyst biopsy with comprehensive chromosome screening and fresh embryo transfer significantly increases in vitro fertilization implantation and delivery rates: a randomized controlled trial. Fertility and sterility. 2013 Sep 1;100(3):697-703.

26. Forman EJ, Hong KH, Ferry KM, Tao X, Taylor D, Levy B, Treff NR, Scott Jr RT. In vitro fertilization with single euploid blastocyst transfer: a randomized controlled trial. Fertility and sterility. 2013 Jul 1;100(1):100-7.

27. Wells D, Kaur K, Grifo J, Glassner M, Taylor JC, Fragouli E, Munne S. Clinical utilisation of a rapid low-pass whole genome sequencing technique for the diagnosis of aneuploidy in human embryos prior to implantation. Journal of medical genetics. 2014 Aug 1;51(8):553-62.

28. Rubio C, Bellver J, Rodrigo L, Castillón G, Guillén A, Vidal C, Giles J, Ferrando M, Cabanillas S, Remohi J, Pellicer A. In vitro fertilization with preimplantation genetic diagnosis for aneuploidies in advanced maternal age: a randomized, controlled study. Fertility and sterility. 2017 May 1;107(5):1122-9.

29. Conn CM, Harper JC, Winston RM, Delhanty JD. Infertile couples with Robertsonian translocations: preimplantation genetic analysis of embryos reveals chaotic cleavage divisions. Human Genetics. 1998 Jan;102:117-23.

30. Scriven PN, Handyside AH, Ogilvie CM. Chromosome translocations: segregation modes and strategies for preimplantation genetic diagnosis. Prenatal Diagnosis: Published in Affiliation With the International Society for Prenatal Diagnosis. 1998 Dec;18(13):1437-49.

31. Coonen E, Martini E, Dumoulin JC, Hollanders-Crombach HT, de Die-Smulders C, Geraedts JP, Hopman AH, Evers JL. Preimplantation genetic diagnosis of a reciprocal translocation t (3; 11)(q27. 3; q24. 3) in siblings. Molecular human reproduction. 2000 Mar 1;6(3):199-206.

32. Munné S, Sandalinas M, Escudero T, Fung J, Gianaroli L, Cohen J. Outcome of preimplantation genetic diagnosis of translocations. Fertility and Sterility. 2000 Jun 1;73(6):1209-18.

33. Escudero T, Abdelhadi I, Sandalinas M, Munne S. Predictive value of sperm fluorescence in situ hybridization analysis on the outcome of preimplantation genetic diagnosis for translocations. Fertility and sterility. 2003 Jun 1;79:1528-34.

34. Melotte C, Debrock S, D'Hooghe T, Fryns JP, Vermeesch JR. Preimplantation genetic diagnosis for an insertional translocation carrier. Human Reproduction. 2004 Dec 1;19(12):2777-83.

35. Le Caignec C, Spits C, Sermon K, De Rycke M, Thienpont B, Debrock S, Staessen C, Moreau Y, Fryns JP, Van Steirteghem A, Liebaers I. Single-cell chromosomal imbalances detection by array CGH. Nucleic acids research. 2006 Jan 1;34(9):e68-.

36. Traversa MV, Carey L, Leigh D. A molecular strategy for routine preimplantation genetic diagnosis in both reciprocal and Robertsonian translocation carriers. MHR: Basic science of reproductive medicine. 2010 Feb 19;16(5):329-37.

37. Fiorentino F, Kokkali G, Biricik A, Stavrou D, Ismailoglu B, De Palma R, Arizzi L, Harton G, Sessa M, Pantos K. Polymerase chain reaction-based detection of chromosomal imbalances on embryos: the evolution of preimplantation genetic diagnosis for chromosomal translocations. Fertility and sterility. 2010 Nov 1;94(6):2001-11.

38. Rius M, Obradors A, Daina G, Ramos L, Pujol A, Martinez-Passarell O, Marquès L, Oliver-Bonet M, Benet J, Navarro J. Detection of unbalanced chromosome segregations in preimplantation genetic diagnosis of translocations by short comparative genomic hibridization. Fertility and sterility. 2011 Jul 1;96(1):134-42.

39. Fiorentino F, Spizzichino L, Bono S, Biricik A, Kokkali G, Rienzi L, Ubaldi FM, Iammarrone E, Gordon A, Pantos K. PGD for reciprocal and Robertsonian translocations using array comparative genomic hybridization. Human Reproduction. 2011 Jul 1;26(7):1925-35.

40. Treff NR, Thompson K, Rafizadeh M, Chow M, Morrison L, Tao X, Garnsey H, Reda CV, Metzgar TL, Neal S, Jalas C. SNP array-based analyses of unbalanced embryos as a reference to distinguish between balanced translocation carrier and normal blastocysts. Journal of Assisted Reproduction and Genetics. 2016 Aug;33:1115-9.

41. Zhang S, Lei C, Wu J, Zhou J, Sun H, Fu J, Sun Y, Sun X, Lu D, Zhang Y. The establishment and application of preimplantation genetic haplotyping in embryo diagnosis for reciprocal and Robertsonian translocation carriers. BMC medical genomics. 2017 Dec;10(1):1-9.

42. Tan YQ, Tan K, Zhang SP, Gong F, Cheng DH, Xiong B, Lu CF, Tang XC, Luo KL, Lin G, Lu GX. Single-nucleotide polymorphism microarray-based preimplantation genetic diagnosis is likely to improve the clinical outcome for translocation carriers. Human Reproduction. 2013 Sep 1;28(9):2581-92.

43. Chow JF, Yeung WS, Lee VC, Lau EY, Ng EH. Evaluation of preimplantation genetic testing for chromosomal structural rearrangement by a commonly used next generation sequencing workflow. European Journal of Obstetrics & Gynecology and Reproductive Biology. 2018 May 1;224:66-73.

44. Treff NR, Eccles J, Lello L, Bechor E, Hsu J, Plunkett K, Zimmerman R, Rana B, Samoilenko A, Hsu S, Tellier LC. Utility and first clinical application of screening embryos for polygenic disease risk reduction. Frontiers in endocrinology. 2019 Dec 4;10:845.

45. Kumar A, Im K, Banjevic M, Ng PC, Tunstall T, Garcia G, Galhardo L, Sun J, Schaedel ON, Levy B, Hongo D. Whole-genome risk prediction of common diseases in human preimplantation embryos. Nature medicine. 2022 Mar;28(3):513-6.

46. Yan L, Huang L, Xu L, Huang J, Ma F, Zhu X, et al. Live births after simultaneous avoidance of monogenic diseases and chromosome abnormality by next-generation sequencing with linkage analyses. Proc Natl Acad Sci U S A 2015;112:15964-9

47. Dokras A, Sargent IL, Ross C, Gardner RL, Barlow DH. Trophectoderm biopsy in human blastocysts. Hum Reprod 1990;5: 821-825.

48. Kokkali G, Vrettou C, Traeger-Synodinos J, Jones GM, Cram DS, Stavrou D, Trounson AO, Kanavakis E, Pantos K. Birth of a healthy infant following trophectoderm biopsy from blastocysts for PGD of β-thalassaemia major: case report. Hum Reprod 2005;20:1855-1859.

49. Palini S, Galluzzi L, De Stefani S, Bianchi M,Wells D, Magnani M, Bulletti C. Genomic DNA in human blastocoele fluid. Reprod Biomed Online 2013;26:603-610.

50. Gianaroli L, Magli MC, Pomante A, Crivello AM, Cafueri G, Valerio M, Ferraretti AP. Blastocentesis: a source of DNA for preimplantation genetic testing. Results from a pilot study. Fertil Steril 2014;102:1692-1699.

51. Tobler KJ, Zhao Y, Ross R, Benner AT, Xu X, Du L, Broman K, Thrift K, Brezina PR, Kearns WG. Blastocoel fluid from differentiated blastocysts harbors embryonic genomic material capable of a whole-genome deoxyribonucleic acid amplification and comprehensive chromosome microarray analysis. Fertil Steril 2015;104: 418-425.

52. Magli MC, Pomante A, Cafueri G, Valerio M, Crippa A, Ferraretti AP, Gianaroli L. Preimplantation genetic testing: Polar bodies, blastomeres, trophectoderm cells, or blastocoelic fluid? Fertil Steril 2016;105:676-683e5.

53. Zhang Y, Li N, Wang L, Sun H, Ma M, Wang H, Xu X, Zhang W, Liu Y, Cram DS et al. Molecular analysis of DNA in blastocoele fluid using next-generation sequencing. J Assist Reprod Genet 2016;33: 637-645.

54. Shangguan T, He W, Li H, Shang X, Liu Y, Bai X, Li M, Xie J. Detection and analysis of DNAmaterial in human blastocoel fluid. Biomed Genet Genomics 2017;2:1-5.

55. Capalbo A, Romanelli V, Patassini C, Poli M, Girardi L, Giancani A, Stoppa M, Cimadomo D, Ubaldi FM, Rienzi L. Diagnostic efficacy of blastocoel fluid and spent media as sources of DNA for preimplantation genetic testing in standard clinical conditions. Fertil Steril 2018;110:870-879.

56. Tšuiko O, Zhigalina DI, Jatsenko T, Skryabin NA, Kanbekova OR, Artyukhova VG, Svetlakov AV, Teearu K, Trošin A, Salumets A, Kurg A. Karyotype of the blastocoel fluid demonstrates low concordance with both trophectoderm and inner cell mass. Fertility and sterility. 2018 Jun 1;109(6):1127-34.

57. Magli MC, Albanese C, Crippa A, Tabanelli C, Ferraretti AP, Gianaroli L. Deoxyribonucleic acid detection in blastocoelic fluid: a new predictor of embryo ploidy and viable pregnancy. Fertil Steril 2018;111:77-85.

58. Galluzzi L, Palini S, De Stefani S, Andreoni F, Primiterra M, Diotallevi A, Bulletti C, Magnani M. Extracellular embryo genomic DNA and its potential for genotyping applications. Futur Sci OA 2015;1:FSO62.

59. Wu H, Ding C, Shen X, Wang J, Li R, Cai B, Xu Y, Zhong Y, Zhou C. Medium-based noninvasive preimplantation genetic diagnosis for human α-thalassemias-SEA. Medicine (Baltimore) 2015;94: e669.

60. Xu J, Fang R, Chen L, Chen D, Xiao J-P, Yang W, Wang H, Song X, Ma T, Bo S et al. Noninvasive chromosome screening of human embryos by genome sequencing of embryo culture medium for in vitro fertilization. Proc Natl Acad Sci 2016;113:11907-11912.

61. Shamonki MI, Jin H, Haimowitz Z, Liu L. Proof of concept: preimplantation genetic screening without embryo biopsy through analysis of cell-free DNA in spent embryo culture media. Fertil Steril 2016;106:1312-1318.

62. Feichtinger M, Vaccari E, Carli L, Wallner E, Mädel U, Figl K, Palini S, Feichtinger W. Non-invasive preimplantation genetic screening using array comparative genomic hybridization on spent culture media: a proof-of-concept pilot study. Reprod Biomed Online 2017;34:583-589.

63. Lane M, Zander-Fox DL, Hamilton H, Jasper MJ, Hodgson BL, Fraser M, Bell F. Ability to detect aneuploidy from cell free DNA collected from media is dependent on the stage of development of the embryo. Fertil Steril 2017;108:e61.

64. Liu WQ, Liu JQ, Du HZ, Ling JW, Sun XF, Chen DJ. Non-invasive preimplantation aneuploidy screening and diagnosis of beta thalassemia IVSII654 mutation using spent embryo culture medium. Ann Med 2017;49:319-328.

65. Ho JR, Arrach N, Rhodes-Long K, Ahmady A, Ingles S, Chung K, Bendikson KA, Paulson RJ, McGinnis LK. Pushing the limits of detection: investigation of cell-free DNA for aneuploidy screening in embryos. Fertil Steril 2018;110:467-475.e2.

66. Vera-Rodriguez M, Diez-Juan A, Jimenez-Almazan J, Martinez S, Navarro R, Peinado V, Mercader A, Meseguer M, Blesa D, Moreno I et al. Origin and composition of cell-free DNA in spent medium from human embryo culture during preimplantation development. Obstet Gynecol Surv 2018;33:745-756.

67. Capalbo A, Romanelli V, Patassini C, Poli M, Girardi L, Giancani A, Stoppa M, Cimadomo D, Ubaldi FM, Rienzi L. Diagnostic efficacy of blastocoel fluid and spent media as sources of DNA for preimplantation genetic testing in standard clinical conditions. Fertil Steril 2018;110:870-879.

68. Fang R, Yang W, Zhao X, Xiong F, Guo C, Xiao J, Chen L, Song X, Wang H, Chen J et al. Chromosome screening using culture medium of embryos fertilised in vitro: A pilot clinical study. J Transl Med 2019;17:1-8.

69. Huang L, Bogale B, Tang Y, Lu S, Xie XS, Racowsky C. Noninvasive preimplantation genetic testing for aneuploidy in spent medium may be more reliable than trophectoderm biopsy. Proc Natl Acad Sci 2019;116:201907472

70. Rubio C, Rienzi L, Navarro-Sánchez L, Cimadomo D, Garcia-Pascual CM, Albricci L, Soscia D, Valbuena D, Capalbo A, Ubaldi F et al. Embryonic cell-free DNA versus trophectoderm biopsy for aneuploidy testing: concordance rate and clinical implications. Fertil Steril 2019;112:510-519.

71. Yeung QSY, Zhang YX, Chung JPW, Lui WT, Kwok YKY, Gui B, Kong GWS, Cao Y, Li TC, Choy KW. A prospective study of non-invasive preimplantation genetic testing for aneuploidies (NiPGT-A) using next-generation sequencing (NGS) on spent culture media (SCM). J Assist Reprod Genet 2019;36:1609-1621.

72. Jiao J, Shi B, Sagnelli M, Yang D, Yao Y, LiW, Shao L, Lu S, Li D,Wang X. Minimally invasive preimplantation genetic testing using blastocyst culture medium. Hum Reprod 2019;34:1369-1379.

73. Ou Z, Deng Y, Liang Y, Chen Z, Sun L. Improved non-invasive preimplantation genetic testing for beta-thalassemia using spent embryo culture medium containing blastocoelic fluid. Frontiers in endocrinology. 2022 Jan 20;12:1941.

74. Galluzzi L, Palini S, De Stefani S, Andreoni F, Primiterra M, Diotallevi A, Bulletti C, Magnani M. Extracellular embryo genomic DNA and its potential for genotyping applications. Futur Sci OA 2015;1:FSO62.

75. Chan KA, Zhang J, Hui AB, Wong N, Lau TK, Leung TN, Lo KW, Huang DW, Lo YD. Size distributions of maternal and fetal DNA in maternal plasma. Clinical chemistry. 2004 Jan 1;50(1):88-92.

76. Huang L, Ma F, Chapman A, Lu S, Xie XS. Single-cell whole-genome amplification and sequencing: methodology and applications. Annual review of genomics and human genetics. 2015 Aug 24;16:79-102.

77. Chen C, Xing D, Tan L, Li H, Zhou G, Huang L, Xie XS. Single-cell whole-genome analyses by Linear Amplification via Transposon Insertion (LIANTI). Science. 2017 Apr 14;356(6334):189-94.

78. CHEN, Chongyi; XING, Dong; XIE, Xiaoliang Sunney. Methods of amplifying nucleic acid sequences mediated by transposase/transposon DNA complexes. U.S. Patent No 10,894,980, 2021.

79. Leaver M, Wells D. Non-invasive preimplantation genetic testing (niPGT): the next revolution in reproductive genetics?. Human reproduction update. 2020 Jan 1;26(1):16-42.

80. Cimadomo D, Rienzi L, Capalbo A, Rubio C, Innocenti F, Garcia-Pascual CM, Ubaldi FM, Handyside A. The dawn of the future: 30 years from the first biopsy of a human embryo. The detailed history of an ongoing revolution. Human Reproduction Update. 2020 Jul;26(4):453-73.

81. De Rycke M., Goossens V., Kokkali G., Meijer-Hoogeveen M., Coonen E., Moutou C. ESHRE PGD Consortium data collection XIV-XV: Cycles from January 2011 to December 2012 with pregnancy follow-up to October 2013. Hum. Reprod. 2017;32:1974-1994. doi: 10.1093/humrep/dex265.

82. ESHRE PGT-M Working Group, Carvalho F, Moutou C, Dimitriadou E, Dreesen J, Giménez C, Goossens V, Kakourou G, Vermeulen N, Zuccarello D, De Rycke M. ESHRE PGT Consortium good practice recommendations for the detection of monogenic disorders. Human reproduction open. 2020(3):1-18. Doi:10.1093/hropen/hoaa018.

83. Xue, A. et al. Genome-wide association analyses identify 143 risk variants and putative regulatory mechanisms for type 2 diabetes. Nat. Commun. 9, 2941 (2018).

84. Fan, H. C., Gu, W., Wang, J., Blumenfeld, Y. J., El-Sayed, Y. Y., & Quake, S. R. (2012). Non-invasive prenatal measurement of the fetal genome. Nature, 487(7407), 320-324.

85. Nabieva, E, Sharma, S M, Kapushev Y, et al. Accurate fetal variant calling in the presence of maternal cell contamination. European Journal of Human Genetics, 2020, 28(11): 1615-1623.

86. Lander, E.S. and Green, P. Construction of multilocus genetic linkage maps in humans, Proceedings of the National Academy of Sciences, 1987; 84 (8), 2363-2367

87. Kruglyak L, Daly M J, Reeve-Daly M P, et al. Parametric and nonparametric linkage analysis: a unified multipoint approach. American journal of human genetics, 1996, 58(6): 1347.

88. Idury R M, Elston R C. A faster and more general hidden Markov model algorithm for multi- point likelihood calculations. Human heredity, 1997, 47(4): 197-202.

89. Kruglyak L, Lander E S. Faster multipoint linkage analysis using Fourier transforms. J. Comput. Biol. 1998;5(1):1-7.

90. Abecasis G R, Cherny S S, Cookson W O, et al. Merlin-rapid analysis of dense genetic maps using sparse gene flow trees. Nature genetics, 2002, 30(1): 97-101.

91. Shitara, Akihiro, et al. Cell-free DNA in spent culture medium effectively reflects the chromosomal status of embryos following culturing beyond implantation compared to trophectoderm biopsy. PLoS One 16.2 (2021): e0246438.

92. Yin, Baoli, et al. Validation of preimplantation genetic tests for aneuploidy (PGT-A) with DNA from spent culture media (SCM): concordance assessment and implication. Reproductive Biology and Endocrinology 19.1 (2021): 41.

## Claims

1. A method for amplifying the DNA in a solution, wherein the DNA content in the solution can be at the minimum of picogram level, the method comprising the following steps:

    combining lysis buffer with the solution, heating the solution, then combining protease with the solution, incubating the solution, followed by inactivation of the protease under high temperatures to obtain lysate;

combining transposome with the lysate, wherein the transposome includes a transposase and a transposon, wherein the transposon comprises a transposase binding site and an RNA polymerase promoter sequence, wherein

the transposome binds to DNA in the solution, the transposase cuts the DNA in the solution to produce DNA fragments and the transposon becomes incorporated or inserted into the DNA fragments;

a 9-bp gap resulting from transposon insertion is filled and extended down to both ends of each fragment;

performing in vitro transcription using the original sequence as a template to form RNA;

combining the RNA with a DNA primer and a reverse transcription system to form a first strand cDNA via reverse transcription, wherein the DNA primer is complementary to the respective RNA strand of the sequence of transposase binding site; and

forming a second strand DNA via cycling amplification.

2. The method of claim 1, wherein:

(i) the solution is a culture medium or a blastocoele fluid, and wherein preferably the 3'-end of the DNA primer contains the sequence of 5'-GACAG-3' or 5'-CTGTC-3', wherein more preferably the DNA primer contains the sequence set forth as 5'-AGATGTGTATAAGAGACAG-3' (SEQ ID NO.: 1), or 5'-TCTACACATATTCTCTGTC-3' (SEQ ID NO.: 2) or is at least 70%, preferably 80%, preferably 90%, preferably 95%, preferably 100% identical with SEQ ID NO.: 1 or SEQ ID NO.: 2;

(ii) the DNA primer has a length of 15bp to 20bp;

(iii) the lysis buffer is 2x to 10x lysis buffer;

(iv) the working concentration of the protease in the lysis mixture is higher than 0.6 mg/mL, preferably in the range of 0.8-2 mg/mL;

(v) the incubation is performed at 45-60°C for 1 to 3 hours;

(vi) the inactivation is performed at 80-90°C;

(vii) the working concentration of the transposome is higher than 6 nM, preferably in the range of 10-40 nM; or

(viii) the cycling amplification is performed more than 4 cycles, such as 4 to 20 cycles, preferably 6 to 15 cycles.

3. The method of claims 1 or 2 comprising the following steps:

combining 2x to 10x lysis buffer with the culture medium, heating the culture medium, combining protease with the culture medium to the working concentration of 0.8-2 mg/mL and incubating the culture medium at 45-60°C for 1 to 3 hours, followed by inactivation of the protease at 80-90°C to obtain lysate; combining transposome with the lysate to the working concentration of 10-40 nM, wherein the transposome includes a transposase and a transposon, wherein the transposon comprises a transposase binding site and an RNA polymerase promoter sequence, wherein the transposome binds to DNA in the solution, the transposase cuts the DNA in the solution to produce DNA fragments and the transposon becomes incorporated or inserted into the DNA fragments;

a 9-bp gap resulting from transposon insertion is filled and extended down to both ends of each fragment;

performing in vitro transcription using the original sequence as a template to form RNA;

combining the RNA with DNA primer pairing with the nucleic acid sequence conjugated with the transposome and a reverse transcription system, forming a first strand cDNA via reverse transcription, wherein the DNA primer contains the sequence set forth as 5'-AGATGTGTATAAGAGACAG-3' (SEQ ID NO.: 1) or 5'-TCTACACATAT TCTCTGTC-3' (SEQ ID NO.: 2), or is at least 70%, preferably 80%, preferably 90%, preferably 95%, preferably 100% identity with SEQ ID NO.: 1 or SEQ ID NO.: 2; and

forming a second strand DNA via 6 to 15 cycles of amplification.

4. A method of analyzing a biological sample of culture medium of an in vitro cultured embryo to determine the susceptibility of a genetic disease in the embryo, comprising:

1) Amplifying the DNA molecules from the biological sample of culture medium of the in vitro cultured embryo using the method according to any one of claims 1-3;

2) Preparing DNA libraries and sequencing the DNA molecules from the biological sample to obtain a plurality of sequence reads;

3) Performing mapping and SNP calling, by a computer system, to generate the SNP data and controlling its quality;

4) Conducting haplotype pre-phasing, by a computer system, for the blood samples of both parents or solely the disease-carrying parent;

5) Calculating the likelihood of the four possible inheritance scenarios at each single SNP representing whether the genetic chains are inherited from the paternal or maternal alleles of the parents, taking into account the maternal cell contamination (MCC) rate and haplotype status;

6) Estimating the MCC rate, and determining the haplotype status of each SNP to represent whether only DNA from one parent is present in the culture medium;

7) Calculating the likelihood of observing the SNP data within regions adjacent to the disease-causing mutation site, combining the likelihood at each single SNP and the recombination probabilities;

8) Determining whether the embryo carries the disease-causing chromosome, and providing a level of confidence.

5. A computer program comprising a plurality of instructions capable of being executed by a computer system and arranged when executed to control the computer system to analyze a biological sample of culture medium of an in vitro cultured embryo to determine the susceptibility of a genetic disease in the embryo, configured, when executed, to cause the computer system to perform:

1) Perform mapping and SNP calling, by a computer system, on a plurality of sequence reads to generate the SNP data and controlling its quality, wherein the plurality of sequence reads was obtained by preparing DNA libraries and sequencing DNA molecules amplified by the method according to any of claims 1 to 3 from the biological sample of culture medium of the in vitro cultured embryo;

2) Conduct haplotype pre-phasing, by a computer system, for the blood samples of both parents or solely the disease-carrying parent;

3) Calculate the likelihood of the four possible inheritance scenarios at each single SNP representing whether the genetic chains are inherited from the paternal or maternal alleles of the parents, taking into account the maternal cell contamination (MCC) rate and haplotype status;

4) Estimate the MCC rate, and determining the haplotype status of each SNP to represent whether only DNA from one parent is present in the culture medium;

5) Calculate the likelihood of observing the SNP data within regions adjacent to the disease-causing mutation site, combining the likelihood at each single SNP and the recombination probabilities;

6) Determine whether the embryo carries the disease-causing chromosome, and providing a level of confidence.

6. The method of claim 4, or the computer program of claim 5, wherein:

(i) the embryo has contamination caused by maternal cells and/or haplotype loss;

(ii) in step 3) of the method or step 1) caused by the computer program, mapping and SNP calling outputs the physical location, allele type, allele depth and sequencing quality for each SNP of the biological sample of culture medium of the in vitro cultured embryo;

(iii) in step 3) of the method or step 1) caused by the computer program, controlling the quality of the SNP data is performed via filtering out the SNPs with low gene quality (GQ) values of the biological sample of culture medium of the in vitro cultured embryo, preferably filtering out the SNPs with the lowest 20% GQ values of the biological sample of culture medium of the in vitro cultured embryo;

(iv) wherein in step 3) of the method or step 1) caused by the computer program, estimating the sequencing error rate and the relative density of SNPs in the region adjacent to the disease-causing mutation site, and excluding the sample of the culture medium with an extremely low SNP density, or high sequencing error rate, wherein preferably the excluded SNP density is smaller than 0.001, or the excluded sequencing error rate is larger than 0.2;

(v) in step 4) of the method or step 2) caused by the computer program, employing any one or more of the following samples in the family: the blood sample of proband, or the blood sample of at least one of the grandparents;

(vi) in step 5) of the method or step 3) caused by the computer program, integrating the estimated sequencing error rate when calculating the likelihood for each individual SNP;

(vii) step 6) of the method or step 4) caused by the computer program further include the following steps:

a) Initially estimating the MCC rate and haplotype status of each SNP in the region adjacent to the disease-causing mutation site; and

b) Recursively updating the MCC rate and haplotype status of each SNP, wherein preferably step b) using a label-searching method or after step a), excluding the sample with abnormal MCC rate, wherein preferably the abnormal MCC rate is larger than 0.65 or less than - 0.5;

(viii) in step 7) of the method or step 5) caused by the computer program, using a Bayesian model and a recursive algorithm;

(ix) in step 8) of the method or step 6) caused by the computer program, first plotting the log likelihood curve for both upstream and downstream regions of the disease-causing mutation site; or

(x) the genetic disease includes both monogenic and polygenic diseases.

7. The method of claims 4 or 6, or the computer program of claim 5 or 6, wherein after step 8) of the method or step 6) caused by the computer program, combining multiple identified SNPs of the embryo to determine its susceptibility to a polygenic disease.

8. Computer system for analyzing a biological sample of culture medium of an in vitro cultured embryo to determine the susceptibility of a genetic disease in the embryo, comprising:

1) Means for performing mapping and SNP calling, by a computer system, on a plurality of sequence reads to generate the SNP data and controlling its quality, wherein the plurality of sequence reads was obtained by preparing DNA libraries and sequencing DNA molecules amplified by the method according to any of claims 1 to 3 from the biological sample of culture medium of the in vitro cultured embryo;

2) Means for conducting haplotype pre-phasing, by a computer system, for the blood samples of both parents or solely the disease-carrying parent;

3) Means for calculating the likelihood of the four possible inheritance scenarios at each single SNP representing whether the genetic chains are inherited from the paternal or maternal alleles of the parents, taking into account the maternal cell contamination (MCC) rate and haplotype status;

4) Means for estimating the MCC rate, and determining the haplotype status of each SNP to represent whether only DNA from one parent is present in the culture medium;

5) Means for calculating the likelihood of observing the SNP data within regions adjacent to the disease-causing mutation site, combining the likelihood at each single SNP and the recombination probabilities;

6) Means for determining whether the embryo carries the disease-causing chromosome, and providing a level of confidence.

9. The computer system of claim 8, wherein:

(i) the embryo has contamination caused by maternal cells and/or haplotype loss;

(ii) in means 1) mapping and SNP calling outputs the physical location, allele type, allele depth and sequencing quality for each SNP of the biological sample of culture medium of the in vitro cultured embryo;

(iii) in means 1) controlling the quality of the SNP data is performed via filtering out the SNPs with low gene quality (GQ) values of the biological sample of culture medium of the in vitro cultured embryo;

(iv) filtering out the SNPs with the lowest 20% GQ values of the biological sample of culture medium of the in vitro cultured embryo;

(v) in means 1), estimating the sequencing error rate and the relative density of SNPs in the region adjacent to the disease-causing mutation site, and excluding the sample of the culture medium with an extremely low SNP density, or high sequencing error rate, wherein preferably the excluded SNP density is smaller than 0.001, or the excluded sequencing error rate is larger than 0.2;

(vi) in means 2) employing any one or more of the following samples in the family: the blood sample of proband, or the blood sample of at least one of the grandparents; or

(vii) in means 3), integrating the estimated sequencing error rate when calculating the likelihood for each individual SNP.

10. The computer system of claim 8, wherein means 4) further includes the following functions:

a) Initially estimating the MCC rate and haplotype status of each SNP in the region adjacent to the disease-causing mutation site; and

b) Recursively updating the MCC rate and haplotype status of each SNP, wherein preferably:

(i) function b) using a label-searching method; or

(ii) after function a), excluding the sample with abnormal MCC rate, wherein preferably the abnormal MCC rate is larger than 0.65 or less than -0.5.

11. The computer system of claim 8, wherein:

(i) in means 5) using a Bayesian model and a recursive algorithm;

(ii) in means 6), first plotting the log likelihood curve for both upstream and downstream regions of the disease-causing mutation site; or

(iii) the genetic disease includes both monogenic and polygenic diseases.

12. The computer system of any one of claims 8 to 11, further comprising means for combining multiple identified SNPs of the embryo to determine its susceptibility to a polygenic disease.

**Patentansprüche**

1. Verfahren zum Amplifizieren der DNA in einer Lösung, wobei der DNA-Gehalt in der Lösung mindestens auf Pikogramm-Niveau sein kann, wobei das Verfahren die folgenden Schritte umfasst:

Vereinigen von Lysepuffer mit der Lösung, Erhitzen der Lösung, dann Vereinigen von Protease mit der Lösung, Inkubieren der Lösung, gefolgt von Inaktivierung der Protease bei hohen Temperaturen, um Lysat zu erhalten; Vereinigen von Transposom mit dem Lysat, wobei das Transposom eine Transposase und ein Transposon aufweist, wobei das Transposon eine Transposase-Bindungsstelle und eine RNA-Polymerase-Promotorsequenz umfasst, wobei das Transposom an DNA in der Lösung bindet, die Transposase die DNA in der Lösung schneidet, um DNA-Fragmente zu erzeugen, und das Transposon in die DNA-Fragmente inkorporiert oder eingefügt wird;

eine 9-bp-Lücke, die aus der Transposon-Insertion resultiert, wird aufgefüllt und bis zu beiden Enden jedes Fragments verlängert;

Durchführen von In-vitro-Transkription unter Verwendung der ursprünglichen Sequenz als eine Matrize, um RNA zu bilden;

Vereinigen der RNA mit einem DNA-Primer und einem reversen Transkriptionssystem, um eine Erststrang-cDNA mittels reverser Transkription zu bilden, wobei der DNA-Primer komplementär zu dem jeweiligen RNA-Strang der Sequenz der Transposase-Bindungsstelle ist; und

Bilden einer Zweitstrang-DNA mittels zyklischer Amplifikation.

2. Verfahren nach Anspruch 1, wobei:

(i) die Lösung ein Kulturmedium oder eine Blastocoelflüssigkeit ist, und wobei vorzugsweise das 3'-Ende des DNA-Primers die Sequenz von 5'-GACAG-3' oder 5'-CTGTC-3' enthält, wobei noch bevorzugter der DNA-Primer die als 5'-AGATGTGTATAAGAGACAG-3' (SEQ ID NO.: 1) oder 5'-TCTACACATATTCTCTGTC-3' (SEQ ID NO.: 2) angegebene Sequenz enthält oder zu mindestens 70 %, vorzugsweise 80 %, vorzugsweise 90 %, vorzugsweise 95 %, vorzugsweise 100 % identisch mit SEQ ID NO.: 1 oder SEQ ID NO.: 2 ist;

(ii) der DNA-Primer eine Länge von 15 bp bis 20 bp hat;

(iii) der Lysepuffer 2x- bis 10x-Lysepuffer ist;

(iv) die Arbeitskonzentration der Protease in der Lysemischung höher als 0,6 mg/ml ist, vorzugsweise im Bereich von 0,8-2 mg/ml;

(v) die Inkubation bei 45-60 °C für 1 bis 3 Stunden durchgeführt wird;

(vi) die Inaktivierung bei 80-90 °C durchgeführt wird;

(vii) die Arbeitskonzentration des Transposoms höher als 6 nM ist, vorzugsweise im Bereich von 10-40 nM; oder

(viii) die zyklische Amplifikation über mehr als 4 Zyklen, wie z. B. 4 bis 20 Zyklen, vorzugsweise 6 bis 15 Zyklen, durchgeführt wird.

3. Verfahren nach den Ansprüchen 1 oder 2, umfassend die folgenden Schritte:

Vereinigen von 2x- bis 10x-Lysepuffer mit dem Kulturmedium, Erhitzen des Kulturmediums, Vereinigen von Protease mit dem Kulturmedium auf die Arbeitskonzentration von 0,8-2 mg/ml und Inkubieren des Kulturmediums bei 45-60 °C für 1 bis 3 Stunden, gefolgt von Inaktivierung der Protease bei 80-90 °C, um Lysat zu erhalten;

Vereinigen von Transposom mit dem Lysat auf die Arbeitskonzentration von 10-40 nM, wobei das Transposom eine Transposase und ein Transposon aufweist, wobei das Transposon eine Transposase-Bindungsstelle und eine RNA-Polymerase-Promotorsequenz umfasst, wobei das Transposom an DNA in der Lösung bindet, die Transposase die DNA in der Lösung schneidet, um DNA-Fragmente zu erzeugen, und das Transposon in die DNA-Fragmente inkorporiert oder eingefügt wird;

eine 9-bp-Lücke, die aus der Transposon-Insertion resultiert, wird aufgefüllt und bis zu beiden Enden jedes Fragments verlängert;

Durchführen von In-vitro-Transkription unter Verwendung der ursprünglichen Sequenz als eine Matrize, um RNA zu bilden;

Vereinigen der RNA mit einem DNA-Primer, der mit der mit dem Transposom konjugierten Nukleinsäuresequenz paart, und einem reversen Transkriptionssystem, Bilden einer Erststrang-cDNA mittels reverser Transkription, wobei der DNA-Primer die als 5'-AGATGTGTATAAGAGACAG-3' (SEQ ID NO.: 1) oder 5'-TCTACACATATTC TCTGTC-3' (SEQ ID NO.: 2) angegebene Sequenz enthält, oder zu mindestens 70 %, vorzugsweise 80 %, vorzugsweise 90 %, vorzugsweise 95, vorzugsweise 100 % Identität mit SEQ ID NO.: 1 oder SEQ ID NO.: 2 aufweist; und

Bilden einer Zweitstrang-DNA mittels 6 bis 15 Amplifikationszyklen.

4. Verfahren zum Analysieren einer biologischen Probe von Kulturmedium eines in vitro kultivierten Embryos, um die Anfälligkeit für eine genetische Erkrankung bei dem Embryo zu bestimmen, umfassend:

1) Amplifizieren der DNA-Moleküle aus der biologischen Probe von Kulturmedium des in vitro kultivierten Embryos unter Verwendung des Verfahrens nach einem der Ansprüche 1-3;

2) Herstellen von DNA-Bibliotheken und Sequenzieren der DNA-Moleküle aus der biologischen Probe, um eine Vielzahl von gelesenen Sequenzen zu erhalten;

3) Durchführen von Mapping und SNP-Calling durch ein Computersystem, um die SNP-Daten zu erzeugen und deren Qualität zu kontrollieren;

4) Durchführen von Haplotyp-Prä-Phasing durch ein Computersystem für die Blutproben beider Elternteile oder ausschließlich des krankheitstragenden Elternteils;

5) Berechnen der Wahrscheinlichkeit der vier möglichen Vererbungsszenarien an jedem einzelnen SNP, die darstellen, ob die genetischen Ketten von den väterlichen oder mütterlichen Allelen der Eltern vererbt werden, unter Berücksichtigung der mütterlichen Zellkontaminationsrate (MCC) und des Haplotyp-Status;

6) Schätzen der MCC-Rate und Bestimmen des Haplotyp-Status jedes SNP, um darzustellen, ob nur DNA von einem Elternteil im Kulturmedium vorhanden ist;

7) Berechnen der Wahrscheinlichkeit, dass die SNP-Daten innerhalb von Regionen, die an die krankheitsverursachende Mutationsstelle angrenzen, beobachtet werden, Kombinieren der Wahrscheinlichkeit an jedem einzelnen SNP und der Rekombinationswahrscheinlichkeiten;

8) Bestimmen, ob der Embryo das krankheitsverursachende Chromosom trägt, und Bereitstellen eines Konfidenzniveaus.

5. Computerprogramm, das eine Vielzahl von Anweisungen umfasst, die von einem Computersystem ausgeführt werden können und angeordnet sind, wenn sie ausgeführt werden, um das Computersystem zu steuern, um eine biologische Probe von Kulturmedium eines in vitro kultivierten Embryos zu analysieren, um die Anfälligkeit für eine genetische Erkrankung bei dem Embryo zu bestimmen, die konfiguriert sind, wenn sie ausgeführt werden, das Computersystem zu veranlassen, folgendes durchzuführen:

1) Durchführen von Mapping und SNP-Calling durch ein Computersystem an einer Vielzahl von gelesenen Sequenzen, um die SNP-Daten zu erzeugen und deren Qualität zu kontrollieren, wobei die Vielzahl von gelesenen Sequenzen durch Herstellen von DNA-Bibliotheken und Sequenzieren von DNA-Molekülen erhalten wurde, die durch das Verfahren nach einem der Ansprüche 1 bis 3 aus der biologischen Probe von Kulturmedium des in vitro kultivierten Embryos amplifiziert wurden;

2) Durchführen von Haplotyp-Prä-Phasing durch ein Computersystem für die Blutproben beider Elternteile oder ausschließlich des krankheitstragenden Elternteils;

3) Berechnen der Wahrscheinlichkeit der vier möglichen Vererbungsszenarien an jedem einzelnen SNP, die darstellen, ob die genetischen Ketten von den väterlichen oder mütterlichen Allelen der Eltern vererbt werden, unter Berücksichtigung der mütterlichen Zellkontaminationsrate (MCC) und des Haplotyp-Status;

4) Schätzen der MCC-Rate und Bestimmen des Haplotyp-Status jedes SNP, um darzustellen, ob nur DNA von einem Elternteil im Kulturmedium vorhanden ist;

5) Berechnen der Wahrscheinlichkeit, dass die SNP-Daten innerhalb von Regionen, die an die krankheitsverursachende Mutationsstelle angrenzen, beobachtet werden, Vereinigen der Wahrscheinlichkeit an jedem einzelnen SNP und der Rekombinationswahrscheinlichkeiten;

6) Bestimmen, ob der Embryo das krankheitsverursachende Chromosom trägt, und Bereitstellen eines Konfidenzniveaus.

6. Verfahren nach Anspruch 4 oder Computerprogramm nach Anspruch 5, wobei:

(i) der Embryo eine durch mütterliche Zellen verursachte Kontamination und/oder einen Haplotyp-Verlust aufweist;

(ii) in Schritt 3) des Verfahrens oder Schritt 1), der durch das Computerprogramm verursacht wird, Mapping und SNP-Calling die physikalische Position, den Alleltyp, die Alleltiefe und die Sequenzierqualität für jeden SNP der biologischen Probe von Kulturmedium des in vitro kultivierten Embryos ausgeben;

(iii) in Schritt 3) des Verfahrens oder Schritt 1), der durch das Computerprogramm verursacht wird, das Kontrollieren der Qualität der SNP-Daten durch Herausfiltern der SNPs mit niedrigen Genqualitätswerten (GQ) der biologischen Probe von Kulturmedium des in vitro kultivierten Embryos durchgeführt wird, vorzugsweise durch das Herausfiltern der SNPs mit den niedrigsten 20 % GQ-Werten der biologischen Probe von Kulturmedium des in vitro kultivierten Embryos;

(iv) wobei in Schritt 3) des Verfahrens oder Schritt 1), der durch das Computerprogramm verursacht wird, Schätzen der Sequenzierfehlerrate und der relativen Dichte von SNPs in der Region, die an die krankheitsverursachende Mutationsstelle angrenzt, und Ausschließen der Probe des Kulturmediums mit einer extrem niedrigen SNP-Dichte oder hohen Sequenzierfehlerrate, wobei vorzugsweise die ausgeschlossene SNP-Dichte kleiner als 0,001 oder die ausgeschlossene Sequenzierfehlerrate größer als 0,2 ist;

(v) in Schritt 4) des Verfahrens oder Schritt 2), der durch das Computerprogramm verursacht wird, Verwenden einer oder mehrerer der folgenden Proben in der Familie: die Blutprobe des Probanden oder die Blutprobe von mindestens einem der Großeltern;

(vi) in Schritt 5) des Verfahrens oder Schritt 3), der durch das Computerprogramm verursacht wird, Integrieren der geschätzten Sequenzierfehlerrate bei der Berechnung der Wahrscheinlichkeit für jeden einzelnen SNP;

(vii) Schritt 6) des Verfahrens oder Schritt 4), der durch das Computerprogramm verursacht wird, ferner die folgenden Schritte aufweist:

a) anfängliches Schätzen der MCC-Rate und des Haplotyp-Status jedes SNP in der Region, die an die krankheitsverursachende Mutationsstelle angrenzt; und

b) rekursives Aktualisieren der MCC-Rate und des Haplotyp-Status jedes SNP, wobei vorzugsweise Schritt b) ein Kennzeichnungs-Such-Verfahren verwendet oder nach Schritt a), Ausschließen der Probe mit abnormaler MCC-Rate, wobei vorzugsweise die abnormale MCC-Rate größer als 0,65 oder kleiner als -0,5 ist;

(viii) in Schritt 7) des Verfahrens oder Schritt 5), der durch das Computerprogramm verursacht wird, Verwenden eines Bayes'schen Modells und eines rekursiven Algorithmus;

(ix) in Schritt 8) des Verfahrens oder Schritt 6), der durch das Computerprogramm verursacht wird, zuerst Auftragen der Log-Plausibilitäts-Kurve für sowohl vorgeschaltete als auch nachgeschaltete Regionen der krankheitsverursachenden Mutationsstelle; oder

(x) die genetische Erkrankung sowohl monogene als auch polygene Erkrankungen aufweist.

7. Verfahren nach den Ansprüchen 4 oder 6 oder Computerprogramm nach Anspruch 5 oder 6, wobei nach Schritt 8) des Verfahrens oder Schritt 6), der durch das Computerprogramm verursacht wird, mehrere identifizierter SNPs des Embryos kombiniert werden, um seine Anfälligkeit für eine polygene Erkrankung zu bestimmen.

8. Computersystem zum Analysieren einer biologischen Probe von Kulturmedium eines in vitro kultivierten Embryos, um die Anfälligkeit für eine genetische Erkrankung bei dem Embryo zu bestimmen, umfassend:

1) Mittel zum Durchführen von Mapping und SNP-Calling durch ein Computersystem an einer Vielzahl von gelesenen Sequenzen, um die SNP-Daten zu erzeugen und deren Qualität zu kontrollieren, wobei die Vielzahl von gelesenen Sequenzen durch Herstellen von DNA-Bibliotheken und Sequenzieren von DNA-Molekülen erhalten wurde, die durch das Verfahren nach einem der Ansprüche 1 bis 3 aus der biologischen Probe von Kulturmedium des in vitro kultivierten Embryos amplifiziert wurden;

2) Mittel zum Durchführen von Haplotyp-Prä-Phasing durch ein Computersystem für die Blutproben beider Elternteile oder ausschließlich des krankheitstragenden Elternteils;

3) Mittel zum Berechnen der Wahrscheinlichkeit der vier möglichen Vererbungsszenarien an jedem einzelnen SNP, die darstellen, ob die genetischen Ketten von den väterlichen oder mütterlichen Allelen der Eltern vererbt werden, unter Berücksichtigung der mütterlichen Zellkontaminationsrate (MCC) und des Haplotyp-Status;

4) Mittel zum Schätzen der MCC-Rate und zum Bestimmen des Haplotyp-Status jedes SNP, um darzustellen, ob nur DNA von einem Elternteil im Kulturmedium vorhanden ist;

5) Mittel zum Berechnen der Wahrscheinlichkeit, dass die SNP-Daten innerhalb von Regionen, die an die krankheitsverursachende Mutationsstelle angrenzen, beobachtet werden, Vereinigen der Wahrscheinlichkeit an

EP 4 569 133 B1

jedem einzelnen SNP und der Rekombinationswahrscheinlichkeiten;
6) Mittel zum Bestimmen, ob der Embryo das krankheitsverursachende Chromosom trägt, und zum Bereitstellen eines Konfidenzniveaus.

**9.** Computersystem nach Anspruch 8, wobei:

(i) der Embryo eine durch mütterliche Zellen verursachte Kontamination und/oder einen Haplotyp-Verlust aufweist;
(ii) in Mittel 1), Mapping und SNP-Calling die physikalische Position, den Alleltyp, die Alleltiefe und die Sequenzierqualität für jeden SNP der biologischen Probe von Kulturmedium des in vitro kultivierten Embryos ausgeben;
(iii) in Mittel 1), das Kontrollieren der Qualität der SNP-Daten durch Herausfiltern der SNPs mit niedrigen Genqualitätswerten (GQ) der biologischen Probe von Kulturmedium des in vitro kultivierten Embryos durchgeführt wird;
(iv) die SNPs mit den niedrigsten 20 % GQ-Werten der biologischen Probe von Kulturmedium des in vitro kultivierten Embryos herausgefiltert werden;
(v) in Mittel 1), Schätzen der Sequenzierfehlerrate und der relativen Dichte von SNPs in der Region, die an die krankheitsverursachende Mutationsstelle angrenzt, und Ausschließen der Probe des Kulturmediums mit einer extrem niedrigen SNP-Dichte oder hohen Sequenzierfehlerrate, wobei vorzugsweise die ausgeschlossene SNP-Dichte kleiner als 0,001 oder die ausgeschlossene Sequenzierfehlerrate größer als 0,2 ist;
(vi) in Mittel 2), Verwenden einer oder mehrerer der folgenden Proben in der Familie: die Blutprobe des Probanden oder die Blutprobe von mindestens einem der Großeltern; oder
(vii) in Mittel 3), Integrieren der geschätzten Sequenzierfehlerrate bei der Berechnung der Wahrscheinlichkeit für jeden einzelnen SNP.

**10.** Computersystem nach Anspruch 8, wobei Mittel 4) ferner die folgenden Funktionen aufweist:

a) anfängliches Schätzen der MCC-Rate und des Haplotyp-Status jedes SNP in der Region, die an die krankheitsverursachende Mutationsstelle angrenzt; und
b) rekursives Aktualisieren der MCC-Rate und des Haplotyp-Status jedes SNP, wobei vorzugsweise:

(i) Funktion b) ein Kennzeichnungs-Such-Verfahren verwendet; oder
(ii) nach Funktion a), Ausschließen der Probe mit abnormaler MCC-Rate, wobei vorzugsweise die abnormale MCC-Rate größer als 0,65 oder kleiner als -0,5 ist.

**11.** Computersystem nach Anspruch 8, wobei:

(i) in Mittel 5) ein Bayes'sches Modell und ein rekursiver Algorithmus verwendet wird;
(ii) in Mittel 6), zuerst die Log-Plausibilitäts-Kurve für sowohl vorgeschaltete als auch nachgeschaltete Regionen der krankheitsverursachenden Mutationsstelle aufgetragen wird; oder
(iii) die genetische Erkrankung sowohl monogene als auch polygene Erkrankungen aufweist.

**12.** Computersystem nach einem der Ansprüche 8 bis 11, ferner umfassend Mittel zum Kombinieren mehrerer identifizierter SNPs des Embryos, um seine Anfälligkeit für eine polygene Erkrankung zu bestimmen.

**Revendications**

**1.** Méthode pour amplifier l'ADN dans une solution, dans laquelle la teneur en ADN de la solution peut être au minimum de l'ordre du picogramme, la méthode comprenant les étapes suivantes :

combinaison d'un tampon de lyse avec la solution, chauffage de la solution, puis combinaison d'une protéase avec la solution, incubation de la solution, et ensuite inactivation de la protéase à température élevée pour que soit obtenu un lysat ;
combinaison d'un transposome avec le lysat, dans laquelle le transposome inclut une transposase et un transposon, dans laquelle le transposon comprend un site de liaison à une transposase et une séquence de promoteur d'ARN polymérase, dans laquelle le transposome se lie à l'ADN dans la solution, la transposase coupe l'ADN dans la solution pour produire des fragments d'ADN et le transposon devient incorporé ou inséré dans les fragments d'ADN ;

une brèche de 9 pb résultant de l'insertion d'un transposon est comblée et prolongée jusqu'aux deux extrémités de chaque fragment ;

mise en oeuvre d'une transcription in vitro utilisant la séquence originale en tant que matrice pour former un ARN ;

combinaison de l'ARN avec une amorce ADN et un système de transcription inverse pour former un premier brin d'ADNc via transcription inverse, dans laquelle l'amorce ADN est complémentaire du brin d'ARN respectif de la séquence du site de liaison à une transposase ; et

formation d'un deuxième brin d'ADN via amplification par cyclage.

2. Méthode selon la revendication 1, dans laquelle :

(i) la solution est un milieu de culture ou un liquide blastocœlien, dans laquelle de préférence l'extrémité 3' de l'amorce ADN contient la séquence 5'-GACAG-3' ou 5'-CTGTC-3', dans laquelle mieux encore l'amorce ADN contient la séquence représentée par 5'-AGATGTGTATAAGAGACAG-3' (SEQ ID NO : 1) ou 5'-TCTACACAT ATTCTCTGTC-3' (SEQ ID NO : 2) ou est identique à au moins 70 %, de préférence 80 %, de préférence 90 %, de préférence 95 %, de préférence 100 % à la SEQ ID NO : 1 ou à la SEQ ID NO : 2 ;
(ii) l'amorce ADN a une longueur de 15 pb à 20 pb ;
(iii) le tampon de lyse est un tampon de lyse 2x à 10x ;
(iv) la concentration de travail de la protéase dans le mélange de lyse est supérieure à 0,6 mg/ml, de préférence située dans la plage allant de 0,8 à 2 mg/ml ;
(v) l'incubation est effectuée à 45-60 °C pendant 1 à 3 heures ;
(vi) l'inactivation est effectuée à 80-90 °C ;
(vii) la concentration de travail du transposome est supérieure à 6 nM, de préférence située dans la plage allant de 10 à 40 nM ; ou
(viii) l'amplification par cyclage est effectuée sur plus de 4 cycles, tels que 4 à 20 cycles, de préférence 6 à 15 cycles.

3. Méthode selon la revendication 1 ou 2, comprenant les étapes suivantes :

combinaison de tampon de lyse 2x à 10x avec le milieu de culture, chauffage du milieu de culture, combinaison d'une protéase avec le milieu de culture jusqu'à la concentration de travail de 0,8 à 2 mg/ml, et incubation du milieu de culture à 45-60 °C pendant 1 à 3 heures, après quoi inactivation de la protéase à 80-90 °C pour que soit obtenu un lysat ;

combinaison d'un transposome avec le lysat jusqu'à la concentration de travail de 10 à 40 nM, dans laquelle le transposome inclut une transposase et un transposon, dans laquelle le transposon comprend un site de liaison à une transposase et une séquence de promoteur d'ARN polymérase, dans laquelle le transposome se lie à l'ADN dans la solution, la transposase coupe l'ADN dans la solution pour produire des fragments d'ADN et le transposon devient incorporé ou inséré dans les fragments d'ADN ;

une brèche de 9 pb résultant de l'insertion d'un transposon est comblée et prolongée jusqu'aux deux extrémités de chaque fragment ;

mise en oeuvre d'une transcription in vitro utilisant la séquence originale en tant que matrice pour former un ARN ;

combinaison de l'ARN avec une amorce ADN s'appariant à la séquence d'acide nucléique conjuguée au transposome et un système de transcription inverse, ce qui forme un premier brin d'ADNc via transcription inverse, dans laquelle l'amorce d'ADN contient la séquence représentée par 5'-AGATGTGTATAAGAGACAG-3' (SEQ ID NO : 1) ou 5'-TCTACACATATTCTCTGTC-3' (SEQ ID NO : 2) ou est d'au moins 70 %, de préférence 80 %, de préférence 90 %, de préférence 95 %, de préférence 100 % à la SEQ ID NO : 1 ou à la SEQ ID NO : 2 ; et

formation d'un deuxième brin d'ADN via 6 à 15 cycles d'amplification.

4. Méthode d'analyse d'un échantillon biologique de milieu de culture d'un embryon cultivé in vitro pour déterminer la susceptibilité d'une maladie génétique dans l'embryon, comprenant :

1) l'amplification de molécules d'ADN provenant de l'échantillon biologique de milieu de culture de l'embryon cultivé in vitro utilisant la méthode de l'une quelconque des revendications 1 à 3 ;
2) préparation de bibliothèques d'ADN et séquençage des molécules d'ADN provenant de l'échantillon biologique pour que soit obtenue une pluralité de lectures de séquences ;
3) mise en œuvre d'un alignement et d'une détection de SNP, par un système informatique, pour générer les données de SNP et en contrôler la qualité ;
4) mise en œuvre d'un pré-phasage d'haplotype, par un système informatique, pour les échantillons sanguins des deux parents ou uniquement du parent porteur d'une maladie ;

5) calcul du risque des quatre scénarios d'hérédité possibles au niveau de chaque SNP isolé, indiquant si les chaînes génétiques sont héritées des allèles paternels ou maternels des parents, et tenant compte du taux de contamination par les cellules maternelles (MCC) et du statut haplotypique ;

6) estimation du taux de MCC et détermination du statut haplotypique de chaque SNP afin de représenter si seul l'ADN de l'un des parents est présent dans le milieu de culture ;

7) calcul du risque d'observer les données de SNP au sein de régions adjacentes au site de la mutation responsable de la maladie, combinant le risque de chaque SNP individuel et les probabilités de recombinaison ;

8) détermination que l'embryon est ou non porteur du chromosome responsable de la maladie et fourniture d'un niveau de confiance.

5. Programme informatique comprenant une pluralité d'instructions capables d'être exécutées par un système informatique et disposées, lorsqu'elles sont exécutées, de manière à commander le système informatique afin qu'il analyse un échantillon biologique de milieu de culture d'un embryon cultivé in vitro pour déterminer la susceptibilité d'une maladie génétique dans l'embryon, configuré, lorsqu'il est exécuté, pour amener le système informatique à :

1) effectuer un alignement et une détection de SNP, par le système informatique, sur une pluralité de lectures de séquences pour générer les données de SNP et en contrôler la qualité, dans laquelle la pluralité de lectures de séquences a été obtenue par préparation de bibliothèques d'ADN et séquençage de molécules d'ADN amplifiées par la méthode de l'une quelconque des revendications 1 à 3 à partir de l'échantillon biologique de milieu de culture de l'embryon cultivé in vitro;

2) réaliser un pré-phasage d'haplotype, par le système informatique, pour les échantillons sanguins des deux parents ou uniquement du parent porteur d'une maladie;

3) calculer le risque des quatre scénarios d'hérédité possibles au niveau de chaque SNP isolé, indiquant si les chaînes génétiques sont héritées des allèles paternels ou maternels des parents, et tenant compte du taux de contamination par les cellules maternelles (MCC) et du statut haplotypique ;

4) estimer le taux de MCC et déterminer le statut haplotypique de chaque SNP afin de représenter si seul l'ADN de l'un des parents est présent dans le milieu de culture ;

5) calculer le risque d'observer les données de SNP au sein de régions adjacentes au site de la mutation responsable de la maladie, combinant le risque de chaque SNP individuel et les probabilités de recombinaison ;

6) déterminer si l'embryon est ou non porteur du chromosome responsable de la maladie et fournir un niveau de confiance.

6. Méthode selon la revendication 4 ou programme informatique selon la revendication 5, dans lequel :

(i) l'embryon présente une contamination due à des cellules maternelles et/ou à une perte d'haplotype ;

(ii) dans l'étape 3) de la méthode ou l'étape 1) conduite par le programme informatique, l'alignement et la détection de SNP délivrent en sortie l'emplacement physique, le type d'allèle, la profondeur d'allèle et la qualité de séquençage pour chaque SNP de l'échantillon biologique de milieu de culture de l'embryon cultivé in vitro ;

(iii) dans l'étape 3) de la méthode ou l'étape 1) conduite par le programme informatique, le contrôle de la qualité des données de SNP est effectué via une élimination par filtration des SNP ayant de faibles valeurs de qualité génique (GQ) de l'échantillon biologique de milieu de culture de l'embryon cultivé in vitro, de préférence une élimination par filtration des SNP ayant les valeurs GQ de 20 % les plus faibles de l'échantillon biologique de milieu de culture de l'embryon cultivé in vitro ;

(iv) dans l'étape 3) de la méthode ou l'étape 1) conduite par le programme informatique, le taux d'erreurs de séquençage et la densité relative des SNP dans la région adjacente au site de mutation responsable de la maladie sont estimés, et l'échantillon du milieu de culture ayant une densité de SNP extrêmement faible, ou un taux d'erreurs de séquençage élevé, est exclu, dans laquelle de préférence la densité des SNP exclus est inférieure à 0,001, ou le taux d'erreurs de séquençage exclues est supérieur à 0,2 ;

(v) dans l'étape 4) de la méthode ou l'étape 2) conduite par le programme informatique, un ou plusieurs des échantillons suivants dans la famille sont utilisés : l'échantillon sanguin du proposant, ou l'échantillon sanguin d'au moins un des grands-parents ;

(vi) dans l'étape 5) de la méthode ou l'étape 3) conduite par le programme informatique, le taux d'erreurs de séquençage estimé est intégré lors du calcul du risque pour chaque SNP individuel ;

(vii) l'étape 6) de la méthode ou l'étape 4) conduite par le programme informatique comprend en outre les étapes suivantes :

a) l'estimation initiale du taux de MCC et du statut haplotypique de chaque SNP dans la région adjacente au site de la mutation responsable de la maladie ; et

b) la mise à jour récursive du taux de MCC et du statut haplotypique de chaque SNP, dans laquelle de préférence l'étape b) utilise une méthode de recherche de marqueur ou, après l'étape a), l'échantillon ayant un taux de MCC anormal est exclu, dans laquelle de préférence le taux de MCC anormal est supérieur à 0,65 ou inférieur à -0,5 ;

(viii) dans l'étape 7) de la méthode ou l'étape 5) conduite par le programme informatique, un modèle bayésien et un algorithme récursif sont utilisés ;

(ix) dans l'étape 8) de la méthode ou l'étape 6) conduite par le programme informatique, la courbe de risque logarithmique pour les régions tant en amont qu'en aval du site de la mutation responsable de la maladie est tout d'abord tracée ; ou

(x) la maladie génétique inclut les maladies tant monogéniques que polygéniques.

7. Méthode selon la revendication 4 ou 6, ou programme informatique selon la revendication 5 ou 6, dans lequel, après l'étape 8) de la méthode ou l'étape 6) conduite par le programme informatique, de multiples SNP identifiés de l'embryon sont combinés pour que soit déterminée sa susceptibilité envers une maladie polygénique.

8. Système informatique pour analyser un échantillon biologique de milieu de culture d'un embryon cultivé in vitro pour déterminer la susceptibilité d'une maladie génétique dans l'embryon, comprenant :

1) Un moyen pour effectuer un alignement et une détection de SNP, par le système informatique, sur une pluralité de lectures de séquences pour générer les données de SNP et en contrôler la qualité, dans lequel la pluralité de lectures de séquences a été obtenue par préparation de bibliothèques d'ADN et séquençage de molécules d'ADN amplifiées par la méthode de l'une quelconque des revendications 1 à 3 à partir de l'échantillon biologique de milieu de culture de l'embryon cultivé in vitro ;

2) Un moyen pour réaliser un pré-phasage d'haplotype, par le système informatique, pour les échantillons sanguins des deux parents ou uniquement du parent porteur d'une maladie ;

3) Un moyen pour calculer le risque des quatre scénarios d'hérédité possibles au niveau de chaque SNP isolé, indiquant si les chaînes génétiques sont héritées des allèles paternels ou maternels des parents, et tenant compte du taux de contamination par les cellules maternelles (MCC) et du statut haplotypique ;

4) Un moyen pour estimer le taux de MCC et déterminer le statut haplotypique de chaque SNP afin de représenter si seul l'ADN de l'un des parents est présent dans le milieu de culture ;

5) Un moyen pour calculer le risque d'observer les données de SNP au sein de régions adjacentes au site de la mutation responsable de la maladie, combinant le risque à chaque SNP individuel et les probabilités de recombinaison ;

6) moyen pour déterminer si l'embryon est ou non porteur du chromosome responsable de la maladie et fournir un niveau de confiance.

9. Système selon la revendication 8, dans lequel :

(i) l'embryon présente une contamination due à des cellules maternelles et/ou à une perte d'haplotype ;

(ii) dans le moyen 1), l'alignement et la détection de SNP délivrent en sortie l'emplacement physique, le type d'allèle, la profondeur d'allèle et la qualité de séquençage pour chaque SNP de l'échantillon biologique de milieu de culture de l'embryon cultivé in vitro ;

(iii) dans le moyen 1), le contrôle de la qualité des données de SNP est effectué via une élimination par filtration des SNP ayant de faibles valeurs de qualité génique (GQ) de l'échantillon biologique de milieu de culture de l'embryon cultivé in vitro ;

(iv) les SNP ayant les valeurs GQ de 20 % les plus faibles sont éliminées par filtration de l'échantillon biologique de milieu de culture de l'embryon cultivé in vitro ;

(v) dans le moyen 1), le taux d'erreurs de séquençage et la densité relative des SNP dans la région adjacente au site de mutation responsable de la maladie sont estimés, et l'échantillon du milieu de culture ayant une densité de SNP extrêmement faible, ou un taux d'erreurs de séquençage élevé, est exclu, dans lequel de préférence la densité des SNP exclus est inférieure à 0,001, ou le taux d'erreurs de séquençage exclues est supérieur à 0,2 ;

(vi) dans le moyen 2), un ou plusieurs des échantillons suivants dans la famille sont utilisés : l'échantillon sanguin du proposant, ou l'échantillon sanguin d'au moins un des grands-parents ; ou

(vii) dans le moyen 3), le taux d'erreurs de séquençage estimé est intégré lors du calcul du risque pour chaque SNP individuel.

**10.** Système informatique selon la revendication 8, dans lequel le moyen 4) inclut en outre les fonctions suivantes :

a) l'estimation initiale du taux de MCC et du statut haplotypique de chaque SNP dans la région adjacente au site de la mutation responsable de la maladie ; et
b) la mise à jour récursive du taux de MCC et du statut haplotypique de chaque SNP, dans lequel de préférence

(i) la fonction b) utilise une méthode de recherche de marqueur ; ou
(ii) après la fonction a), l'échantillon ayant un taux de MCC anormal est exclu, dans lequel de préférence le taux de MCC anormal est supérieur à 0,65 ou inférieur à -0,5.

**11.** Système informatique selon la revendication 8, dans lequel :

(i) dans le moyen 5), un modèle bayésien et un algorithme récursif sont utilisés ;
(ii) dans le moyen 6), la courbe de risque logarithmique pour les régions tant en amont qu'en aval du site de la mutation responsable de la maladie est tout d'abord tracée ; ou
(iii) la maladie génétique inclut les maladies tant monogéniques que polygéniques.

**12.** Système informatique selon l'une quelconque des revendications 8 à 11, comprenant en outre un moyen pour combiner de multiples SNP identifiés de l'embryon pour identifier sa susceptibilité envers une maladie polygénique.

Fig. 1

Fig. 2

Fig. 3

Fig. 4

Fig. 5

**Father** **Mother**

**Proband** **SCM-02** **SCM-05** **SCM-06** **SCM-07**

P

Fig. 6

Fig. 7

Fig. 8

**REFERENCES CITED IN THE DESCRIPTION**

**Patent documents cited in the description**

- WO 2020115511 A
- US 10894980 B, Chen
- WO 9810077 A
- EP 2527438 A
- EP 2376517 A

**Non-patent literature cited in the description**

- **MAEKAWA, T.** ; **YANAGIHARA, K.** ; **OHTSUBO, E**. A cell-free system of Tn3 transposition and transposition immunity. *Genes Cells*, 1996, vol. 1, 1007-1016
- **CRAIG, N.L**. Tn7: a target site-specific transposon. *Mol. Microbiol.*, 1991, vol. 5, 2569-2573
- **CHALMERS, R.** ; **SEWITZ, S.** ; **LIPKOW, K.** ; **CRELLIN, P**. Complete nucleotide sequence of Tn10. *J. Bacteriol*, 2000, vol. 182, 2970-2972
- **LI, X.** ; **BURNIGHT, E.R.** ; **COONEY, A.L.** ; **MALANI, N.** ; **BRADY, T.** ; **SANDER, J.D.** ; **STABER, J.** ; **WHEELAN, S.J.** ; **JOUNG, J.K.** ; **MCCRAY, P.B., JR. et al.** PiggyBac transposase tools for genome engineering. *Proc. Natl. Acad. Sci. USA*, 2013, vol. 110, E2279-2287
- **IVICS, Z.** ; **HACKETT, P.B.** ; **PLASTERK, R.H.** ; **IZSVAK, Z.** Molecular reconstruction of Sleeping Beauty, a Tc1-like transposon from fish, and its transposition in human cells. *Cell*, 1997, vol. 91, 501-510
- **KAWAKAMI, K**. Tol2: a versatile gene transfer vector in vertebrates. *Genome Biol*, 2007, vol. 8 (1), S7
- **JORGENSEN, E.D.** ; **DURBIN, R.K.** ; **RISMAN, S.S.** ; **MCALLISTER, W.T**. Specific contacts between the bacteriophage T3, T7, and SP6 RNA polymerases and their promoters. *J. Biol. Chem.*, 1991, vol. 266, 645-651
- **MELTON, D.A.** ; **KRIEG, P.A.** ; **REBAGLIATI, M.R.** ; **MANIATIS, T.** ; **ZINN, K.** ; **GREEN, M.R**. Efficient in vitro synthesis of biologically active RNA and RNA hybridization probes from plasmids containing a bacteriophage SP6 promoter. *Nucleic Acids Res.*, 1984, vol. 12, 7035-7056
- **GORYSHIN, I.Y** ; **W.S. REZNIKOFF**. Tn5 in vitro transposition. *The Journal of biological chemistry*, 1998, vol. 273 (13), 7367-74
- **DAVIES, D.R. et al.** Three-dimensional structure of the Tn5 synaptic complex transposition intermediate. *Science*, 2000, vol. 289 (5476), 77-85
- **GORYSHIN, I.Y. et al.** Insertional transposon mutagenesis by electroporation of released Tn5 transposition complexes. *Nature biotechnology*, 2000, vol. 18 (1), 97-100
- **STEINIGER-WHITE, M.** ; **I. RAYMENT** ; **W.S. REZNIKOFF**. Structure/function insights into Tn5 transposition. *Current opinion in structural biology*, 2004, vol. 14 (1), 50-7
- **ADEY, A. et al.** Rapid, low-input, low-bias construction of shotgun fragment libraries by high-density in vitro transposition. *Genome biology*, 2010, vol. 11 (12), R119
- **MARINE, R. et al.** Evaluation of a transposase protocol for rapid generation of shotgun high-throughput sequencing libraries from nanogram quantities of DNA.. *Applied and environmental microbiology*, 2011, vol. 77 (22), 8071-9
- **PARKINSON, N.J. et al.** Preparation of high-quality next-generation sequencing libraries from picogram quantities of target DNA. *Genome research*, 2012, vol. 22 (1), 125-33
- **ADEY, A** ; **J. SHENDURE**. Ultra-low-input, tagmentation-based whole-genome bisulfite sequencing. *Genome research*, 2012, vol. 22 (6), 1139-43
- **PICELLI, S. et al.** Full-length RNA-seqfrom single cells using Smart-seq2. *Nature protocols*, 2014, vol. 9 (1), 171-81
- **BUENROSTRO, J.D. et al.** Transposition of native chromatin for fast and sensitive epigenomic profiling of open chromatin, DNA-binding proteins and nucleosome position. *Nature methods*, 2013
- **VAN GELDER, R.N. et al.** Amplified RNA synthesized from limited quantities of heterogeneous cDNA. *Proceedings of the National Academy of Sciences of the United States of America*, 1990, vol. 87 (5), 1663-7
- **KAWASAKI, E.S**. Microarrays and the gene expression profile of a single cell. *Annals of the New York Academy of Sciences*, 2004, vol. 1020, 92-100

- **LIVESEY, F.J.** Strategies for microarray analysis of limiting amounts of RNA. *Briefings in functional genomics & proteomics*, 2003, vol. 2 (1), 31-6
- **TANG, F.** ; **K. LAO** ; **M.A. SURANI**. Development and applications of single-cell transcriptome analysis. *Nature methods*, 2011, vol. 8 (4), 6-11
- **HASHIMSHONY, T. et al.** CEL-Seq: single-cell RNA-Seq by multiplexed linear amplification. *Cell reports*, 2012, vol. 2 (3), 666-73
- **SHANKARANARAYANAN, P. et al.** Single-tube linear DNA amplification for genome-wide studies using a few thousand cells. *Nature protocols*, 2012, vol. 7 (2), 328-38
- **C CHEN** ; **D XING** ; **L TAN** ; **H LI** ; **G ZHOU** ; **L HUANG** ; **XS XIE**. *Science*, 2017, vol. 356 (6334), 189-194
- **HANDYSIDE AH** ; **PENKETH RJ** ; **WINSTON RM** ; **PATTINSON JK** ; **DELHANTY JD** ; **TUDDENHAM EG**. Biopsy of human preimplantation embryos and sexing by DNA amplification. *The Lancet*, 18 February 1989, vol. 333 (8634), 347-9
- **HANDYSIDE AH** ; **KONTOGIANNI EH** ; **HARDY KR** ; **WINSTON RM**. Pregnancies from biopsied human preimplantation embryos sexed by Y-specific DNA amplificatio. *Nature*, 19 April 1990, vol. 344 (6268), 768-70
- **KEREM BS** ; **ROMMENS JM** ; **BUCHANAN JA** ; **MARKIEWICZ D** ; **COX TK** ; **CHAKRAVARTI A** ; **BUCHWALD M** ; **TSUI LC**. Identification of the cystic fibrosis gene: genetic analysis. *Science*, 08 September 1989, vol. 245 (4922), 1073-80
- **HANDYSIDE AH** ; **LESKO JG** ; **TARIN JJ** ; **WINSTON RM** ; **HUGHES MR**. Birth of a normal girl after in vitro fertilization and preimplantation diagnostic testing for cystic fibrosis. *New England Journal of Medicine*, 24 September 1992, vol. 327 (13), 905-9
- **LIU J** ; **LISSENS W** ; **SILBER SJ** ; **DEVROEY P** ; **LIEBAERS I** ; **VAN STEIRTEGHEM A**. Birth after preimplantation diagnosis of the cystic fibrosisΔ F508 mutation by polymerase chain reaction in human embryos resulting from intracytoplasmic sperm injection with epididymal sperm. *Jama*, 21 December 1994, vol. 272 (23), 1858-60
- **HARTON GL** ; **TSIPOURAS P** ; **SISSON ME** ; **STARR KM** ; **MAHONEY BS** ; **FUGGER EF** ; **SCHULMAN JD** ; **KILPATRICK MW** ; **LEVINSON G** ; **BLACK SH**. Preimplantation genetic testing for Marfan syndrome. *MHR: Basic science of reproductive medicine*, 01 September 1996, vol. 2 (9), 713-5
- **AO A** ; **WELLS D** ; **HANDYSIDE AH** ; **WINSTON RM** ; **DELHANTY JD**. Preimplantation genetic diagnosis of inherited cancer: familial adenomatous polyposis coli. *Journal of assisted reproduction and genetics*, March 1998, vol. 15, 140-4
- **SERMON K** ; **GOOSSENS V** ; **SENECA S** ; **LISSENS W** ; **DE VOS A** ; **VANDERVORST M** ; **VAN STEIRTEGHEM A** ; **LIEBAERS I**. Preimplantation diagnosis for Huntington's disease (HD): clinical application and analysis of the HD expansion in affected embryos. *Prenatal Diagnosis: Published in Affiliation With the International Society for Prenatal Diagnosis*, December 1998, vol. 18 (13), 1427-36
- **XU K** ; **SHI ZM** ; **VEECK LL** ; **HUGHES MR** ; **ROSENWAKS Z**. First unaffected pregnancy using preimplantation genetic diagnosis for sickle cell anemia. *Jama*, 12 May 1999, vol. 281 (18), 1701-6
- **HUSSEY ND** ; **DONGGUI H** ; **FROILAND DA** ; **HUSSEY DJ** ; **HAAN EA** ; **MATTHEWS CD** ; **CRAIG JE**. Analysis of five Duchenne muscular dystrophy exons and gender determination using conventional duplex polymerase chain reaction on single cells. *Molecular human reproduction*, 01 November 1999, vol. 5 (11), 1089-94
- **RAY PF** ; **GIGAREL N** ; **PAUL BONNEFONT J** ; **ATTIÉ T** ; **HAMAMAH S** ; **FRYDMAN N** ; **VEKEMANS M** ; **FRYDMAN R** ; **MUNNICH A**. First specific preimplantation genetic diagnosis for ornithine trans-carbamylase deficiency. *Prenatal diagnosis*, December 2000, vol. 20 (13), 1048-54
- **DE RYCKE M** ; **VAN DE VELDE H** ; **SERMON K** ; **LISSENS W** ; **DE VOS A** ; **VANDERVORST M** ; **VANDERFAEILLIE A** ; **VAN STEIRTEGHEM A** ; **LIEBAERS I**. Preimplantation genetic diagnosis for sickle - cell anemia and for β - thalassemia. *Prenatal Diagnosis: Published in Affiliation With the International Society for Prenatal Diagnosis*, March 2001, vol. 21 (3), 214-22
- **MOUTOU C** ; **GARDES N** ; **RONGIERES C** ; **OHL J** ; **BETTAHAR - LEBUGLE K** ; **WITTEMER C** ; **GERLINGER P** ; **VIVILLE S**. Allele - specific amplification for preimplantation genetic diagnosis (PGD) of spinal muscular atrophy. *Prenatal diagnosis*, June 2001, vol. 21 (6), 498-503
- **VERLINSKY Y** ; **RECHITSKY S** ; **SCHOOLCRAFT W** ; **STROM C** ; **KULIEVA**. Preimplantation diagnosis for Fanconi anemia combined with HLA matching. *Jama*, 27 June 2001, vol. 285 (24), 3130-3
- **SERMON K** ; **SENECA S** ; **DE RYCKE M** ; **GOOSSENS V** ; **VAN DE VELDE H** ; **DE VOS A** ; **PLATTEAU P** ; **LISSENS W** ; **VAN STEIRTEGHEM A** ; **LIEBAERS I**. PGD in the lab for triplet repeat diseases-myotonic dystrophy, Huntington's disease and Fragile-X syndrome. *Molecular and Cellular Endocrinology*, 22 October 2001, vol. 183, 77-85

- **GIRARDET A** ; **HAMAMAH S** ; **ANAHORY T** ; **DECHAUD H** ; **SARDA P** ; **HEDON B** ; **DEMAILLE J** ; **CLAUSTRES M**. First preimplantation genetic diagnosis of hereditary retinoblastoma using informative microsatellite markers. *MHR: Basic science of reproductive medicine*, 01 February 2003, vol. 9 (2), 111-6
- **FIORENTINO F** ; **BIRICIK A** ; **NUCCITELLI A** ; **DE PALMA R** ; **KAHRAMAN S** ; **IACOBELLI M** ; **TRENGIA V** ; **CASERTA D** ; **BONU MA** ; **BORINI A**. Strategies and clinical outcome of 250 cycles of preimplantation genetic diagnosis for single gene disorders. *Human Reproduction*, 01 March 2006, vol. 21 (3), 670-84
- **MUNNÉ S** ; **LEE A** ; **ROSENWAKS Z** ; **GRIFO J** ; **COHEN J**. Fertilization and early embryology: Diagnosis of major chromosome aneuploidies in human preimplantation embryos. *Human reproduction*, 01 December 1993, vol. 8 (12), 2185-91
- **WILTON L** ; **WILLIAMSON R** ; **MCBAIN J** ; **EDGAR D** ; **VOULLAIRE L**. Birth of a healthy infant after preimplantation confirmation of euploidy by comparative genomic hybridization. *New England Journal of Medicine*, 22 November 2001, vol. 345 (21), 1537-41
- **WELLS D** ; **ESCUDERO T** ; **LEVY B** ; **HIRSCHHORN K** ; **DELHANTY JD** ; **MUNNE S**. First clinical application of comparative genomic hybridization and polar body testing for preimplantation genetic diagnosis of aneuploidy. *Fertility and sterility*, 01 September 2002, vol. 78 (3), 543-9
- **TREFF NR** ; **SU J** ; **TAO X** ; **LEVY B** ; **SCOTT JR RT**. Accurate single cell 24 chromosome aneuploidy screening using whole genome amplification and single nucleotide polymorphism microarrays. *Fertility and sterility*, 01 November 2010, vol. 94 (6), 2017-21
- **GUTIÉRREZ-MATEO C** ; **COLLS P** ; **SÁNCHEZ-GARCÍA J** ; **ESCUDERO T** ; **PRATES R** ; **KETTERSON K** ; **WELLS D** ; **MUNNÉ S**. Validation of microarray comparative genomic hybridization for comprehensive chromosome analysis of embryos. *Fertility and sterility*, 01 March 2011, vol. 95 (3), 953-8
- **YANG Z** ; **LIU J** ; **COLLINS GS** ; **SALEM SA** ; **LIU X** ; **LYLE SS** ; **PECK AC** ; **SILLS ES** ; **SALEM RD**. Selection of single blastocysts for fresh transfer via standard morphology assessment alone and with array CGH for good prognosis IVF patients: results from a randomized pilot study. *Molecular cytogenetics*, December 2012, vol. 5, 1-8
- **SCOTT JR RT** ; **UPHAM KM** ; **FORMAN EJ** ; **HONG KH** ; **SCOTT KL** ; **TAYLOR D** ; **TAO X** ; **TREFF NR**. Blastocyst biopsy with comprehensive chromosome screening and fresh embryo transfer significantly increases in vitro fertilization implantation and delivery rates: a randomized controlled trial. *Fertility and sterility*, 01 September 2013, vol. 100 (3), 697-703
- **FORMAN EJ** ; **HONG KH** ; **FERRY KM** ; **TAO X** ; **TAYLOR D** ; **LEVY B** ; **TREFF NR** ; **SCOTT JR RT**. In vitro fertilization with single euploid blastocyst transfer: a randomized controlled trial. *Fertility and sterility*, 01 July 2013, vol. 100 (1), 100-7
- **WELLS D** ; **KAUR K** ; **GRIFO J** ; **GLASSNER M** ; **TAYLOR JC** ; **FRAGOULI E** ; **MUNNE S**. Clinical utilisation of a rapid low-pass whole genome sequencing technique for the diagnosis of aneuploidy in human embryos prior to implantation. *Journal of medical genetics*, 01 August 2014, vol. 51 (8), 553-62
- **RUBIO C** ; **BELLVER J** ; **RODRIGO L** ; **CASTILLÓN G** ; **GUILLÉN A** ; **VIDAL C** ; **GILES J** ; **FERRANDO M** ; **CABANILLAS S** ; **REMOHI J**. In vitro fertilization with preimplantation genetic diagnosis for aneuploidies in advanced maternal age: a randomized, controlled study. *Fertility and sterility*, 01 May 2017, vol. 107 (5), 1122-9
- **CONN CM** ; **HARPER JC** ; **WINSTON RM** ; **DELHANTY JD**. Infertile couples with Robertsonian translocations: preimplantation genetic analysis of embryos reveals chaotic cleavage divisions. *Human Genetics*, January 1998, vol. 102, 117-23
- **SCRIVEN PN** ; **HANDYSIDE AH** ; **OGILVIE CM**. Chromosome translocations: segregation modes and strategies for preimplantation genetic diagnosis. *Prenatal Diagnosis: Published in Affiliation With the International Society for Prenatal Diagnosis*, December 1998, vol. 18 (13), 1437-49
- **COONEN E** ; **MARTINI E** ; **DUMOULIN JC** ; **HOLLANDERS-CROMBACH HT** ; **DE DIE-SMULDERS C** ; **GERAEDTS JP** ; **HOPMAN AH** ; **EVERS JL**. Preimplantation genetic diagnosis of a reciprocal translocation t (3; 11)(q27. 3; q24. 3) in siblings. *Molecular human reproduction*, 01 March 2000, vol. 6 (3), 199-206
- **MUNNÉ S** ; **SANDALINAS M** ; **ESCUDERO T** ; **FUNG J** ; **GIANAROLI L** ; **COHEN J**. Outcome of preimplantation genetic diagnosis of translocations. *Fertility and Sterility*, 01 June 2000, vol. 73 (6), 1209-18
- **ESCUDERO T** ; **ABDELHADI I** ; **SANDALINAS M** ; **MUNNE S**. Predictive value of sperm fluorescence in situ hybridization analysis on the outcome of preimplantation genetic diagnosis for translocations. *Fertility and sterility*, 01 June 2003, vol. 79, 1528-34

- **MELOTTE C ; DEBROCK S ; D'HOOGHE T ; FRYNS JP ; VERMEESCH JR**. Preimplantation genetic diagnosis for an insertional translocation carrier. *Human Reproduction*, 01 December 2004, vol. 19 (12), 2777-83
- **LE CAIGNEC C ; SPITS C ; SERMON K ; DE RYCKE M ; THIENPONT B ; DEBROCK S ; STAESSEN C ; MOREAU Y ; FRYNS JP ; VAN STEIRTEGHEM A**. Single-cell chromosomal imbalances detection by array CGH. *Nucleic acids research*, 01 January 2006, vol. 34 (9), e68
- **TRAVERSA MV ; CAREY L ; LEIGH D**. A molecular strategy for routine preimplantation genetic diagnosis in both reciprocal and Robertsonian translocation carriers. *MHR: Basic science of reproductive medicine*, 19 February 2010, vol. 16 (5), 329-37
- **FIORENTINO F ; KOKKALI G ; BIRICIK A ; STAVROU D ; ISMAILOGLU B ; DE PALMA R ; ARIZZI L ; HARTON G ; SESSA M ; PANTOS K**. Polymerase chain reaction-based detection of chromosomal imbalances on embryos: the evolution of preimplantation genetic diagnosis for chromosomal translocations. *Fertility and sterility*, 01 November 2010, vol. 94 (6), 2001-11
- **RIUS M ; OBRADORS A ; DAINA G ; RAMOS L ; PUJOL A ; MARTINEZ-PASSARELL O ; MARQUÈS L ; OLIVER-BONET M ; BENET J ; NAVARRO J**. Detection of unbalanced chromosome segregations in preimplantation genetic diagnosis of translocations by short comparative genomic hibridization. *Fertility and sterility*, 01 July 2011, vol. 96 (1), 134-42
- **FIORENTINO F ; SPIZZICHINO L ; BONO S ; BIRICIK A ; KOKKALI G ; RIENZI L ; UBALDI FM ; IAMMARRONE E ; GORDON A ; PANTOS K**. PGD for reciprocal and Robertsonian translocations using array comparative genomic hybridization. *Human Reproduction*, 01 July 2011, vol. 26 (7), 1925-35
- **TREFF NR ; THOMPSON K ; RAFIZADEH M ; CHOW M ; MORRISON L ; TAO X ; GARNSEY H ; REDA CV ; METZGAR TL ; NEAL S**. SNP array-based analyses of unbalanced embryos as a reference to distinguish between balanced translocation carrier and normal blastocysts. *Journal of Assisted Reproduction and Genetics*, August 2016, vol. 33, 1115-9
- **ZHANG S ; LEI C ; WU J ; ZHOU J ; SUN H ; FU J ; SUN Y ; SUN X ; LU D ; ZHANG Y**. The establishment and application of preimplantation genetic haplotyping in embryo diagnosis for reciprocal and Robertsonian translocation carriers. *BMC medical genomics*, December 2017, vol. 10 (1), 1-9
- **TAN YQ ; TAN K ; ZHANG SP ; GONG F ; CHENG DH ; XIONG B ; LU CF ; TANG XC ; LUO KL ; LIN G**. Single-nucleotide polymorphism microarray-based preimplantation genetic diagnosis is likely to improve the clinical outcome for translocation carriers. *Human Reproduction*, 01 September 2013, vol. 28 (9), 2581-92
- **CHOW JF ; YEUNG WS ; LEE VC ; LAU EY ; NG EH**. Evaluation of preimplantation genetic testing for chromosomal structural rearrangement by a commonly used next generation sequencing workflow. *European Journal of Obstetrics & Gynecology and Reproductive Biology*, 01 May 2018, vol. 224, 66-73
- **TREFF NR ; ECCLES J ; LELLO L ; BECHOR E ; HSU J ; PLUNKETT K ; ZIMMERMAN R ; RANA B ; SAMOILENKO A ; HSU S**. Utility and first clinical application of screening embryos for polygenic disease risk reduction. *Frontiers in endocrinology*, 04 December 2019, vol. 10, 845
- **KUMAR A ; IM K ; BANJEVIC M ; NG PC ; TUNSTALL T ; GARCIA G ; GALHARDO L ; SUN J ; SCHAEDEL ON ; LEVY B**. Whole-genome risk prediction of common diseases in human preimplantation embryos. *Nature medicine*, March 2022, vol. 28 (3), 513-6
- **YAN L ; HUANG L ; XU L ; HUANG J ; MA F ; ZHU X et al.** Live births after simultaneous avoidance of monogenic diseases and chromosome abnormality by next-generation sequencing with linkage analyses. *Proc Natl Acad Sci U S A*, 2015, vol. 112, 15964-9
- **DOKRAS A ; SARGENT IL ; ROSS C ; GARDNER RL ; BARLOW DH**. Trophectoderm biopsy in human blastocysts. *Hum Reprod*, 1990, vol. 5, 821-825
- **KOKKALI G ; VRETTOU C ; TRAEGER-SYNODINOS J ; JONES GM ; CRAM DS ; STAVROU D ; TROUNSON AO ; KANAVAKIS E ; PANTOS K**. Birth of a healthy infant following trophectoderm biopsy from blastocysts for PGD of β-thalassaemia major: case report. *Hum Reprod*, 2005, vol. 20, 1855-1859
- **PALINI S ; GALLUZZI L ; DE STEFANI S ; BIANCHI M ; WELLS D ; MAGNANI M ; BULLETTI C**. Genomic DNA in human blastocoele fluid. *Reprod Biomed Online*, 2013, vol. 26, 603-610
- **GIANAROLI L ; MAGLI MC ; POMANTE A ; CRIVELLO AM ; CAFUERI G ; VALERIO M ; FERRARETTI AP**. Blastocentesis: a source of DNA for preimplantation genetic testing. *Results from a pilot study. Fertil Steril*, 2014, vol. 102, 1692-1699

- **TOBLER KJ** ; **ZHAO Y** ; **ROSS R** ; **BENNER AT** ; **XU X** ; **DU L** ; **BROMAN K** ; **THRIFT K** ; **BREZINA PR** ; **KEARNS WG**. Blastocoel fluid from differentiated blastocysts harbors embryonic genomic material capable of a whole-genome deoxyribonucleic acid amplification and comprehensive chromosome microarray analysis. *Fertil Steril*, 2015, vol. 104, 418-425

- **MAGLI MC** ; **POMANTE A** ; **CAFUERI G** ; **VALERIO M** ; **CRIPPA A** ; **FERRARETTI AP** ; **GIANAROLI L**. Preimplantation genetic testing: Polar bodies, blastomeres, trophectoderm cells, or blastocoelic fluid?. *Fertil Steril*, 2016, vol. 105, 676-683

- **ZHANG Y** ; **LI N** ; **WANG L** ; **SUN H** ; **MA M** ; **WANG H** ; **XU X** ; **ZHANG W** ; **LIU Y** ; **CRAM DS et al.** Molecular analysis of DNA in blastocoele fluid using next-generation sequencing. *J Assist Reprod Genet*, 2016, vol. 33, 637-645

- **SHANGGUAN T** ; **HE W** ; **LI H** ; **SHANG X** ; **LIU Y** ; **BAI X** ; **LI M** ; **XIE J**. Detection and analysis of DNA material in human blastocoel fluid. *Biomed Genet Genomics*, 2017, vol. 2, 1-5

- **CAPALBO A** ; **ROMANELLI V** ; **PATASSINI C** ; **POLI M** ; **GIRARDI L** ; **GIANCANI A** ; **STOPPA M** ; **CIMADOMO D** ; **UBALDI FM** ; **RIENZI L**. Diagnostic efficacy of blastocoel fluid and spent media as sources of DNA for preimplantation genetic testing in standard clinical conditions. *Fertil Steril*, 2018, vol. 110, 870-879

- **MAGLI MC** ; **ALBANESE C** ; **CRIPPA A** ; **TABANELLI C** ; **FERRARETTI AP** ; **GIANAROLI L**. Deoxyribonucleic acid detection in blastocoelic fluid: a new predictor of embryo ploidy and viable pregnancy. *Fertil Steril*, 2018, vol. 111, 77-85

- **GALLUZZI L** ; **PALINI S** ; **DE STEFANI S** ; **ANDREONI F** ; **PRIMITERRA M** ; **DIOTALLEVI A** ; **BULLETTI C** ; **MAGNANI M**. Extracellular embryo genomic DNA and its potential for genotyping applications. *Futur Sci OA*, 2015, vol. 1, FSO62

- **WU H** ; **DING C** ; **SHEN X** ; **WANG J** ; **LI R** ; **CAI B** ; **XU Y** ; **ZHONG Y** ; **ZHOU C**. Medium-based noninvasive preimplantation genetic diagnosis for human α-thalassemias-SEA. *Medicine (Baltimore)*, 2015, vol. 94, e669

- **XU J** ; **FANG R** ; **CHEN L** ; **CHEN D** ; **XIAO J-P** ; **YANG W** ; **WANG H** ; **SONG X** ; **MA T** ; **BO S et al.** Noninvasive chromosome screening of human embryos by genome sequencing of embryo culture medium for in vitro fertilization. *Proc Natl Acad Sci*, 2016, vol. 113, 11907-11912

- **SHAMONKI MI** ; **JIN H** ; **HAIMOWITZ Z** ; **LIU L**. Proof of concept: preimplantation genetic screening without embryo biopsy through analysis of cell-free DNA in spent embryo culture media. *Fertil Steril*, 2016, vol. 106, 1312-1318

- **FEICHTINGER M** ; **VACCARI E** ; **CARLI L** ; **WALLNER E** ; **MÄDEL U** ; **FIGL K** ; **PALINI S** ; **FEICHTINGER W**. Non-invasive preimplantation genetic screening using array comparative genomic hybridization on spent culture media: a proof-of-concept pilot study. *Reprod Biomed Online*, 2017, vol. 34, 583-589

- **LANE M** ; **ZANDER-FOX DL** ; **HAMILTON H** ; **JASPER MJ** ; **HODGSON BL** ; **FRASER M** ; **BELL F**. Ability to detect aneuploidy from cell free DNA collected from media is dependent on the stage of development of the embryo. *Fertil Steril*, 2017, vol. 108, e61

- **LIU WQ** ; **LIU JQ** ; **DU HZ** ; **LING JW** ; **SUN XF** ; **CHEN DJ**. Non-invasive preimplantation aneuploidy screening and diagnosis of beta thalassemia IVSII654 mutation using spent embryo culture medium. *Ann Med*, 2017, vol. 49, 319-328

- **HO JR** ; **ARRACH N** ; **RHODES-LONG K** ; **AHMADY A** ; **INGLES S** ; **CHUNG K** ; **BENDIKSON KA** ; **PAULSON RJ** ; **MCGINNIS LK**. Pushing the limits of detection: investigation of cell-free DNA for aneuploidy screening in embryos. *Fertil Steril*, 2018, vol. 110, 467-475

- **VERA-RODRIGUEZ M** ; **DIEZ-JUAN A** ; **JIMENEZ-ALMAZAN J** ; **MARTINEZ S** ; **NAVARRO R** ; **PEINADO V** ; **MERCADER A** ; **MESEGUER M** ; **BLESA D** ; **MORENO I et al.** Origin and composition of cell-free DNA in spent medium from human embryo culture during preimplantation development. *Obstet Gynecol Surv*, 2018, vol. 33, 745-756

- **FANG R** ; **YANG W** ; **ZHAO X** ; **XIONG F** ; **GUO C** ; **XIAO J** ; **CHEN L** ; **SONG X** ; **WANG H** ; **CHEN J et al.** Chromosome screening using culture medium of embryos fertilised in vitro: A pilot clinical study. *J Transl Med*, 2019, vol. 17, 1-8

- **HUANG L** ; **BOGALE B** ; **TANG Y** ; **LU S** ; **XIE XS** ; **RACOWSKY C**. Noninvasive preimplantation genetic testing for aneuploidy in spent medium may be more reliable than trophectoderm biopsy. *Proc Natl Acad Sci*, 2019, vol. 116, 201907472

- **RUBIO C** ; **RIENZI L** ; **NAVARRO-SÁNCHEZ L** ; **CIMADOMO D** ; **GARCIA-PASCUAL CM** ; **ALBRICCI L** ; **SOSCIA D** ; **VALBUENA D** ; **CAPALBO A** ; **UBALDI F et al.** Embryonic cell-free DNA versus trophectoderm biopsy for aneuploidy testing: concordance rate and clinical implications. *Fertil Steril*, 2019, vol. 112, 510-519

- **YEUNG QSY** ; **ZHANG YX** ; **CHUNG JPW** ; **LUI WT** ; **KWOK YKY** ; **GUI B** ; **KONG GWS** ; **CAO Y** ; **LI TC** ; **CHOY KW**. A prospective study of non-invasive preimplantation genetic testing for aneuploidies (NiPGT-A) using next-generation sequencing (NGS) on spent culture media (SCM). *J Assist Reprod Genet*, 2019, vol. 36, 1609-1621

- **JIAO J** ; **SHI B** ; **SAGNELLI M** ; **YANG D** ; **YAO Y** ; **LIW** ; **SHAO L** ; **LU S** ; **LI D** ; **WANG X**. Minimally invasive preimplantation genetic testing using blastocyst culture medium. *Hum Reprod*, 2019, vol. 34, 1369-1379
- **OU Z** ; **DENG Y** ; **LIANG Y** ; **CHEN Z** ; **SUN L**. Improved non-invasive preimplantation genetic testing for beta-thalassemia using spent embryo culture medium containing blastocoelic fluid. *Frontiers in endocrinology*, 20 January 2022, vol. 12, 1941
- **CHAN KA** ; **ZHANG J** ; **HUI AB** ; **WONG N** ; **LAU TK** ; **LEUNG TN** ; **LO KW** ; **HUANG DW** ; **LO YD**. Size distributions of maternal and fetal DNA in maternal plasma. *Clinical chemistry*, 01 January 2004, vol. 50 (1), 88-92
- **HUANG L** ; **MA F** ; **CHAPMAN A** ; **LU S** ; **XIE XS**. Single-cell whole-genome amplification and sequencing: methodology and applications. *Annual review of genomics and human genetics*, 24 August 2015, vol. 16, 79-102
- **CHEN C** ; **XING D** ; **TAN L** ; **LI H** ; **ZHOU G** ; **HUANG L** ; **XIE XS**. Single-cell whole-genome analyses by Linear Amplification via Transposon Insertion (LIANTI). *Science*, 14 April 2017, vol. 356 (6334), 189-94
- **LEAVER M** ; **WELLS D**. Non-invasive preimplantation genetic testing (niPGT): the next revolution in reproductive genetics?. *Human reproduction update*, 01 January 2020, vol. 26 (1), 16-42
- **CIMADOMO D** ; **RIENZI L** ; **CAPALBO A** ; **RUBIO C** ; **INNOCENTI F** ; **GARCIA-PASCUAL CM** ; **UBALDI FM** ; **HANDYSIDE A**. The dawn of the future: 30 years from the first biopsy of a human embryo. The detailed history of an ongoing revolution. *Human Reproduction Update*, July 2020, vol. 26 (4), 453-73
- **DE RYCKE M.** ; **GOOSSENS V.** ; **KOKKALI G.** ; **MEIJER-HOOGEVEEN M.** ; **COONEN E.** ; **MOUTOU C**. ESHRE PGD Consortium data collection XIV-XV: Cycles from January 2011 to December 2012 with pregnancy follow-up to October 2013. *Hum. Reprod*, 2017, vol. 32, 1974-1994
- **CARVALHO F** ; **MOUTOU C** ; **DIMITRIADOU E** ; **DREESEN J** ; **GIMÉNEZ C** ; **GOOSSENS V** ; **KAKOUROU G** ; **VERMEULEN N** ; **ZUCCARELLO D** ; **DE RYCKE M**. ESHRE PGT Consortium good practice recommendations for the detection of monogenic disorders. *Human reproduction open*, vol. 2020 (3), 1-18
- **XUE, A et al.** Genome-wide association analyses identify 143 risk variants and putative regulatory mechanisms for type 2 diabetes. *Nat. Commun*, 2018, vol. 9, 2941
- **FAN, H. C.** ; **GU, W.** ; **WANG, J.** ; **BLUMENFELD, Y. J.** ; **EL-SAYED, Y. Y.** ; **QUAKE, S. R.** Non-invasive prenatal measurement of the fetal genome. *Nature*, 2012, vol. 487 (7407), 320-324
- **NABIEVA, E** ; **SHARMA, S M** ; **KAPUSHEV Y et al.** Accurate fetal variant calling in the presence of maternal cell contamination. *European Journal of Human Genetics*, 2020, vol. 28 (11), 1615-1623
- **LANDER, E.S** ; **GREEN, P**. Construction of multi-locus genetic linkage maps in humans. *Proceedings of the National Academy of Sciences*, 1987, vol. 84 (8), 2363-2367
- **KRUGLYAK L** ; **DALY M J** ; **REEVE-DALY M P et al.** Parametric and nonparametric linkage analysis: a unified multipoint approach. *American journal of human genetics*, 1996, vol. 58 (6), 1347
- **IDURY R M** ; **ELSTON R C**. A faster and more general hidden Markov model algorithm for multi- point likelihood calculations. *Human heredity*, 1997, vol. 47 (4), 197-202
- **KRUGLYAK L** ; **LANDER E S**. Faster multipoint linkage analysis using Fourier transforms. *J. Comput. Biol.*, 1998, vol. 5 (1), 1-7
- **ABECASIS G R** ; **CHERNY S S** ; **COOKSON W O et al.** Merlin-rapid analysis of dense genetic maps using sparse gene flow trees. *Nature genetics*, 2002, vol. 30 (1), 97-101
- **SHITARA** ; **AKIHIRO et al.** Cell-free DNA in spent culture medium effectively reflects the chromosomal status of embryos following culturing beyond implantation compared to trophectoderm biopsy. *PLoS One*, 2021, vol. 16 (2), e0246438
- **YIN** ; **BAOLI et al.** Validation of preimplantation genetic tests for aneuploidy (PGT-A) with DNA from spent culture media (SCM): concordance assessment and implication. *Reproductive Biology and Endocrinology*, 2021, vol. 19 (1), 41